# EUROPEAN PATENT SPECIFICATION

(11) **EP 4 211 246 B1**
(45) Date of publication and mention of the grant of the patent: **23.07.2025**
(21) Application number: 21867505.6
(22) Date of filing: 08.09.2021
(51) Int. Cl.: C12N 15/113, C12Q 1/6806, C12Q 1/6869, C12Q 1/6855

(54) **ADAPTORS AND METHODS FOR HIGH EFFICIENCY CONSTRUCTION OF GENETIC LIBRARIES AND GENETIC ANALYSIS**
ADAPTER UND VERFAHREN ZUR HOCHEFFIZIENTEN KONSTRUKTION GENETISCHER BIBLIOTHEKEN UND GENETISCHE ANALYSE
ADAPTATEURS ET PROCÉDÉS DE CONSTRUCTION À HAUT RENDEMENT DE BIBLIOTHÈQUES GÉNÉTIQUES ET D'ANALYSE GÉNÉTIQUE

(30) Priority: 08.09.2020 US 202063075543 P
(43) Date of publication of application: 19.07.2023
(73) Proprietor: Resolution Bioscience, Inc., Madison, WI 53719 (US)
(72) Inventor: GUO, Jiannan, Madison, WI 53719 (US); HERNANDEZ, Jennifer, Madison, WI 53719 (US)
(74) Representative: Forresters IP LLP
(86) International application number: PCT/US2021/049448
(87) International publication number: WO 2022/055984

(56) References cited:
- EP-A2- 3 551 769
- WO-A1-2018/050722
- WO-A1-2018/119399
- WO-A1-2018/140695
- WO-A1-2019/140201
- WO-A1-2020/106906
- WO-A2-2020/132316
- JP-A- 2020 516 281
- US-A1- 2017 355 984
- US-A1- 2018 245 072

## Description

### FIELD OF THE DISCLOSURE

The present disclosure relates to compositions and methods for high efficiency construction of genetic libraries and methods of use thereof. The genetic libraries produced using the compositions and methods described herein may be used for genetic analysis.

### SEQUENCE LISTING

The instant application contains a Sequence Listing which has been submitted electronically in ASCII format. Said ASCII copy, created on September 7, 2021, is named CLFK_006_01WO_SeqList_ST25 and is about 20 KB in size.

### BACKGROUND

Next generation sequencing (NGS) can be used in a variety of clinical settings to identify genetic changes. NGS is roughly divided into the process elements of sample preprocessing, library preparation, sequencing, and bioinformatics. Currently, sample preprocessing and library preparation are labor intensive processes that are done mostly without automation. Library preparation protocols usually consist of a multistep process and require costly reagents and substantial hands-on time. To address this bottleneck in NGS, an automated NGS method that allows sample multiplexing, high throughput, and increased sensitivity is highly desired.
WO 2020/132316, WO 2019/140201 and WO 2018/140695 disclose methods for amplifying and sequencing target sequences in a low-input sample.
WO 2018/050722 discloses methods for labelling individual nucleic acid molecules present in a sample involving adaptors designed to allow incorporation of a barcode (Tag) by extension.

### BRIEF SUMMARY

The invention is defined in the appended claims.

The present disclosure provides a multifunctional adaptor comprising a) a ligation strand oligonucleotide, and b) a non-ligation strand oligonucleotide that is capable of hybridizing to a region at the 3' end of the ligation strand oligonucleotide and forming a duplex therewith; wherein the ligation strand oligonucleotide, upon contact with a double-stranded DNA (dsDNA) fragment from a sample, ligates to the 5' end of each strand of the dsDNA fragment; wherein the ligation strand oligonucleotide comprises (i) a 3' terminal overhang; (ii) an amplification region comprising a polynucleotide sequence capable of serving as a primer recognition site; (iii) a unique multifunctional ID region; (iv) a unique molecule identifier (UMI) multiplier; and (v) an anchor region comprising a polynucleotide sequence that is at least partially complementary to the non-ligation strand oligonucleotide; wherein the dsDNA fragment comprises a phosphate group at the 5' terminus of each strand and an overhang at the 3' terminus of each strand; wherein each dsDNA fragment can be identified by the combination of the multifunctional ID region and the UMI multiplier; and wherein the sample can be identified by the multifunctional ID region.

In some embodiments of the multifunctional adaptors of the disclosure, the ligation strand oligonucleotide comprises a dT overhang at the 3' terminus and the dsDNA fragment comprises a dA overhang at the 3' terminus of each strand.

In some embodiments of the multifunctional adaptors of the disclosure, the ligation strand oligonucleotide comprises a dA overhang at the 3' terminus and the dsDNA fragment comprises a dT overhang at the 3' terminus of each strand.

In some embodiments of the multifunctional adaptors of the disclosure, the ligation strand oligonucleotide comprises a dC overhang at the 3' terminus and the dsDNA fragment comprises a dG overhang at the 3' terminus of each strand.

In some embodiments of the multifunctional adaptors of the disclosure, the ligation strand oligonucleotide comprises a dG overhang at the 3' terminus and the dsDNA fragment comprises a dC overhang at the 3' terminus of each strand.

In some embodiments of the multifunctional adaptors of the disclosure, the amplification region in the ligation strand oligonucleotide comprises a polynucleotide sequence capable of serving as a primer recognition site for PCR, LAMP, NASBA, SDA, RCA, or LCR.

In some embodiments of the multifunctional adaptors of the disclosure, the non-ligation strand oligonucleotide comprises a modification at its 3' terminus that prevents ligation to the 5' end of the dsDNA fragment and/or adaptor dimer formation.

In some embodiments of the multifunctional adaptors of the disclosure, the sample is isolated or derived from a mammal. In some embodiments, the mammal is an animal model for a human disease. In some embodiments, the mammal is a mouse, rat, guinea pig, rabbit, pig, cat, dog, sheep or horse. In some embodiments, the mammal is a non-human primate (NHP). In some embodiments, the mammal a human.

In some embodiments of the multifunctional adaptors of the disclosure, the sample is isolated or derived from one or more cell types. In some embodiments, the sample is isolated or derived from one or more tissue types. In some embodiments, the sample is isolated or derived from one or more sources. In some embodiments, the one or more sources comprise a donor. In some embodiments, the donor is a human. In some embodiments, the donor is a healthy or control donor. In some embodiments, the one or more sources comprise a patient or subject (e.g. of a clinical trial). In some embodiments, the patient or the subject is a human. In some embodiments, the patient or the subject is a healthy or control patient or subject. In some embodiments, the patient or the subject is a test patient or subject. In some embodiments, the patient or the subject presents a sign or symptom of a disease or disorder. In some embodiments, the patient or the subject is pregnant. In some embodiments, the patient or the subject presents a family history or a genetic marker of a disease or disorder.

In some embodiments of the multifunctional adaptors of the disclosure, sample is a tissue biopsy. In some embodiments, the tissue biopsy is taken from a tumor or a tissue suspected of being a tumor.

In some embodiments of the multifunctional adaptors of the disclosure, the dsDNA fragment is cell free DNA (cfDNA), genomic DNA (gDNA), complementary DNA (cDNA), mitochondrial DNA, methylated DNA, or demethylated DNA. In some embodiments, the dsDNA fragment comprises one or more of a cell free DNA (cfDNA), a genomic DNA (gDNA), a complementary DNA (cDNA), a mitochondrial DNA, a methylated DNA, and a demethylated DNA. In some embodiments, the dsDNA fragment comprises a cell free DNA (cfDNA). In some embodiments, the dsDNA fragment comprises a genomic DNA (gDNA). In some embodiments, the dsDNA fragment comprises a complementary DNA (cDNA). In some embodiments, the dsDNA fragment comprises a mitochondrial DNA. In some embodiments, the dsDNA fragment comprises a methylated DNA. In some embodiments, the dsDNA fragment comprises a demethylated DNA.

In some embodiments of the multifunctional adaptors of the disclosure, the dsDNA is isolated or derived from a sample or a test sample. In some embodiments, the sample or the test sample comprises a biological sample. In some embodiments, the biological sample comprises a biological fluid selected from the group consisting of: amniotic fluid, blood, plasma, serum, semen, lymphatic fluid, cerebral spinal fluid, ocular fluid, urine, saliva, stool, mucous, tears and sweat. In some embodiments, the biological sample or the biological fluid comprises amniotic fluid. In some embodiments, the biological sample or the biological fluid comprises one or more of whole blood, plasma, buffy coat, and serum. In some embodiments, the biological sample or the biological fluid comprises lymphatic fluid. In some embodiments, the biological sample or the biological fluid comprises cerebral spinal fluid. In some embodiments, the biological sample or the biological fluid comprises urine. In some embodiments, the biological sample or the biological fluid comprises one or more of saliva, stool, mucus, tears and sweat.

In some embodiments of the multifunctional adaptors of the disclosure, the dsDNA fragments are obtained by a method comprising fragmenting genomic DNA to produce at least one DNA fragment. In some embodiments, the method further comprises, prior to the fragmenting step, isolating genomic DNA from a sample comprising at least one cell. In some embodiments, fragmenting comprises contacting the genomic DNA with at least one enzyme, wherein the enzyme digests the genomic DNA to produce at least one DNA fragment. In some embodiments, fragmenting comprises applying mechanical stress to the genomic DNA to produce at least one DNA fragment. In some embodiments, fragmenting comprises contacting genomic DNA with one or more compounds to chemically disrupt one or more bonds of the genomic DNA. In some embodiments, the mechanical stress comprises sonicating the genomic DNA to produce at least one DNA fragment. In some embodiments, following the fragmenting step, the method further comprises contacting the at least one DNA fragment and an enzyme, wherein the enzyme digests one or both ends of the DNA fragment to produce a DNA fragment comprising one or more blunt end(s). In some embodiments, following the fragmenting step, the method further comprises attaching a deoxyribonucleic acid adenine (dA) tail to one or both blunt ends of the at least one DNA fragment. In some embodiments, following the fragmenting step, the method further comprises phosphorylating one or both ends of the at least one DNA fragment. In some embodiments, following the fragmenting step, the method further comprises attaching of the tail and the phosphorylating steps either simultaneously or sequentially. In some embodiments, following the fragmenting step, the method further comprises attaching of the tail and the phosphorylating steps sequentially. In some embodiments, the attaching of the tail step follows the phosphorylating step. In some embodiments, the phosphorylating step follows the attaching of the tail step.

In some embodiments of the multifunctional adaptors of the disclosure, the dsDNA fragments are obtained by the steps comprising: (a) isolating genomic DNA from the test sample; and (b) fragmenting the genomic DNA to obtain the genomic DNA fragment. In some embodiments, step (b) is performed by contacting the genomic DNA with at least one digestion enzyme. In some embodiments, step (b) is performed by applying mechanical stress to the genomic DNA. In some embodiments, the mechanical stress is applied by sonicating the genomic DNA.

In some embodiments of the multifunctional adaptors of the disclosure, the dsDNA fragments are obtained by the steps comprising: (a) isolating cellular DNA from the test sample; and (b) fragmenting the cellular DNA to obtain the genomic DNA fragment. In some embodiments, step (b) is performed by contacting the cellular DNA with at least one digestion enzyme. In some embodiments, step (b) is performed by applying mechanical stress to the cellular DNA. In some embodiments, the mechanical stress is applied by sonicating the cellular DNA.

In some embodiments of the multifunctional adaptors of the disclosure, the amplification region is between 10 and 50 nucleotides in length. In some embodiments, the amplification region is between 20 and 30 nucleotides in length. In some embodiments, the amplification region is 25 nucleotides in length.

In some embodiments of the multifunctional adaptors of the disclosure, the multifunctional ID region is between 3 and 50 nucleotides in length. In some embodiments, the multifunctional ID region is between 3 and 15 nucleotides in length. In some embodiments, the multifunctional ID region is 8 nucleotides in length.

In some embodiments of the multifunctional adaptors of the disclosure, the UMI multiplier is adjacent to or contained within the multifunctional ID region. In some embodiments, the UMI multiplier is between 1 and 5 nucleotides in length. In some embodiments, the UMI multiplier is 3 nucleotides in length, and comprises one of 64 possible nucleotide sequences.

In some embodiments of the multifunctional adaptors of the disclosure, the anchor region is between 1 and 50 nucleotides in length. In some embodiments, the anchor region is between 5 and 25 nucleotides in length. In some embodiments, the anchor region is 10 nucleotides in length.

In some embodiments of the multifunctional adaptors of the disclosure, a plurality of multifunctional adaptors is ligated to a plurality of dsDNA fragments.

In some embodiments, the dsDNA fragments are end-repaired prior to ligating with a plurality of multifunctional adaptors.

In some embodiments of the multifunctional adaptors of the disclosure, the amplification regions of each multifunctional adaptor of the plurality of multifunctional adaptors comprise an identical nucleotide sequence. In some embodiments, the identical nucleotide sequence is a PCR primer binding site.

In some embodiments of the multifunctional adaptors of the disclosure, the multifunctional ID region of each multifunctional adaptor of the plurality of multifunctional adaptors comprises one of between 2 and 10,000 unique nucleotide sequences. In some embodiments, the multifunctional ID region of each multifunctional adaptor of the plurality of multifunctional adaptors comprises one of between 50 and 500 unique nucleotide sequences. In some embodiments, the multifunctional ID region of each multifunctional adaptor of the plurality of multifunctional adaptors comprises one of between 100 and 400 unique nucleotide sequences. In some embodiments, the multifunctional ID region of each multifunctional adaptor of the plurality of multifunctional adaptors comprises one of 60 unique nucleotide sequences.

In some embodiments of the multifunctional adaptors of the disclosure, the multifunctional ID region of each multifunctional adaptor of the plurality of multifunctional adaptors is 8 nucleotides in length.

In some embodiments of the multifunctional adaptors of the disclosure, each multifunctional adaptor of the plurality of multifunctional adaptors comprises one of between 64 and 2,560,000 unique nucleotide sequences.

In some embodiments of the multifunctional adaptors of the disclosure, each multifunctional adaptor of the plurality of multifunctional adaptors comprises one of 3840 unique nucleotide sequences, and each nucleotide sequence is discrete from any other sequence of the 3840 unique nucleotide sequences by Hamming distance of at least two.

In some embodiments of the multifunctional adaptors of the disclosure, each of the plurality of multifunctional adaptors comprises a UMI multiplier that is adj acent to or contained within the multifunctional ID region.

In some embodiments of the multifunctional adaptors of the disclosure, the UMI multiplier of each multifunctional adaptor of the plurality of multifunctional adaptors is between 1 and 5 nucleotides in length. In some embodiments, the UMI multiplier of each multifunctional adaptor of the plurality of multifunctional adaptors is 3 nucleotides in length.

In some embodiments of the multifunctional adaptors of the disclosure, the anchor region of each multifunctional adaptor of the plurality of multifunctional adaptors comprises one of four nucleotide sequences, and each multifunctional ID region of a given sequence can be paired to each one of the four anchor regions.

In some embodiments of the multifunctional adaptors of the disclosure, the amplification regions of each multifunctional adaptor of the plurality of multifunctional adaptors comprise an identical nucleotide sequence; wherein the multifunctional ID region of each multifunctional adaptor of the plurality of multifunctional adaptors is 8 nucleotides in length; wherein the nucleotide sequence of each multifunctional ID region is discrete from the nucleotide sequence of any other multifunctional ID regions of the plurality of multifunctional adaptors by Hamming distance of at least two; wherein each of the plurality of multifunctional adaptors comprises a UMI multiplier that is adjacent to or contained within the multifunctional ID region, wherein the UMI multiplier of each multifunctional adaptor of the plurality of multifunctional adaptors is three nucleotides in length, and wherein the UMI multiplier of each of the possible nucleotide sequences is paired to each multifunctional ID region of the plurality of multifunctional adaptors, wherein the anchor region of each multifunctional adaptor of the plurality of multifunctional adaptors comprises one of four nucleotide sequences, and wherein each multifunctional ID region of a given sequence can be paired to each one of the four anchor regions.

The disclosure provides a complex comprising a multifunctional adaptor and a dsDNA fragment, wherein the multifunctional adaptor is selected from any one of the multifunctional adaptors disclosed.

The disclosure provides a plurality of multifunctional adaptors of the disclosure. In some embodiments, the plurality may also be referred to as a pool. In some embodiments, the plurality of multifunctional adaptors comprise a set of adaptors applied to a sample. In some embodiments, within the set of adaptors applied to a sample, each multifunctional adaptor of the plurality of multifunctional adaptors contains a unique ID region or a unique UMI. In some embodiments, the number of multifunctional adaptor of the plurality of multifunctional adaptors may be increased or decreased to accommodate the sample or cellular DNA target of the sample. In some embodiments, the number of multifunctional adaptor of the plurality of multifunctional adaptors may be increased or decreased to correspond to a level of multiplexing required to detect and or analyze a cellular DNA target of the sample. In some embodiments, the number of multifunctional adaptor of the plurality of multifunctional adaptors may be increased or decreased by increasing or decreasing the number of unique ID regions or unique UMIs within the plurality of multifunctional adaptors applied to a sample. In some embodiments, the number of multifunctional adaptor of the plurality of multifunctional adaptors may be increased or decreased by increasing or decreasing the number of nucleotides of the ID region or the anchor region.

The disclosure provides a method for making an adaptor-tagged DNA library comprising: (a) ligating a plurality of multifunctional adaptors with a plurality of dsDNA fragments to generate a plurality of multifunctional adaptor/dsDNA fragment complexes, wherein the multifunctional adaptor is selected from any one of the multifunctional adaptors disclosed; and (b) contacting the multifunctional adaptor/dsDNA fragment complexes from step (a) with one or more enzymes to form an adaptor-tagged DNA library comprising a plurality of contiguous adaptor-tagged dsDNA fragments. In some embodiments, each multifunctional adaptor/dsDNA fragment complex of the plurality of complexes comprises a multifunctional adaptor ligated to each end of the dsDNA fragment.

In some embodiments of the methods of the disclosure, the dsDNA fragment is cell free DNA (cfDNA), genomic DNA (gDNA), complementary DNA (cDNA), mitochondrial DNA, or methylated DNA, or demethylated DNA. In some embodiments, the dsDNA fragment comprises one or more of a cell free DNA (cfDNA), a genomic DNA (gDNA), a complementary DNA (cDNA), a mitochondrial DNA, a methylated DNA, and a demethylated DNA. In some embodiments, the dsDNA fragment comprises a cell free DNA (cfDNA). In some embodiments, the dsDNA fragment comprises a genomic DNA (gDNA). In some embodiments, the dsDNA fragment comprises a complementary DNA (cDNA). In some embodiments, the dsDNA fragment comprises a mitochondrial DNA. In some embodiments, the dsDNA fragment comprises a methylated DNA. In some embodiments, the dsDNA fragment comprises a demethylated DNA.

In some embodiments of the methods of the disclosure, the plurality of dsDNA fragments is end-repaired prior to ligating with a plurality of multifunctional adaptors.

In some embodiments of the methods of the disclosure, the plurality of dsDNA fragments is obtained from a library selected from the list consisting of a low pass whole genome library, an amplicon library, a whole exome library, a cDNA library, or a methylated DNA library.

In some embodiments of the methods of the disclosure, the non-ligation strand oligonucleotide is displaced from the multifunctional adaptor/dsDNA fragment complex.

In some embodiments of the methods of the disclosure, the one or more enzymes comprise a DNA ligase or an RNA ligase. In some embodiments, the DNA ligase comprises a T4 DNA ligase or a Taq DNA ligase.

The disclosure provides a method for making an adaptor-tagged DNA library comprising: (a) ligating a plurality of multifunctional adaptors with a plurality of dsDNA fragments to generate a plurality of multifunctional adaptor/dsDNA fragment complexes, wherein the multifunctional adaptor is selected from any one of the multifunctional adaptors disclosed; and (b) contacting the multifunctional adaptor/dsDNA fragment complexes from step (a) with one or more enzymes to form contiguous adaptor-tagged dsDNA fragments; and amplifying the contiguous adaptor-tagged dsDNA fragments to generate an adaptor-tagged DNA library comprising a plurality of contiguous adaptor-tagged dsDNA fragments.

In some embodiments of the methods of the disclosure, one or more primers are used for amplification. In some embodiments, the one or more primers comprise a universal primer binding sequence that hybridizes to the primer-binding region of the adaptor.

The disclosure provides an adaptor-tagged DNA library produced according to any one of the methods disclosed.

The disclosure provides a method for making a library of hybrid molecules comprising:
(a) making an adaptor-tagged library according to any one of the methods disclosed above;
(b)hybridizing the adaptor-tagged DNA library with one or more multifunctional capture probes to form one or more capture probe/adaptor-tagged DNA complexes, wherein each multifunctional capture probe comprises (i) a first region capable of hybridizing to a partner oligonucleotide, wherein, optionally, the first region comprises a tail sequence comprising a PCR primer binding site; and (ii) a second region capable of hybridizing to a specific target region in the tagged genetic DNA library; (c) isolating the one or more capture probe/adaptor-tagged DNA complexes from step (b), wherein each isolated capture probe/adaptor-tagged DNA complex comprises a capture probe and an adaptor-tagged DNA fragment; and (d) enzymatically processing the one or more isolated capture probe/ adaptor-tagged DNA complexes from step (c) to generate one or more adaptor-tagged hybrid nucleic acid molecules (hybrid molecules), wherein each hybrid molecule comprises the capture probe and a complement of the adaptor-tagged DNA fragment that is 3' from where the capture probe hybridized to the targeted genetic sequence. In some embodiments, the method further comprises (e) performing PCR on the hybrid molecules from step (d) to generate a targeted genetic library comprising amplified hybrid molecules. In some embodiments, the enzymatic processing step of (d) comprises performing 5'-3' DNA polymerase extension of the capture probe using the adaptor-tagged DNA fragment in the complex as a template.

In some embodiments of any one of the methods disclosed, at least one capture probe hybridizes downstream of the targeted genetic sequence and at least one capture probe hybridizes upstream of the targeted genetic sequence.

In some embodiments of any one of the methods disclosed, the capture probe comprises a sequencing primer recognition sequence.

In some embodiments, the disclosure provides a capture probe/adaptor-tagged DNA complex produced according to any one of the methods disclosed.

In some embodiments, the disclosure provides a library of hybrid molecules produced according to any one of the methods disclosed.

In some embodiments, the disclosure provides a targeted genetic library produced according to any one of the methods disclosed.

Some embodiments of the disclosure are drawn to a method comprising performing targeted genetic analysis on a library of hybrid molecules produced according to any one of the methods disclosed. In some embodiments, the targeted genetic analysis is sequence analysis. In some embodiments, the targeted genetic analysis is copy number analysis.

Some embodiments of the disclosure are drawn to a method comprising performing targeted genetic analysis on a targeted genetic library produced according to any one of the methods disclosed. In some embodiments, the targeted genetic analysis is sequence analysis. In some embodiments, the targeted genetic analysis is copy number analysis.

In some embodiments of any one of the methods disclosed, the capture probe region in the hybrid molecule is sequenced. In some embodiments of any one of the methods disclosed, the entire capture probe region in the hybrid molecule is sequenced. In some embodiments of any one of the methods disclosed, a portion of the capture probe region in the hybrid molecule is sequenced.

These and other aspects are addressed in more detail in the detailed description set forth below.

### BRIEF DESCRIPTION OF THE DRAWINGS

**Fig. 1** is a schematic diagram depicting an exemplary multifunctional adaptor of the disclosure. The exemplary multifunctional adaptor comprises, from 5' to 3', a 25-nucleotide amplification region, an 8-nucleotide ID region, a 3-nucleotide UMI multiplier region, and a 10-nucleotide anchor region. The multifunctional adaptor also comprises a dT overhang at the 3' end. The length of each region of the multifunctional adaptor may be varied as described below.
**Fig. 2** is a schematic diagram depicting an embodiment of the overall workflow of the methods in the disclosure. The steps of this workflow will be described in further detail below.

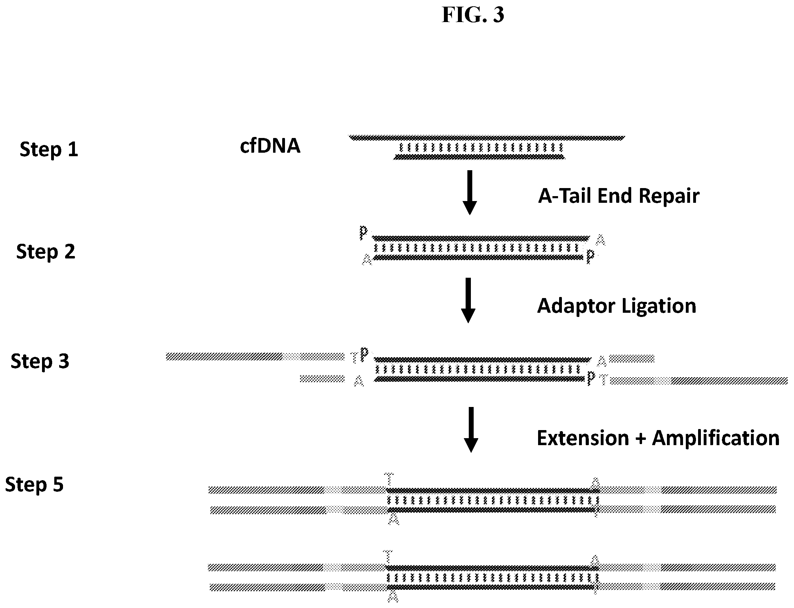

**Fig. 3** is a schematic diagram depicting an exemplary process for generating an adaptor-tagged DNA library according to some embodiments of the methods of the disclosure. In step one, cfDNA is end repaired using NEB Next Ultra II End Repair^{®}/dA-Tailing Module. A base is then added to the 3' end, and the 5' ends are phosphorylated (Step 2). Step 2 may be performed using a thermal cycler. In step 3, multifunctional adaptors having a 3' dT terminal overhang are coupled to each of the 5' end and the 3' end. In an optional step 4 (not shown), affinity beads may be used to separate unligated fragments and adaptors from the adaptor-ligated DNA strands. In some embodiments, the method proceeds directly from step 3 to step 5, amplification. In this step, the Ultra II Q5^{®} enzyme extends the overhangs on the fragments to make a double stranded library and amplifies the fragments using a standard amplification cycle. Optionally, an additional 3-minute extension step may be performed at the beginning to allow for the fill-in of the overhangs at the 5' and 3' end of the fragments.

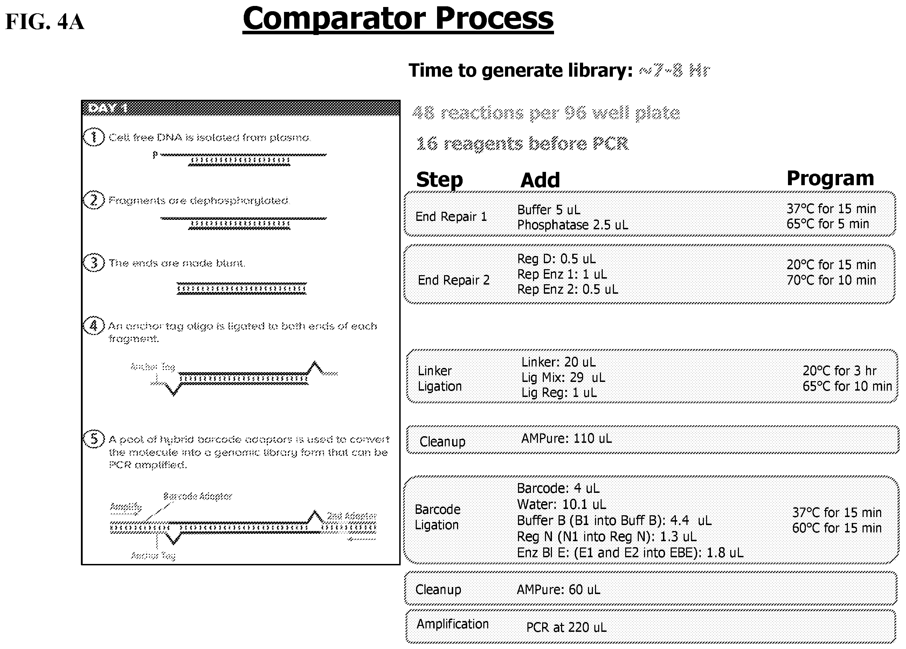

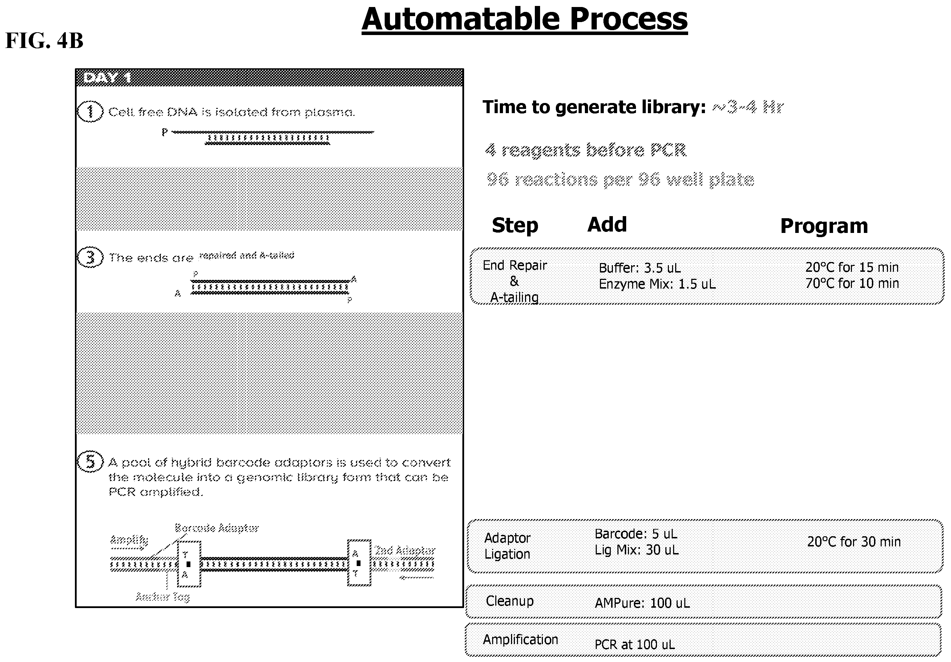

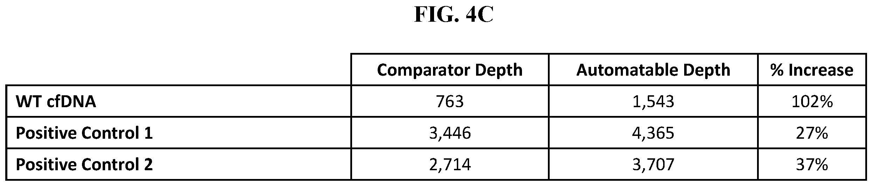

**Fig. 4A****-****Fig. 4C** provide a series of schematic diagrams and a table comparing a comparator process (not designed to be automatable) of generating an adaptor-tagged DNA library to an automatable process of generating an adaptor-tagged DNA library according to the methods in the disclosure. **Fig. 4A** depicts the steps performed in the comparator process, as well as the reagents used and thermocycler programs. **Fig. 4B** depicts the steps performed in the automatable process, as well as reagents used and thermocycler programs. **Fig. 4C** provides depth data from an exemplary wildtype cfDNA sample as well as two control samples, processed using each of the comparator and the automatable processes. Percent increase in depth when using the automatable process is also shown.
**Fig. 5** is a schematic diagram depicting an illustrative method for the preparation and amplification of adaptor-tagged DNA libraries according to some embodiments of the methods of the disclosure.
**Fig. 6** is a schematic diagram depicting capture probe hybridization and extension according to some embodiments of the methods of the disclosure.
**Fig. 7** is a schematic diagram depicting the amplification of targeted (captured) libraries according to some embodiments of the methods of the disclosure.

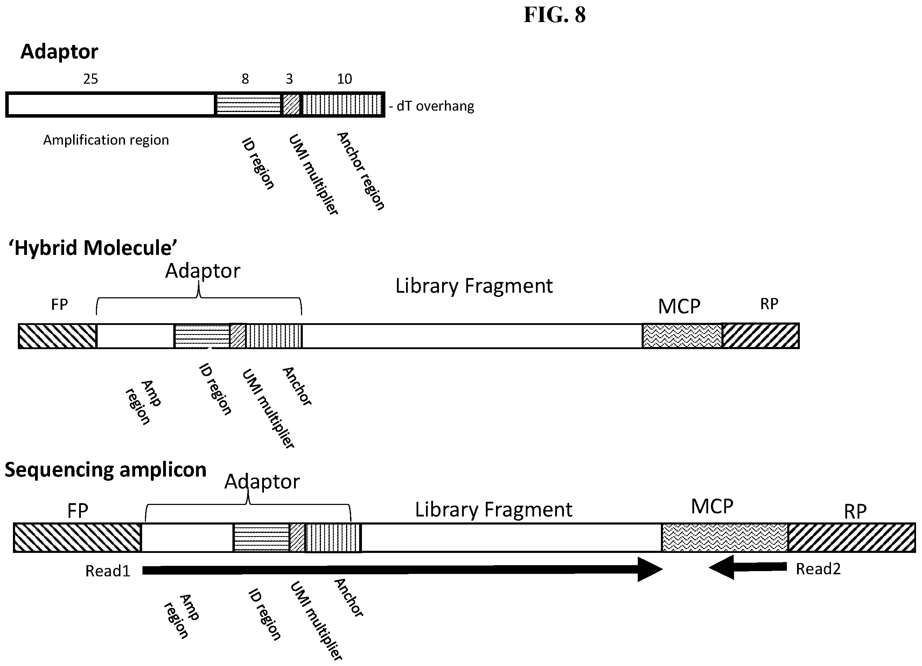

**Fig. 8** is a schematic diagram comparing the adaptor molecule, the "hybrid molecule" and the sequencing amplicon (for NGS). The adaptor molecule comprises, from 5' to 3', a 25-nucleotide amplification region, an 8-nucleotide ID region, a 3-nucleotide UMI multiplier region, a 10-nucleotide anchor region, and a dT overhang at the 3' terminus. The hybrid molecule comprises, from 5' to 3', a forward primer (FP), an adaptor region (comprising an amplification region, an ID region, a UMI multiplier, and an anchor region), a library fragment, a multifunctional capture probe (MCP), and a reverse primer (RP). The FP and RP are used for amplification of the hybrid molecule. As shown in the figure, for sequencing Read 1, sequencing is initiated at the start of the amplification region and proceeds 5' to 3' along the sequencing amplicon. For Read 2, sequencing is initiated at the end of the multifunctional capture probe, and proceeds 3' to 5' along the sequencing amplicon.
**Fig. 9** is a graph depicting the adaptor anchor distribution for comparator and automatable processes.
**Fig. 10A-10B** provide a pair of graphs depicting the high efficiency attachment of adaptors to DNA fragments in an illustrative process of cfDNA library construction. FIG. 10A provides data for Bioanalyzer traces of input cfDNA; FIG. 10B provides data for Bioanalyzer traces of cfDNA after library construction, indicating a majority (> 50%) of the cfDNA comprises 2 barcodes (adaptors).

### DETAILED DESCRIPTION

The compositions and methods of the disclosure solve a previously long-felt but unmet need for multiplexed nucleic acid detection and sequencing, as well as an automatable process to increase efficiency of the overall process, enabling high-throughput analyses. The compositions and methods of the disclosure may be used with various next-generation sequencing (NGS) processes.

The speed and accuracy of automatable DNA library preparation and sequencing processes is particularly important for the rapid detection and diagnosis of late-staged diseases, including cancer, and the early detection of highly infectious diseases prior to transmission. A genetic disease may be treatable, or even preventable at its onset, given a rapid and accurate detection. Moreover, monitoring of treatment efficacy often requires rapid and accurate results, for tracking biomarkers of disease progression or remission thereof.

The disclosure provides adaptors and methods for high efficiency construction of genetic libraries and genetic analysis that allow for automation. In addition to analyses for diagnostic purposes, the methods and compositions in the disclosure may be used in the analysis of any nucleic acid sample. As one example, the methods and compositions of the disclosure may be used in population-scale sequencing of one or more species to identify genetic variation at a population level, for example to address questions in the fields of evolutionary, agricultural, and biological research.

Particularly in those circumstances when highly multiplexed reactions on a large number of samples would optimally be performed in parallel, the compositions and methods of the disclosure provide the efficiency to perform analyses over a large population of samples, for example to trace the origins of disease or infection.

The compositions and methods of the disclosure are designed to minimize the steps required to detect and analyze nucleic acid fragments. Moreover, the compositions and methods of the disclosure are designed to simplify the manipulation of samples from one step to another, in some circumstances allowing multiple steps to occur sequentially in the same reaction vessel. Additionally, the compositions and methods of the disclosure may be used in smaller reaction volumes compared to other commercial processes, thereby reducing dilution of genetic material. This is particularly important when the starting genetic material is scarce or limited, for example when using cell free DNA (cfDNA) or ancient DNA.

In some embodiments, the disclosure provides adaptor designs and methods of using the same that allow detection of multiple types of DNA changes, including (but not limited to) copy number changes, single nucleotide variants, (SNVs), short (less than 40 bp) insertions and deletions (indels), and genomic rearrangements, for example gene fusions such as oncogenic gene fusions, inversions, translocations.

In some embodiments, the disclosure provides methods of preparing tagged DNA libraries according to a streamlined workflow. These methods are particularly useful for high throughput processing and automation, for example, using sample handling robotics for NGS library preparations, enrichment of genetic loci of interest by target capture processes, sequencing of the genetic materials and for genetic analyses.

Use of the disclosed compositions in the disclosed methods is surprisingly effective in increasing cloning efficiency, improving uniform adaptor distribution, and improving performance in terms of greater depth/coverage of sequence reads as well as genomic equivalents.

As a result, the methods provided in the instant disclosure have at least the following superior properties, as compared to standard workflows (such as non-automatable workflows): reduced number of steps, shorter processing time, lower risk for operator error, reduced number of reagents, smaller reaction volumes, and lower cost, thereby making commercialization and automation of such methods and workflows feasible.

In some embodiments of the methods of this disclosure, the methods are referred to as automatable processes. Although the compositions and methods of the disclosure are designed for use on an automated device, the compositions and methods of the disclosure are not required to be automated and, for clarity, could also be performed by non-automated means or on non-automated devices. To provide a basis for comparison, the disclosure provides a "comparator" process- a process not specifically designed for automation or for use with an automated device. When aligned, for example, with the comparator process of the disclosure, the "automatable" processes of the disclosure eliminate several steps while preserving the desired result of a multiplexed nucleic acid detection and analysis.

In some embodiments of the compositions and methods of the disclosure, end-repair is performed in a single step; adaptor ligation is performed in a single step; and extension and amplification of adaptor-tagged DNA fragments is performed in a single step. In some embodiments, the automatable process also reduces the time for library preparation, reduces number of reagents used, and reduces the volume for reactions. In particular, the reduction of reaction volume facilitates automation because a smaller reaction volume can be performed in microtiter plates or tube strips that can be handled by sampling robots. **Fig. 4A-4C** provides schematic diagrams outlining an exemplary comparator process for generating an adaptor-tagged DNA library and an automatable process of the disclosure for generating an adaptor-tagged DNA library of the disclosure.

### Definitions

Unless otherwise defined in the disclosure, scientific and technical terms used in this application shall have the meanings that are commonly understood by those of ordinary skill in the art. Generally, nomenclature used in connection with, and techniques of, chemistry, molecular biology, cell and cancer biology, immunology, microbiology, pharmacology, and protein and nucleic acid chemistry, described in the disclosure, are those well-known and commonly used in the art.

As used in the disclosure, the following terms have the meanings ascribed to them unless specified otherwise.

The articles "a, " "an, " and "the" are used in the disclosure to refer to one or to more than one (*i.e.* to at least one) of the grammatical object of the article. By way of example, "an element" means one element or more than one element.

The use of the alternative (*e.g.,* "or") should be understood to mean either one, both, or any combination thereof of the alternatives.

The term "and/or" should be understood to mean either one, or both of the alternatives.

As used in the disclosure, the term "about" or "approximately" refers to a quantity, level, value, number, frequency, percentage, dimension, size, amount, weight or length that varies by as much as 15%, 10%, 9%, 8%, 7%, 6%, 5%, 4%, 3%, 2% or 1% to a reference quantity, level, value, number, frequency, percentage, dimension, size, amount, weight or length. In some embodiments, the term "about" or "approximately" refers a range of quantity, level, value, number, frequency, percentage, dimension, size, amount, weight or length ± 15%, ± 10%, ± 9%, ± 8%, ± 7%, ± 6%, ± 5%, ± 4%, ± 3%, ± 2%, or ± 1% about a reference quantity, level, value, number, frequency, percentage, dimension, size, amount, weight or length.

As used in the disclosure, the term "isolated" means material that is substantially or essentially free from components that normally accompany it in its native state. In some embodiments, the term "obtained" or "derived" is used synonymously with isolated.

A "subject," "individual," or "patient" as used herein, includes any animal that exhibits a symptom of a condition that can be detected or identified with compositions contemplated herein. Suitable subjects include laboratory animals (such as mouse, rat, rabbit, or guinea pig), farm animals (such as horses, cows, sheep, pigs), and domestic animals or pets (such as a cat or dog). In some embodiments, the subject is a mammal. In some embodiments, the subject is a non-human primate and, in preferred embodiments, the subject is a human.

Transitional phrases such as "comprising", "consisting essentially of", and "consisting of" take the customary definitions as outlined in the United States Patent and Trademark Office's Manual of Patent Examining Procedure (See MPEP 2111.03).

### Adaptor Design

To achieve high throughput capabilities amenable to automation (e.g., using sample-handling robotics), the adaptors and related methods of the disclosure include, in some embodiments, the following features: (i) one-step attachment; (ii) high efficiency attachment; (iii) uniform adaptor distribution; (iv) accommodation of sample multiplexing and sample identification; (v) high number of unique molecule identifiers (UMIs). For example, some embodiments of the adaptors and methods of the disclosure provide the following:

One-step attachment: In some embodiments, the full-length multifunctional adaptor may be attached to the DNA fragment in one step. A "full length" multifunctional adaptor may comprise at least 4 regions: a first amplification region comprising a polynucleotide sequence capable of serving as a primer recognition site, a second multifunctional ID region comprising a unique molecule identifier (UMI), a third region comprising a UMI multiplier, and a fourth region comprising an anchor region. Attaching a full-length multifunctional adaptor may eliminate the need for adaptor ligation in a stepwise manner where the anchor is attached first, then the remaining regions of the adaptor are attached (for example, see the stepwise manner of adaptor ligation in the comparator process of **Fig. 4A**).

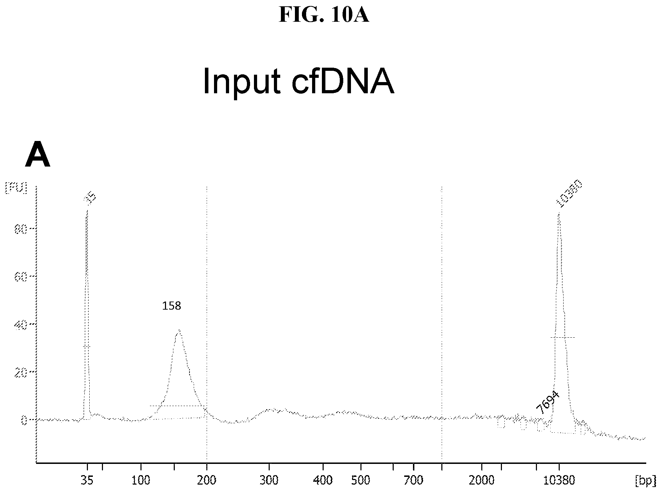

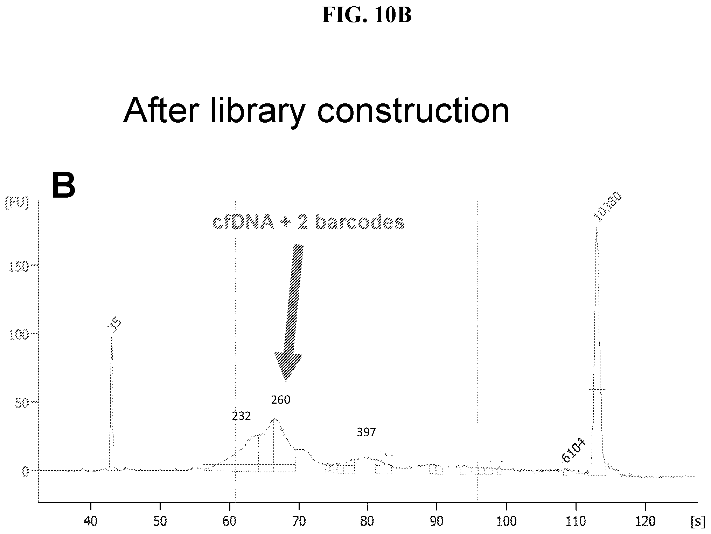

High efficiency attachment: In some embodiments, the multifunctional adaptors may be attached to the DNA fragments with high efficiency. For the purposes of the instant disclosure, the efficiency of adaptor attachment refers to the conversion rate of input DNA fragments to adaptor-tagged DNA library molecules. For example, a DNA fragment may be identified by the ID region of an attached adaptor, and a DNA fragment would not be identifiable using the ID region if it was not attached to an adaptor. Accordingly, a higher efficiency of adaptor attachment may lower the number of input DNA fragments lost in the library conversion process. This is particularly useful in situations where the quantity of available DNA is limited, for example in samples analyzed in connection with many oncology applications and other genetic diseases (e.g. multiple sclerosis, rheumatoid arthritis, Alzheimer's disease). In such situations the occurrence of DNA alterations (e.g., single nucleotide variants (SNVs), indels, copy number changes, DNA rearrangements, optionally related to tumors/cancers) are typically infrequent and thus can be difficult to detect. Highly efficient attachment of adaptors of the disclosure to these DNA fragments may facilitate capture of such infrequent variations. In some embodiments, at least 50% of input DNA fragments are converted into adaptor-tagged DNA library molecules by attachment of the multifunctional adaptors. **Fig.10** provides data for high efficiency attachment of adaptors to DNA fragments in an exemplary process of cfDNA library construction.

Uniform adaptor distribution: Bioinformatics analysis may analyze intra-sample probe performance and inter-sample probe performance. Performance fluctuation between adaptor pools across samples may negatively impact the sensitivity of the analysis. Uniform adaptor distribution in the tagged DNA libraries and capture probe libraries as measured by sequence reads is desirable. In some embodiments, there is the possibility of bias in the distribution of adaptors in the adaptor-tagged DNA library, where some adaptors may be less efficient in ligating to the DNA fragments or may be less efficiently amplified compared to the others in the adaptor pool. This may result in fewer amplicons and fewer reads of those less efficient adaptors during sequencing. While such biased distribution may be tolerated or compensated for by increasing the amount of the less-efficient adaptors in the adaptor pool to provide a more balanced representation of the adaptors in the tagged DNA library and sequencing reads, the compositions and methods of the disclosure provide the option of eliminating such compensation. The adaptors and methods disclosed herein can provide the unexpected benefit of achieving uniform adaptor distribution, wherein each adaptor is represented at roughly the same ratio in sequencing results. This uniform adaptor distribution provides increased sensitivity.

In some embodiments, the uniform adaptor distribution may be achieved by having multiple types of anchor regions that are all represented in each pool of adaptors.

In some embodiments, the uniform adaptor distribution may be achieved by having unique ID regions (each ID region identifies both the sample and the DNA fragment attached thereto) randomly selected for each pool of adaptors.

Accommodation of sample multiplexing and sample identification: To achieve sample multiplexing (i.e., the ability to run different samples simultaneously), in some embodiments, pools of unique adaptors are constructed where each unique adaptor within the same pool is attached to the same sample. From a sequence counting perspective, it is beneficial for each unique adaptor of the pool of adaptors to possess essentially identical behavior to all other adaptors in the pool. In order to achieve this, in some embodiments, each ID region has a Hamming distance of 2 between the ID region any other ID region, thus reducing the chance for a read to be spuriously assigned to the wrong sample. In some embodiments, each pool of adaptors is split into further pools that are paired with specific anchor regions, allowing for further reduction in the possibility of an error in sample de-multiplexing. For example, in an 8mer tag with Hamming distance of 2, the total number of possible sequences is 16,384. The term "paired" when used with respect to two different polynucleotide sequences or regions of DNA comprising different polynucleotide sequences, means that the two different polynucleotide sequences or regions of DNA comprising different polynucleotide sequences are present on the same polynucleotide. For example, if a particular ID region of DNA is said to be paired to a particular amplification region of DNA, it is meant that the ID region and the amplification tag are present on the same DNA polynucleotide molecule.

High number of Unique Molecule Identifiers (UMIs): While it is beneficial in general for adaptors to be functionally equivalent from a molecular biology perspective, it is also desirable that adaptors possess a very large number of unique molecule identifiers (UMIs) (≥ 10,000) that augment the identification of unique genomic fragments. In this context, by "augment," it is meant that the power of identifying a uniquely derived fragment is increased. Each genomic clone fragment has a particular pair of fragmentation sites corresponding to the position in the genomic sequence where the double-strand DNA was cleaved. This cleavage site may be used to differentiate unique genomic clones, because each clone is likely to possess a different cleavage site. However, in libraries that possess thousands of independent clones, uniquely derived fragments may often possess the exact same cleavage sites. Genomic clones (i.e., fragments) sharing the same cleavage site may be classified as either unique or as redundant with respect to other clone sequences derived from the same sample. By attaching adaptors that introduce a high diversity of sequence tags, different genomic clones sharing the same cleavage site are more likely to be identified as unique. In some embodiments, the UMI is created by a combination of the multifunctional ID region with the UMI multiplier. That is, each unique DNA fragment can be identified by the combination of the multifunctional ID region and the UMI multiplier (i.e. identified by the UMI). Furthermore, the combination of the UMI and the cleavage site create a unique molecular identifier element (UMIE), which facilitates the classification of sequence reads as redundant reads or unique reads. Some embodiments contemplate that the UMI multiplier could comprise longer or shorter sequences to increase or lower the overall UMI complexity. In some embodiments, each unique DNA fragment may be identified by the multifunctional ID region alone.

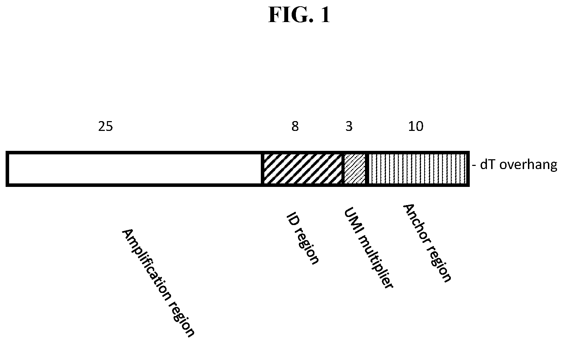

The terms "adaptor", "multifunctional adaptor", and "adaptor module" may be used interchangeably, and refer to a short single- or double-stranded oligonucleotide that can be ligated to an end of a DNA or RNA molecule. Typically, the adaptors described herein comprise at least five elements: (i) a 3' terminal overhang; (ii) an amplification region comprising a polynucleotide sequence capable of serving as a primer recognition site; (iii) a unique multifunctional ID region; (iv) a unique molecule identifier (UMI) multiplier; and (v) an anchor region comprising a polynucleotide sequence that is at least partially complementary to the non-ligation strand oligonucleotide. **Fig. 1** provides an exemplary composition of a multifunctional adaptor according to some embodiments as described herein (only the ligation strand oligonucleotide is shown).

In some embodiments, the adaptor comprises one or more amplification regions, one or more multifunctional ID regions, one or more UMI multipliers, and one or more anchor regions. In some embodiments, the adaptor comprises, in order from 5' to 3', an amplification region, a multifunctional ID region, a UMI multiplier, an anchor region, and a 3' terminal overhang.

In some embodiments, the UMI multiplier is contained within the multifunctional ID region, and the adaptor comprises, in order from 5' to 3', an amplification region, an integrated multifunctional ID region /UMI multiplier region, an anchor region, and a 3' terminal overhang.

In some embodiments, the multifunctional adaptor comprises one or more amplification regions, one or more ID regions, one or more UMI multipliers, one or more anchor regions, and one or more nucleotides in the 3 overhang that are efficient ligation substrates. In additional embodiments, the adaptor module further comprises one or more sequencing primer binding sites.
The structure of illustrative adaptors that may be used in the compositions and methods of the disclosure are provided in Table 2 and Table 3. For example, in some embodiments, the ligation strand of an adaptor may comprise the following structure: AMP-ID Region/UMI Multiplier-ACGTATGCCA (SEQ ID NO: 2)-3'dT. In some embodiments, the ligation strand of an adaptor may comprise the following structure: AMP-ID Region/UMI Multiplier-CTAGCGTTAC (SEQ ID NO: 3)-3'dT. In some embodiments, the ligation strand of an adaptor may comprise the following structure: AMP-ID Region/UMI Multiplier-GATCGACATG (SEQ ID NO: 4)-3'dT. In some embodiments, the ligation strand of an adaptor may comprise the following structure: AMP-ID Region/UMI Multiplier-TGCATCAGGT (SEQ ID NO: 5) - 3'dT. In some embodiments, the non-ligation strand anchor region of an adaptor may comprise the sequence TGGCATACGT (SEQ ID NO: 6). In some embodiments, the non-ligation strand anchor region of an adaptor may comprise the sequence GTAACGCTAG_(SEQ ID NO: 7). In some embodiments, the non-ligation strand anchor region of an adaptor may comprise the sequence CATGTCGATC (SEQ ID NO: 8). In some embodiments, the non-ligation strand anchor region of an adaptor may comprise the sequence ACCTGATGCA (SEQ ID NO: 9).

In some embodiments, an adaptor may comprise a ligation strand with a 3' dT overhang. In some embodiments, the ligation strand with a 3' dT overhang may comprise any one of the sequences shown in Table 3. For example, the ligation strand with a 3' dT overhang may comprise a sequence of any one of SEQ ID NO: 10 to 69. The "NNN" within these ligation strand sequences represents a 3-nucleotide UMI multiplier wherein each N may be selected from any one of A, G, C, or T. In some embodiments, the ligation strand with a 3' dT overhang may comprise a sequence of any one of SEQ ID NO: 10 to 69 with 1, 2, 3, 4, 5, 6, 7, 8, 9, 10 or more nucleotide substitutions.

### Ligation Strand Oligonucleotide

The terms "ligation strand oligonucleotide" and "ligation strand" are used interchangeably.

The disclosure provides, in some embodiments, a ligation strand oligonucleotide comprising (i) a 3' terminal overhang; (ii) an amplification region comprising a polynucleotide sequence capable of serving as a primer recognition site; (iii) a unique multifunctional ID region; (iv) a unique molecule identifier (UMI) multiplier; and (v) an anchor region comprising a polynucleotide sequence that is at least partially complementary to the non-ligation strand oligonucleotide.

In some embodiments, the ligation strand oligonucleotide is not phosphorylated at the 5' terminus.

In some embodiments, the ligation strand oligonucleotide is between about 30 nucleotides and about 70 nucleotides in length. In some embodiments, the ligation strand oligonucleotide is between about 35 and about 65 nucleotides, between about 40 and about 60 nucleotides, or between about 40 and about 50 nucleotides in length. In some embodiments, the ligation strand oligonucleotide is about 47 nucleotides in length.

In some embodiments, the ligation strand oligonucleotide is between 30 nucleotides and 70 nucleotides in length. In some embodiments, the ligation strand oligonucleotide is between 35 and 65 nucleotides, between 40 and 60 nucleotides, or between 40 and 50 nucleotides in length. In some embodiments, the ligation strand oligonucleotide is 47 nucleotides in length.

### Non-Ligation Strand

The terms "non-ligation strand oligonucleotide" and "non-ligation strand" are used interchangeably.

The non-ligation strand oligonucleotide is capable of hybridizing to a region at the 3' end of the ligation strand oligonucleotide and forming a duplex therewith. The non-ligation strand is complementary to at least a portion of the ligation strand in order to form the duplex. This duplex structure may facilitate ligation of the 5' end of the dsDNA to the ligation strand.

In some embodiments, the non-ligation strand is not phosphorylated. Lack of phosphorylation of the non-ligation strand may prevent the non-ligation strand from attaching to the 3' end of the DNA fragment and may reduce the formation of adaptor dimers.

In some embodiments, the non-ligation strand may optionally comprise a modification at its 3' terminus that prevents ligation to the 5' end of the dsDNA fragment and/or adaptor dimer formation. In some embodiments, the modification is a chemical modification.

### 3' Terminal Overhang

The term "3' terminal overhang" refers to one or more nucleotide overhangs or tails at the 3' terminus of a polynucleotide.

In some embodiments, the ligation strand oligonucleotide comprises a dT overhang at the 3' terminus.

In some embodiments, the 3' terminal overhang (e.g., a dT tail) aids in the ligation of the ligation strand to ligate to the 5' end of the DNA fragment, in order to drive the efficient ligation of the multifunctional adaptor to the DNA fragment having a complementary overhang (e.g. dA-overhang/tail).

### Amplification Region

The term "amplification region" refers to an element of the adaptor molecule that comprises a polynucleotide sequence capable of serving as a primer recognition site. The primer recognition site can be for any primer that is suitable for any amplification known in the art, such as methods disclosed in Fakruddin et al. "Nucleic acid amplification: Alternative methods of polymerase chain reaction" J Pharm Bioallied Sci. 2013 Oct-Dec; 5(4): 245-252. For example, such amplification methods may include PCR (polymerase chain reaction), LAMP (loop-mediated isothermal amplification), NASBA (nucleic acid sequence-based amplification), SDA (strand displacement amplification), RCA (rolling circle amplification), LCR (ligase chain reaction).

In some embodiments, an adaptor comprises an amplification region that comprises one or more primer recognition sequences for single-primer amplification of a DNA library. In some embodiments, the amplification region comprises one, two, three, four, five, six, seven, eight, nine, ten, or more primer recognition sequences for single-primer amplification of a DNA library. In some embodiments, the amplification region comprises a PCR primer binding site for an ACA2 primer (SEQ ID NO: 70).

In some embodiments, the amplification region is between about 5 and about 50 nucleotides, between about 10 and about 45 nucleotides, between about 15 and about 40 nucleotides, or between about 20 and about 30 nucleotides in length. In some embodiments, the amplification region is about 10 nucleotides, about 11 nucleotides, about 12 nucleotides, about 13 nucleotides, about 14 nucleotides, about 15 nucleotides, about 16 nucleotides, about 17 nucleotides, about 18 nucleotides, about 19 nucleotides, about 20 nucleotides, about 21 nucleotides, about 22 nucleotides, about 23 nucleotides, about 24 nucleotides, about 25 nucleotides, about 26 nucleotides, about 27 nucleotides, about 28 nucleotides, about 29 nucleotides, about 30 nucleotides, about 31 nucleotides, about 32 nucleotides, about 33 nucleotides, about 34 nucleotides, about 35 nucleotides, about 36 nucleotides, about 37 nucleotides, about 38 nucleotides, about 39 nucleotides, or about 40 nucleotides or more in length. In some embodiments, the amplification region is about 25 nucleotides in length.

In some embodiments, the amplification region is between 5 and 50 nucleotides, between 10 and 45 nucleotides, between 15 and 40 nucleotides, or between 20 and 30 nucleotides in length. In some embodiments, the amplification region is 10 nucleotides, 11 nucleotides, 12 nucleotides, 13 nucleotides, 14 nucleotides, 15 nucleotides, 16 nucleotides, 17 nucleotides, 18 nucleotides, 19 nucleotides, 20 nucleotides, 21 nucleotides, 22 nucleotides, 23 nucleotides, 24 nucleotides, 25 nucleotides, 26 nucleotides, 27 nucleotides, 28 nucleotides, 29 nucleotides, 30 nucleotides, 31 nucleotides, 32 nucleotides, 33 nucleotides, 34 nucleotides, 35 nucleotides, 36 nucleotides, 37 nucleotides, 38 nucleotides, 39 nucleotides, or 40 nucleotides or more in length. In some embodiments, the amplification region is 25 nucleotides in length.

### Multifunctional ID Region

The terms "multifunctional ID region" and "ID region" are used interchangeably and refer to an element of the adaptor that comprises a polynucleotide sequence that uniquely identifies the particular DNA fragment as well as the sample from which it was derived.

In some embodiments, the multifunctional ID region is between about 3 and about 50 nucleotides, between about 3 and about 25 nucleotides, or between about 5 and about 15 nucleotides in length. In some embodiments, the multifunctional ID region is about 3 nucleotides, 4 nucleotides, about 5 nucleotides, about 6 nucleotides, about 7 nucleotides, about 8 nucleotides, about 9 nucleotides, about 10 nucleotides, about 11 nucleotides, about 12 nucleotides, about 13 nucleotides, about 14 nucleotides, about 15 nucleotides, about 16 nucleotides, about 17 nucleotides, about 18 nucleotides, about 19 nucleotides, or about 20 nucleotides or more in length. In some embodiments, the multifunctional ID region is about 8 nucleotides in length.

In some embodiments, the multifunctional ID region is between 3 and 50 nucleotides, between 3 and 25 nucleotides, or between 5 and 15 nucleotides in length. In some embodiments, the multifunctional ID region is 3 nucleotides, 4 nucleotides, 5 nucleotides, 6 nucleotides, 7 nucleotides, 8 nucleotides, 9 nucleotides, 10 nucleotides, 11 nucleotides, 12 nucleotides, 13 nucleotides, 14 nucleotides, 15 nucleotides, 16 nucleotides, 17 nucleotides, 18 nucleotides, 19 nucleotides, or 20 nucleotides or more in length. In some embodiments, the multifunctional ID region is 8 nucleotides in length.

In some embodiments, the multifunctional ID region comprises one of between about 2 and about 10,000 unique nucleotide sequences, between about 50 and about 500 unique nucleotide sequences, or between about 100 and about 400 unique nucleotide sequences. In some embodiments, the multifunctional ID region of each multifunctional adaptor of the plurality of multifunctional adaptors comprises one of about 60 unique nucleotide sequences.

In some embodiments, the multifunctional ID region comprises one of between 2 and 10,000 unique nucleotide sequences, between 50 and 500 unique nucleotide sequences, or between 100 and 400 unique nucleotide sequences. In some embodiments, the multifunctional ID region of each multifunctional adaptor of the plurality of multifunctional adaptors comprises one of 60 unique nucleotide sequences.

In some embodiments, the multifunctional adaptor comprises one of between 64 and 2,560,000 unique nucleotide sequences.

In some embodiments, pre-specified pools (a plurality) of adaptors are provided. Such pre-specified pools are used to represent a single sample. That is, each adaptor sequence in each pool of adaptor oligonucleotides is distinct from each adaptor sequence in every other pool used to identify other samples. One of skill in the art will recognize the number of distinct oligonucleotides in pre-specified pools that are possible for the adaptor oligonucleotides will depend on the length of the multifunctional ID region and/or the UMI multiplier. "Plurality" can refer to a plurality of the same adaptor module or to a pool of different adaptor modules.

In some embodiments, the ID region identifies the individual sample, for example, the genomic library source. In some embodiments, each sample is assigned a plurality (pre-specified pool) of between about 64 and about 2.5 million unique adaptors. In some embodiments, each sample is assigned a plurality (pre-specified pool) of between 64 and 2.5 million unique adaptors. In some embodiments, each sample is assigned a plurality (pre-specified pool) of about 3,840 unique adaptors. In some embodiments, each sample is assigned a plurality (pre-specified pool) of 3,840 unique adaptors. In some embodiments, each sample is assigned a plurality (pre-specified pool) of between about 1 and about 60 unique adaptors. In some embodiments, each sample is assigned a plurality (pre-specified pool) of between 1 and 60 unique adaptors. In some embodiments, each sample is assigned a plurality (pre-specified pool) of 60 unique adaptors, wherein each pre-specified pool of 60 unique adaptors is further divided into 4 sets (each set comprising 15 unique adaptors), wherein each multifunctional ID region of one set is paired to one of the 4 anchor sequences. Therefore, the sample can be identified by the combination of the multifunctional ID region and the anchor region.

In some embodiments, the nucleotide sequence of each multifunctional ID region is discrete from the nucleotide sequence of any other multifunctional ID regions of the plurality of multifunctional adaptors by Hamming distance of at least two (meaning at least two base changes are required to change one ID region into another).

In some embodiments, the ID region identifies the individual DNA fragment to which it is attached, thus the ID regions also serve as fragment tags that can, in one example, enumerate clone diversity for copy number analysis.

In some embodiments, the multifunctional ID region is 8 nucleotides in length and comprises one of 240 unique nucleotide sequences, and the UMI multiplier is 3 nucleotide sequences in length, therefore to total number of unique adaptor sequences would be 240 x 4³= 3840= 15,360. Thus, in some embodiments, each sample may be assigned a set of adaptors ranging from 1~ 15,360 unique adaptors for DNA fragment identification.

In some embodiments, the multifunctional ID region is 8 nucleotides in length and comprises one of 60 unique nucleotide sequences, and the UMI multiplier is 3 nucleotides in length, and each nucleotide sequence is discrete from any other sequence of the 3840 unique nucleotide sequences by Hamming distance of at least two.

Thus, the multifunctional ID region contributes to the identification of both the sample the DNA fragment. This is in stark contrast to the current systems that are used in the art which use a randomly generated tag to identify the sequence and a separate barcode or sequencer indexing to allow for sample multiplexing.

### UMI Multiplier

To further augment the diversity of possible sequence tags (UMIs), UMI multipliers are included in the adaptors. A UMI multiplier is a short sequence of random bases (e.g., NNN, wherein each N may be selected from any one of A, C, G, and T) which, when combined with a UMI, increases the diversity of and total number of adaptor sequences in an adaptor pool. In some embodiments, an adaptor comprises a UMI multiplier, wherein the UMI multiplier is adjacent to or contained within the ID region. In some embodiments, an adaptor comprises an ID region that is eight nucleotides in length and a UMI multiplier that is three nucleotides in length. In some embodiments, the UMI multiplier is three nucleotides in length and comprises one of 64 possible sequences. In some embodiments, the UMI multiplier is located adjacent to or contained within the ID region.

In some embodiments, each nucleotide position of the UMI multiplier can comprise any one of adenine, guanine, cytosine, or thymine. Thus, in some embodiments, a UMI multiplier comprising n number of nucleotides can comprise any of 4ⁿ possible nucleotide sequences. In some embodiments, the UMI multiplier is one nucleotide in length and comprises one of four possible sequences. In some embodiments, the UMI multiplier is two nucleotides in length and comprises one of sixteen possible sequences. In some embodiments, the UMI multiplier is three nucleotides in length and comprises one of 64 possible sequences. In some embodiments, the UMI multiplier is four nucleotides in length and comprises one of 256 possible sequences. In some embodiments, the UMI multiplier is five nucleotides in length and comprises one of 1,024 possible sequences. In some embodiments, the UMI multiplier is six nucleotides in length and comprises one of 4,096 possible sequences. In some embodiments, the UMI multiplier is seven nucleotides in length and comprises one of 16,384 possible sequences. In some embodiments, the UMI multiplier is eight nucleotides in length and comprises one of 65,536 possible sequences. In some embodiments, the UMI multiplier is nine nucleotides in length and comprises one of 262,144 possible sequences. In some embodiments, the UMI multiplier is ten or more nucleotides in length and comprises one of 1,048,576 or more possible sequences.

In some embodiments, the UMI multiplier is at least 1, at least 2, at least 3, at least 4, at least 5, at least 6, at least 7, at least 8, at least 9, or at least 10 nucleotides in length. In some embodiments, the UMI multiplier is between 1 and 5 nucleotides in length.

### Anchor Region

The terms "anchor region" and "anchor sequence" are used interchangeably and refer to a polynucleotide sequence that is at least partially complementary to the non-ligation strand oligonucleotide. In some embodiments, the anchor region is also referred to as the linker. The anchor region may, in some embodiments, comprise one or more of the following properties:
(1) Each anchor sequence may be part of a pool of two or more unique anchor types that collectively represent each of the four possible DNA bases at each site within extension; this feature, balanced base representation, is useful to calibrate proper base calling in sequencing reads in some embodiments. The number of total types of anchor sequences should match the total number of detection modes. For example, four colors are detected in Illumina^{®} sequencing, therefore four types of anchor sequences may be used. To achieve maximum sensitivity, each detection mode may be utilized. The compositions and methods of the disclosure may be used in any mode of detection known in the art, including but not limited to light-based detection, enzyme-based detection, and magnetic detection.
(2) Each anchor sequence may be composed of only two of four possible bases, and these are specifically chosen to be either and equal number of A + C or an equal number of G + T; an anchor sequence formed from only two bases reduces the possibility that the anchor sequence will participate in secondary structure formation that would preclude proper adaptor function.
(3) Because each anchor sequence is composed of equal numbers of A + C or G + T, each anchor sequence may shares roughly the same melting temperature and duplex stability as every other anchor sequence in the pool.
(4) Each type of anchor sequence (ending in either A/T/G/C) may be approximately equally distributed in the sequencing reads, for example in approximately equimolar amounts (i.e. about 25% of the pool have adaptor sequences ending in A, about 25% ending in T, about 25% ending in G, and about 25% ending in C).

In some embodiments, adaptor modules are mixed with DNA fragments in equimolar amounts of adaptors containing different anchor types (e.g. equimolar amounts of anchor 1, anchor 2, anchor 3, anchor 4) to provide a more even adaptor distribution. Exemplary anchor sequences include, but are not limited to: Anchor 1 ACGTATGCCA (SEQ ID NO: 2); Anchor 2 CTAGCGTTAC (SEQ ID NO: 3); Anchor 3 GATCGACATG (SEQ ID NO: 4); and Anchor 4 TGCATCAGGT (SEQ ID NO: 5).

In some embodiments, adaptor sequences end with a T nucleotide at the 3' terminus (3' T overhang). In some embodiments, adaptors have TT as the last 2 nucleotides of the 3' terminus. In some embodiments, adaptors have AT, CT, or GT as the last 2 nucleotides of the 3' terminus.

In general, an ideal distribution of anchor types would result in each anchor type having an identical distribution percentage (i.e., 100% divided by the number of anchor types), resulting in a "uniform" distribution of different adaptors comprising different anchors among all DNA fragments. For example, an ideal distribution of four anchor types would result in about 25% distribution of each anchor type. In some embodiments, the anchor sequences of a given pool have a distribution percentage of between about 5% to about 75% (i.e., the distribution % of the most infrequent anchor type is about 5% and the distribution % of the most frequent anchor type is about 75%). In some embodiments, each anchor sequence of a given pool has a distribution % of about 50%, about 34%, about 28%, about 27%, about 23%, about 14%, or about 9%. In some embodiments, the distribution percentage of each anchor sequence of an automatable process as described herein is at least 5%, at least 10%, at least 25%, or at least 20% closer to the corresponding ideal distribution percentage compared to a comparator process (i.e., a process which is not designed for automation, See Table 4).

In some embodiments, the plurality of adaptors comprises a 3' dT overhang and may include higher amounts of adaptors having anchors with TT as the last two 2 nucleotides of the 3' terminus. Such adaptors may be 1X, 2X, 4X, 5X, 6X, 7X, 8X, 9X, or more than 10X the amount of other anchor types in the pool, resulting in more even distribution of adaptors in sequencing reads.

In some embodiments, the plurality of adaptors can comprise more than one anchor sequence. For example, a plurality of adaptors may contain 4 different anchor sequences are used simultaneously. These anchor sequences may also be used during sample de-multiplexing to lower errors. In addition, the position of sequences within the read is fixed, and therefore the ID regions and anchor should have a fixed position within a sequencing read in order to pass inclusion filters for downstream consideration.

In some embodiments, the anchor region is between 1 and 50 nucleotides in length. In some embodiments, the anchor region is between 4 and 40 nucleotides in length. In some embodiments, the anchor region is between 5 and 25 nucleotides in length. In some embodiments, the anchor region is at least 4 nucleotides, at least six nucleotides, at least 8 nucleotides, at least 10 nucleotides, at least 12 nucleotides, at least 14 nucleotides, or at least 16 nucleotides in length. In some embodiments, the anchor region is 10 nucleotides in length.

### Illustrative Workflow

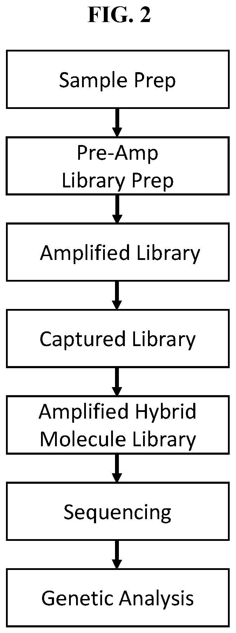

An illustrative workflow of the methods in the disclosure is provided below and depicted in **Fig. 2**

### 1. End-repair

In some embodiments, input DNA fragments are converted to "end-repaired DNA fragments" such that the end-repaired DNA fragments possess 5' phosphate groups and 3' dA nucleotide overhangs in a single reaction mixture (single step). A commercially available kit (e.g. NEB Ultra II End Repair^{®}/dA tailing module E7546L) may be used to end-repair the DNA fragments or one or more of the individual enzymes and buffers as disclosed may be combined for preparation of end-repaired DNA fragments that possess 5' phosphate groups and 3' dA nucleotide overhangs.

In some embodiments, the end-repair reaction volume is lower than 50 µL.

### 2. Adaptor ligation

In some embodiments, a pool of multifunctional adaptors is ligated to end-repaired dsDNA fragments from one or more samples (multiplexing), resulting in adaptor attachment to 5' end of dsDNA fragments.

In some embodiments, the ligation reaction volume is lower than 100 µL.

In some embodiments, the adaptor-tagged DNA fragments are isolated and washed in reaction volumes lower than 100 µL.

### 3. Extension

In some embodiments, 3' dA-tailed DNA fragments are extended from 3' end of the DNA fragment, displacing the non-ligation strand using the ligation strand that is attached to the 5' end of the DNA fragment as template to make contiguous adaptor-tagged dsDNA fragments that are suitable for amplification. The collection of "contiguous adaptor-tagged dsDNA fragments" is the unamplified adaptor-tagged DNA library. **Fig. 3** provides a schematic diagram depicting an exemplary process of generating the adaptor-tagged DNA library according to some methods of the disclosure.

In some embodiments, the extension reaction volume is lower than 100 µL.

### 4. Amplification

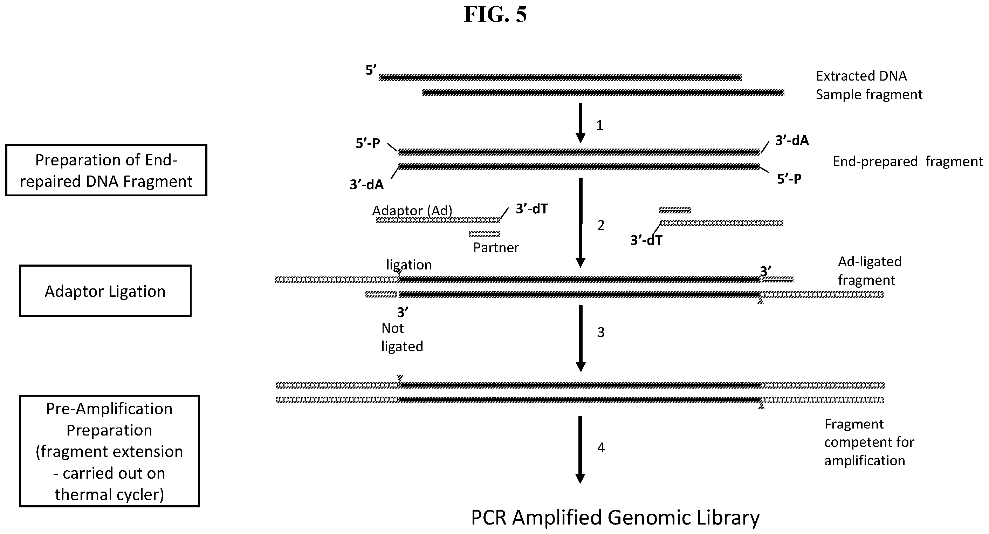

In some embodiments, the unamplified adaptor-tagged DNA library is PCR amplified with a single primer which recognizes the amplification primer binding site in the amplification region of the adaptor, resulting in amplified adaptor-tagged DNA library. In some embodiments, the single primer comprises the sequence of SEQ ID NO: 70. For the purposes of the instant disclosure, "amplified adaptor-tagged-DNA library", "amplified tagged DNA library" and "library product amplified (LPA)" are used interchangeably. **Fig. 5** provides a schematic diagram depicting the preparation and amplification of adaptor-tagged DNA libraries according to the methods in the disclosure.

In some embodiments, the amplification reaction volume is lower than 100 µL.

In some embodiments, the amplified tagged DNA library is further isolated and washed in volumes lower than 100 µL.

In some embodiments, amplification is carried out according to the conditions provided in Table 7. For example, in some embodiments, the methods described herein comprise: 1) carrying out amplification of a library that had been divided into 2 separate tubes under an annealing temperature of 69 °C; 2) carrying out amplification of a library that had been divided into 2 separate tube under an annealing temperature of 65 °C; or 3) carrying out the amplification without dividing the library (in 1 tube) under an annealing temperature of 65 °C.

### 5. Capture and isolation of Genetic locus/loci

In some embodiments, a multifunctional capture probe with its tail region (first region) duplexed at least partially to a biotinylated partner oligonucleotide is hybridized to the amplified tagged DNA library to form one or more capture probe/adaptor-tagged DNA complexes.

### 6. Isolation of amplified tagged DNA molecules-capture probe module complex

In some embodiments, the capture probe/adaptor-tagged DNA complexes (i.e., captured fragments) are separated from un-hybridized fragments (i.e., uncaptured fragments) using magnetic streptavidin-beads.

### 7. Capture Probe Extension

In some embodiments, bead-supported capture probes in the complex are extended from the 3' end using the tagged DNA fragments as templates, creating adaptor-tagged hybrid nucleic acid molecules (hybrid molecules), wherein each hybrid molecule comprises the capture probe and a complement of the adaptor-tagged DNA fragment that is 3' from where the capture probe hybridized to the targeted genetic sequence.

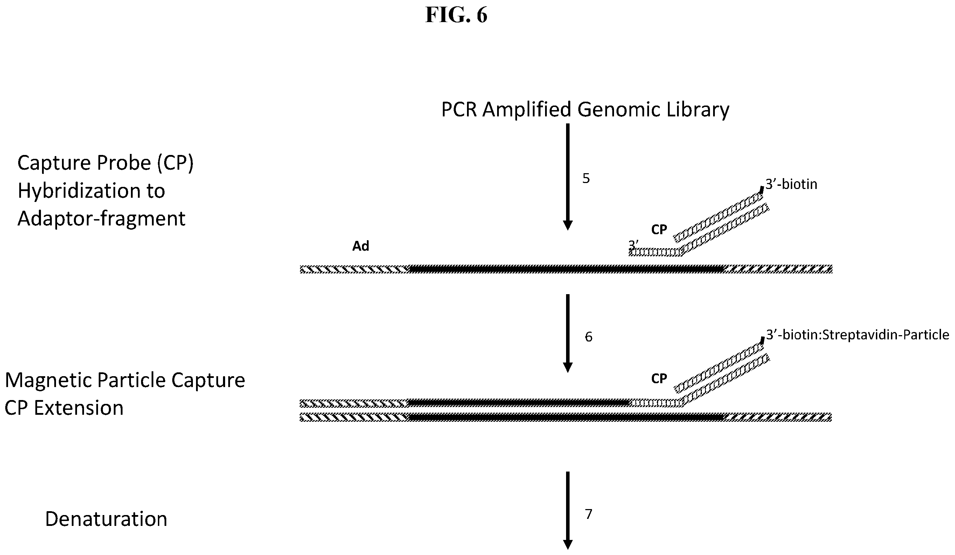

In some embodiments, denaturation releases the hybrid molecule from the magnetic bead into the solution. **Fig. 6** provides a schematic diagram depicting capture probe hybridization and extension.

### 8. Amplification of hybrid molecules

In some embodiments, a Forward Primer (FP) (SEQ ID NO: 71) hybridizes to the primer binding site in the amplification region of the adaptor tag within the hybrid molecules and extends 5' -> 3' the capture probe using the hybrid molecule as template to make a contiguous double stranded hybrid molecule.

In some embodiments, the FP-extended strand in the contiguous double stranded hybrid molecule is denatured and a Reverse Primer (RP) (SEQ ID NO: 72) hybridizes to denatured FP-extended molecule/strand at the incorporated capture probe module tail region in the hybrid molecule.

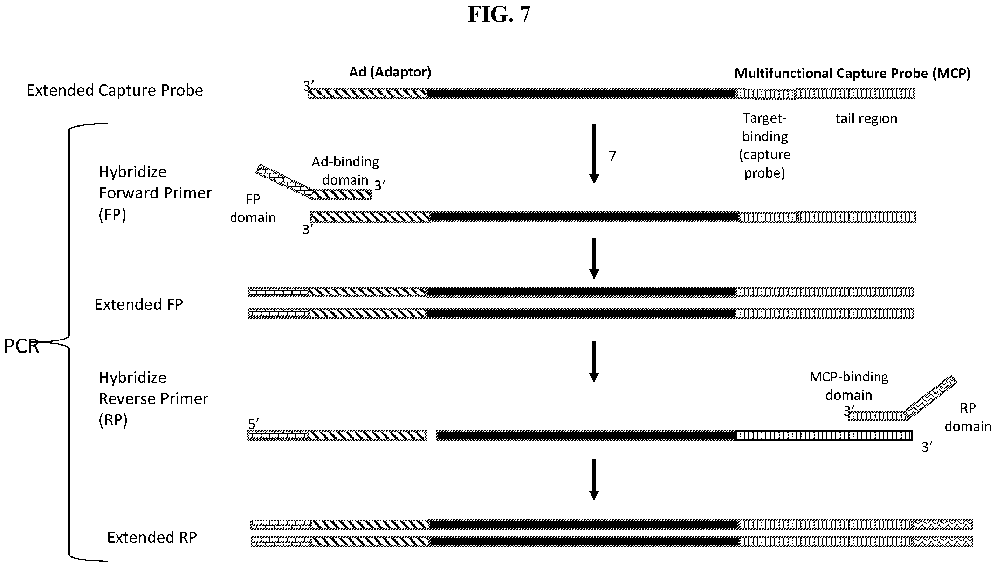

In some embodiments, the RP extends 5' -> 3' using the hybrid molecule as template to make a contiguous double stranded hybrid molecule that is ready for Illumina^{®} sequencing or sequencing by any other known methods in the art. **Fig. 7** provides a schematic diagram depicting the amplification of targeted (captured) libraries.

In some embodiments, the sequencing primers are different from one another. In some embodiments, each end of the hybrid molecule preferentially includes a sequencing primer binding site that is recognized by a sequencing primer such as P5 and P7 sequencing primers, or other Illumina^{®} sequencing primers. The collection of amplified sequencing ready hybrid molecules is referred to as "targeted genetic library", "targeted library", or "Probe captured library (PCL)".

In some embodiments, the amplification reaction volume is lower than 100 µL.

In some embodiments, the amplified hybrid molecules are isolated and washed.

### 9. Sequencing

In some embodiments, next generation Sequencing (NGS) of the amplified hybrid molecules is performed using Illumina^{®} NextSeq^{®} 550 sequencer.

In some embodiments, NGS can be performed on the unamplified adaptor-tagged DNA library, amplified tagged DNA library, library of hybrid molecules (unamplified targeted library), and/or amplified targeted library.

In some embodiments, a sequencing Read 1 (151 nt in length) and a sequencing Read 2 (17 nt in length) is conducted using custom-made forward and reverse sequencing primers.

Sequencing was performed on Illumina NextSeq550, following manufacturer's instructions, using custom primers, Forward Seq Primer and Reverse Seq Primer 62.
Forward Seq Primer: 
CAAGCAGAAGACGGCATACGAGATGTGACTGGCACGGGACCAGAGAATTCGAAT
ACA (SEQ ID NO: 73)
Reverse Seq Primer 62:
   GTGACTGGCACGGGACCAGAGAATTCGAATACA (SEQ ID NO: 74)

### 10. Genetic analysis

In some embodiments, the hybrid molecules, or any of the molecules generated according to methods of the disclosure that can be subject to sequencing using amplification primers or sequencing primers) are subjected to genetic analysis.

In some embodiments, sequence Reads 1 and 2 are used for genetic analysis.

In some embodiments, bioinformatics analysis is performed to identify genetic variants, such as copy numbers, SNVs, Indels, gene and chromosome rearrangements.

### Detailed Methods

### 1. Adaptor-tagged DNA Library Preparation

In some embodiments, methods contemplated in the disclosure comprise generating an adaptor-tagged DNA library comprising treating the dsDNA fragments with one or more end-repair enzymes to generate end-repaired DNA and attaching one or more adaptors to each end of the end-repaired DNA to generate the adaptor-tagged DNA library.

### DNA Sample Preparation

As used in the disclosure, the term "DNA" refers to deoxyribonucleic acid. In some embodiments, the term DNA refers to genomic DNA, recombinant DNA, synthetic DNA, or cDNA. In some embodiments, DNA refers to genomic DNA or cDNA. In some embodiments, the DNA comprises a "target region." DNA libraries contemplated herein include genomic DNA libraries and cDNA libraries constructed from RNA, *e.g.,* an RNA expression library. In some embodiments, the DNA libraries comprise one or more additional DNA sequences and/or tags.

As used in the disclosure, the terms "circulating DNA," "circulating cell-free DNA," and "cell-free DNA" are often used interchangeably and refer to DNA that is extracellular DNA, DNA that has been extruded from cells, or DNA that has been released from necrotic or apoptotic cells. This term is often used in contrast to "cellular genomic DNA" or "cellular DNA," which are used interchangeably herein and refer to genomic DNA that is contained within the cell (i.e. the nuclease) and is only accessible to molecular biological techniques such as those described herein, by lysing or otherwise disrupting the integrity of the cell.

The compositions and methods provided in the disclosure is particularly suited for preparation of precious biological samples that are typically obtained in small amounts, such as cancer tissue biopsy sample or "liquid biopsy" samples which are typically fluids (e.g. urine, CSF, whole blood, plasma, saliva).

In some embodiments, the amount of DNA used for making a library can be any suitable amount. In some embodiments, the amount is between about 1 pg and about 500 ng, between about 1 ng and about 400 ng, between about 5 ng and about 300 ng, between about 10 ng and about 250 ng, or between about 20 ng and about 200 ng. In some embodiments the DNA amount is between about 5 ng and about 50 ng.

In some embodiments, the amount of DNA used for making a library can be any suitable amount. In some embodiments, the amount is between 1 pg and 500 ng, between 1 ng and 400 ng, between 5 ng and 300 ng, between 10 ng and 250 ng, or between 20 ng and 200 ng. In some embodiments the DNA amount is between 5 ng and 50 ng.

In some embodiments, the methods and compositions contemplated in the disclosure use dsDNA that is selected from cell free DNA (cfDNA), genomic DNA (gDNA), complementary DNA (cDNA), mitochondrial DNA, methylated DNA, or demethylated DNA.

In some embodiments, methods of genetic analysis contemplated herein comprise generating a DNA library comprising treating cfDNA or fragmented cellular genomic DNA with one or more end-repair enzymes to generate end-repaired DNA and attaching one or more adaptors to each end of the end-repaired DNA to generate the DNA library.

In some embodiments, the methods and compositions contemplated herein are designed to efficiently analyze, detect, diagnose, and/or monitor change in copy number using genomic DNA as an analyte. In some embodiments, copy number analysis is performed by generating a genomic DNA library from genomic DNA obtained from a test sample, *e.g.,* a biological sample such as a tissue biopsy. In some embodiments, the genomic DNA is circulating or cell free DNA. In some embodiments, the genomic DNA is cellular genomic DNA.

In some embodiments, genomic DNA is obtained from a tissue sample or biopsy taken from a tissue, including but not limited to, bone marrow, esophagus, stomach, duodenum, rectum, colon, ileum, pancreases, lung, liver, prostate, brain, nerves, meningeal tissue, renal tissue, endometrial tissue, cervical tissue, breast, lymph node, muscle, and skin. In some embodiments, the tissue sample is a biopsy of a tumor or a suspected tumor. In some embodiments, the tumor is cancerous or suspected of being cancerous. In some embodiments, the tissue sample comprises cancer cells or cells suspected of being cancerous.

Methods for purifying genomic DNA from cells or from a biologic tissue comprised of cells are well known in the art, and the skilled artisan will recognize optimal procedures or commercial kits depending on the tissue and the conditions in which the tissue is obtained. Some embodiments contemplate that purifying cellular DNA from a tissue will require cell disruption or cell lysis to expose the cellular DNA within, for example by chemical and physical methods such as blending, grinding or sonicating the tissue sample; removing membrane lipids by adding a detergent or surfactants which also serves in cell lysis, optionally removing proteins, for example by adding a protease; removing RNA, for example by adding an RNase; and DNA purification, for example from detergents, proteins, salts and reagents used during cell lysis step. DNA purification may be performed by precipitation, for example with ethanol or isopropanol; by phenol-chloroform extraction.

In some embodiments, cellular DNA obtained from tissues and/or cells are fragmented prior to and or during obtaining, generating, making, forming, and/or producing a genomic DNA library as described in the disclosure. One of skill in the art will understand that there are several suitable techniques for DNA fragmentation, and is able to recognize and identify suitable techniques for fragmenting cellular DNA for the purposes of generating a genomic DNA library for DNA sequencing, including but not limited to next-generation sequencing. Some embodiments contemplate that cellular DNA can be fragmented into fragments of appropriate and/or sufficient length for generating a library by methods including but not limited to physical fragmentation, enzymatic fragmentation, and chemical shearing.

Physical fragmentation can include, but is not limited to, acoustic shearing, sonication, and hydrodynamic shear. In some embodiments, cellular DNA is fragmented by physical fragmentation. In some embodiments, cellular DNA is fragmented by acoustic shearing or sonication. Some embodiments contemplate that acoustic shearing and sonication are common physical methods used to shear cellular DNA. The Covaris^{®} instrument (Woburn, MA) is an acoustic device for breaking DNA into 100-5kb bp. Covaris^{®} also manufactures tubes (gTubes) which will process samples in the 6-20 kb for Mate-Pair libraries. The Bioruptor^{®} (Denville, NJ) is a sonication device utilized for shearing chromatin, DNA and disrupting tissues. Small volumes of DNA can be sheared to 150-1kb in length. Hydroshear^{®} from Digilab^{®} (Marlborough, MA) utilizes hydrodynamic forces to shear DNA. Nebulizers (Life Technologies^{®}, Grand Island, NY) can also be used to atomize liquid using compressed air, shearing DNA into 100-3kb fragments in seconds. Nebulization is low cost, but the process can cause a loss of about 30% of the cellular DNA from the original sample. In some embodiments, cellular DNA is fragmented by sonication.

Enzymatic fragmentation can include, but is not limited to, treatment with a restriction endonuclease, e.g. DNase I, or treatment with a nonspecific nuclease. In some embodiments, cellular DNA is fragmented by enzymatic fragmentation. In some embodiments, the cellular DNA is fragmented by treatment with a restriction endonuclease. In some embodiments, the cellular DNA is fragmented by treatment with a nonspecific nuclease. In some embodiments, the cellular DNA is fragmented by treatment with a transposase. Some embodiments contemplate that enzymatic methods to shear cellular DNA into small pieces include DNAse I, a combination of maltose binding protein (MBP)-T7 Endo I and a non-specific nuclease Vibrio vulnificus (Vvn) nuclease, New England Biolabs^{®}'s (Ipswich, MA) Fragmentase^{®} and Nextera^{™} tagmentation technology (Illumina^{®}, San Diego, CA). The combination of non-specific nuclease and T7 Endo synergistically work to produce non-specific nicks and counter nicks, generating fragments that disassociate 8 nucleotides or less from the nick site. Tagmentation uses a transposase to simultaneously fragment and insert adaptors onto double stranded DNA.

Chemical fragmentation can include treatment with heat and divalent metal cation. In some embodiments, genomic DNA is fragmented by chemical fragmentation. Some embodiments contemplate that chemical shear is more commonly used for the breakup of long RNA fragments as opposed to genomic DNA. Chemical fragmentation is typically performed through the heat digestion of DNA with a divalent metal cation (magnesium or zinc). The length of DNA fragments can be adjusted by increasing or decreasing the time of incubation.

In some embodiments, genomic DNA may be fragmented by sonication using an ultra-sonicator (Covaris^{®}) on a suitable for generating 200 bp fragments.

In some embodiments, the generated fragments may be further purified and size-selected using "double-sided" bead purification with paramagnetic AMPure XP^{®} beads (Beckman^{®}).

In some embodiments, mixtures of sheared cell line DNA can be at various ratios as suitable for the purpose of the studies, and they can be blended with WT cfDNA from female and/or male subjects (to account for genes on X and/or Y chromosomes) to produce lab-generated samples with single nucleotide variants (SNVs) such as single gene polymorphisms (SNPs), insertions and/or deletions (Indels), gene arrangements such as translocations, fusions, inversions, duplications (copy number changes) and other variants at defined allele frequencies (AF).

In some embodiments, the methods and compositions contemplated in the disclosure use dsDNA that is obtained from a low pass whole genome library, an amplicon library, a whole exome library, a cDNA library, or a methylated DNA library.

In some embodiments, the methods and compositions of the disclosure use any one of the DNA samples described in Table 1 as an analyte. For example, in some embodiments, the methods and compositions contemplated in the disclosure use cell-free DNA (cfDNA) as an analyte. In some embodiments, the DNA sample to be used as an analyte comprises synthetic DNA, genomic DNA, or a mixture thereof. In some embodiments, the DNA sample to be used as an analyte comprises HRD (Homologous Repair Deficient) gene variants, such as variants in any one of the following genes: ATM, BRCA1, BRCA2, FANCA, HDAC2, PALB2, ERBB2, TP53, EML4-Alk, EGFR. In some embodiments, the DNA sample to be used as an analyte comprises lung cancer gene variants. In some embodiments, the DNA sample to be used as an analyte comprises DNA from a cell line, such as NA12878, PC-3 or H2228.

In some embodiments, about 10 to about 250 ng of sample DNA is used for analysis. For example, in some embodiments, about 1 to about 100 ng, about 1 to about 50, or about 1 to about 25 ng of DNA is used. In some embodiments, about 20, about 25, or about 50 ng of DNA are used.

In some embodiments, the size distribution of cfDNA to be used as an analyte ranges from about 150 bp to about 180 bp fragments. In some embodiments, the size distribution of cfDNA ranges from 150 bp to 180 bp fragments. Fragmentation of cfDNA may be the result of endonucleolytic and/or exonucleolytic activity and presents a formidable challenge to the accurate, reliable, and robust analysis of cfDNA. Another challenge for analyzing cfDNA is its short half-life in the blood stream, on the order of about 15 minutes. Without wishing to be bound to any particular theory, the present disclosure contemplates, in part, that analysis of cfDNA is like a "liquid biopsy" and is a real-time snapshot of current biological processes.

Moreover, because cfDNA is not found within cells and may be obtained from a number of suitable sources including, but not limited to, biological fluids and stool samples, it is not subject to the existing limitations that plague next generation sequencing analysis, such as direct access to the tissues being analyzed.

In some embodiments, methods of genetic analysis contemplated herein comprise generating a cfDNA library comprising treating cfDNA with one or more end-repair enzymes to generate end-repaired cfDNA and ligating one or more adaptors to each end of the end-repaired cfDNA to generate the cfDNA library.

Illustrative examples of biological fluids that are suitable sources from which to isolate cfDNA in some embodiments include, but are not limited to amniotic fluid, blood, plasma, serum, semen, lymphatic fluid, cerebral spinal fluid, ocular fluid, urine, saliva, mucous, and sweat.

In some embodiments, the biological fluid is blood or blood plasma.

In some embodiments, commercially available kits and other methods known to the skilled artisan can used to isolate cfDNA directly from the biological fluids of a subject or from a previously obtained and optionally stabilized biological sample, e.g., by freezing and/or addition of enzyme chelating agents including, but not limited to EDTA, EGTA, or other chelating agents specific for divalent cations.

In some embodiments, cell free DNA or genomic DNA (e.g. cfDNA or gDNA) isolated from immortalized cells harboring gene variants (Coriell Institute for Medical Research or SeraCare Life Sciences, Inc.) can be used for NGS library construction.

In some embodiments, cell-free DNA may be extracted from plasma samples using a QIAmp DSP Circulating NA kit (Qiagen).

### Single-step DNA End-Repair

While DNA fragments of the disclosure may be obtained in a processed form, the methods of the disclosure allow for the processing of biological samples to obtain DNA fragments that are amenable for ligation to adaptors of the disclosure. For example, a processed form of a DNA fragment of the disclosure includes, but is not limited to, a DNA fragment comprising one or more of a blunted end, a blunted 3' end, a blunted 5' end, an deoxyribonucleic acid adenine (dA)-tail, a dA-tail at a 3' end, a dA-tail at a 5' end, a phosphorylated nucleic acid, a phosphorylated nucleic acid at a 3' end, and a phosphorylated nucleic acid at a 5' end.

In some embodiments, "end repair" may be performed to generate DNA fragments that are dephosphorylated, internally damage repaired, blunt ended, 5' phosphorylated, or to generate DNA fragments with 3' overhangs.

In some embodiments of the methods of the disclosure that include processing of DNA fragments, one or more of a DNA repair reaction to blunt an end, an A-tailing reaction and a phosphorylation reaction may be performed in a single step.

In some embodiments, generating a genomic DNA library comprises the end-repair of isolated cfDNA or fragmented cellular DNA. The fragmented cfDNA or cellular DNA is processed by end-repair enzymes to generate end-repaired cfDNA with blunt ends, 5'-overhangs, or 3'-overhangs. In some embodiments, the end-repair enzymes can yield for example. In some embodiments, the end-repaired cfDNA or cellular DNA contains blunt ends. In some embodiments, the end-repaired cellular DNA or cfDNA is processed to contain blunt ends. In some embodiments, the blunt ends of the end-repaired cfDNA or cellular DNA are further modified to contain a single base pair overhang. In some embodiments, end-repaired cfDNA or cellular DNA containing blunt ends can be further processed to contain adenine (A)/thymine (T) overhang. In some embodiments, end-repaired cfDNA or cellular DNA containing blunt ends can be further processed to contain adenine (A)/thymine (T) overhang as the single base pair overhang. In some embodiments, the end-repaired cfDNA or cellular DNA has non-templated 3' overhangs. In some embodiments, the end-repaired cfDNA or cellular DNA is processed to contain 3' overhangs. In some embodiments, the end-repaired cfDNA or cellular DNA is processed with terminal transferase (TdT) to contain 3' overhangs. In some embodiments, a G-tail can be added by TdT. In some embodiments, the end-repaired cfDNA or cellular DNA is processed to contain overhang ends using partial digestion with any known restriction enzymes (e.g., with the enzyme Sau3A, and the like).

In some embodiments, dephosphorylation of DNA fragment can be performed by thermolabile phosphatases such as alkaline phosphatases. Commercial examples include APex^{™} Heat Labile Alkaline phosphatase, NTPhos^{™} Thermolabile Phosphatase, KT^{™} Thermolabile Phosphatase and shrimp alkaline phosphatase (SAP).

In some embodiments, internal DNA damage may be repaired by one or more repair enzymes that may repair internal damage in the DNA fragments. Examples include Taq DNA ligase, Endonuclease IV, Bst DNA polymerase Fpg, Uracil-DNA Glycolase (UDG), T4 PDG, and endonuclease VIII. In some embodiments, all the foregoing enzymes may be used. A commercially available cocktail of the foregoing enzymes (e.g. the PreCR Enzyme kit) may be used or a cocktail may be prepared by addition of one or more the individual enzymes in any combination. In some embodiments, the DNA internal damage repair may not be performed.

In some embodiments, internal DNA damage repair, end-repair, and terminal transferase (TdT) for dA-tailing may be performed in a single step and single reaction mixture. In some embodiments, a commercially available kit such as the PreCR enzyme kit or Quick blunt kit from NEB can be used for the single step reaction.

In some embodiments, DNA end repairing may be done by use of one or more end-repair enzymes to create blunt ended DNA fragments. The enzymes may include 3'- 5' exonuclease, 5' - 3' DNA polymerase (e.g. Klenow fragment), and 5' FLAP endonuclease.

In some embodiments, DNA end-repair, 5' phosphorylation, and terminal transferase (TdT) for dA-tailing may be performed in a single step and single reaction mixture to generate dsDNA fragments that are 5' phosphorylated with 3'-overhang ends, e.g., 5' phosphorylated and 3'dA-tailed. In some embodiments, commercially available kits such as the Next Ultra II End repair/dA-tailing kit from NEB can be used for the single step reaction.

In some embodiments, the present disclosure contemplates that appropriate amounts of fragmented DNA samples can be "single-step end-repaired", by combining into a single mixture enzymes and reagents for each of the following reactions: dephosphorylation, internal DNA damage repair, blunt end creation, 5' end phosphorylation, and 3' overhang creation. This single-step single-reaction process generates end-repaired double stranded DNA fragments having a 5' phosphorylated end and a 3' overhang. In some embodiments, the 3' overhang comprises a dA tail.

In some embodiments, the amount of DNA that can be end-repaired can be any suitable amount. In some embodiments, the amount of DNA to be end-repaired is between 1 ng and 500 ng, between 5 ng and 400 ng, between 10 ng and 300 ng, between 15 ng and 250 ng, or between 20 ng and 200 ng. In some embodiments, the amount of DNA to be end-repaired is between 20 ng and 50 ng.

### Adaptor Ligation to End-Repaired DNA

**In** some embodiments, a ligation step comprises ligating an adaptor module to the end-repaired cfDNA to generate a "tagged" cfDNA library. In some embodiments, a single adaptor module is employed. In some embodiments, two, three, four or five adaptor modules are employed. In some embodiments, an adaptor module of identical sequence is ligated to each end of the fragmented end-repaired DNA. In some embodiments, adaptor modules of non-identical sequences are ligated to the two ends of each fragmented end-repaired DNA.

Ligation of one or more adaptors contemplated herein may be carried out by methods known to those of ordinary skill in the art. In some embodiments, one or more adaptors contemplated herein are ligated to end-repaired cfDNA that comprises blunt ends. In some embodiments, one or more adaptors are ligated to end-repaired cfDNA that comprises complementary ends appropriate for the ligation method employed. In some embodiments, one or more adaptors are ligated to end-repaired cfDNA that comprises a 3' overhang.

In some embodiments, attaching the genomic DNA fragments to a plurality of adaptors includes the steps of attaching the end repaired cfDNA or cellular DNA fragments to an oligonucleotide containing at least a portion of an anchor region. In some embodiments, the oligonucleotide contains the whole anchor region. In some embodiments, the oligonucleotide is a DNA duplex comprising a 5' phosphorylated attachment strand duplexed with a partner strand, wherein the partner strand is blocked from attachment by chemical modification at its 3' end, and wherein the attachment strand is attached to the genomic DNA fragment. In some embodiments, the DNA fragments attached with at least a portion of the anchor region are then annealed with DNA oligonucleotides encoding the full-length adaptor sequences. In some embodiments, one or more polynucleotide kinases, one or more DNA ligases, and/or one or more DNA polymerases are added to the genomic DNA fragments and the DNA oligonucleotides encoding the full-length adaptor sequence. In some embodiments, the polynucleotide kinase is T4 polynucleotide kinase. In some embodiments, the DNA ligase is Taq DNA ligase. In some embodiments, the DNA polymerase is Taq polymerase. In some embodiments, the DNA polymerase is full length Bst polymerase.

In some embodiments, the adaptors and DNA fragments can be mixed with ligation buffer, reagents and ligation enzyme such as DNA ligases (e.g. T4 ligase or Taq ligase) and/or RNA ligases. Such ligases can be used for ligating the single stranded ligation strand with the 3' overhang as described above to a single stranded DNA fragment.

In some embodiments, the ligation strand of the multifunctional adaptor ligates to the 5' end of the dsDNA fragment in a single step via the complementation of the 3' terminal overhang of the ligation strand and the 3' overhang of the DNA fragment, while the non-ligation strand remains unattached to the 3' end of the DNA fragment.

In some embodiments, a ligation step comprises ligating a multifunctional adaptor with a dsDNA fragment to generate a multifunctional adaptor/dsDNA fragment complex. In some embodiments, a single adaptor is employed. In some embodiments, two, three, four or five adaptors are employed. In some embodiments, an adaptor module of identical sequence is attached to each end of the fragmented end-repaired DNA.

In some embodiments, the same adaptor is attached to both ends of the DNA fragment. In some embodiments, different adaptors are attached to different ends of the dsDNA fragment.

In some embodiments, a ligation step comprises:
(a) ligating a plurality of multifunctional adaptors with a plurality of dsDNA fragments to generate a plurality of multifunctional adaptor/dsDNA fragment complexes, wherein the multifunctional adaptor is any one of the multifunctional adaptors in the disclosure;
(b) contacting the adaptor/DNA fragment complexes from step (a) with one or more enzymes to form an adaptor-tagged DNA library comprising a plurality of contiguous double-stranded adaptor-tagged DNA fragments.

In some embodiments, the adaptor/DNA fragment complexes in step (b) is made into contiguous double stranded adaptor-tagged DNA fragments by DNA polymerase extension using the ligation strand as template.

In some embodiments, the unattached non-ligation strand is displaced by 5'-3' polymerase extension of the DNA fragment using the ligation strand as template. In some embodiments, the non-ligation strand may optionally comprise a modification at its 3' terminus that prevents ligation to the 5' end of the dsDNA fragment and/or adaptor dimer formation.

In some embodiments, the non-ligation strand is ligated to the 3' end of the DNA fragment by DNA polymerase nick-repair (nick translation), using the ligation strand as template.

In some embodiments, the dsDNA fragment is cell free DNA (cfDNA), genomic DNA (gDNA), complementary DNA (cDNA), mitochondrial DNA, or methylated DNA, or demethylated DNA.

In some embodiments, the plurality of dsDNA fragments is end-repaired prior to ligating with a plurality of multifunctional adaptors.

In some embodiments, the plurality of dsDNA fragments is obtained from a library selected from the list consisting of a low pass whole genome library, an amplicon library, a whole exome library, a cDNA library, or a methylated DNA library.

In some embodiments, the adaptor ligation period can be any period suitable for ligation. In some embodiments, the period is at least about 5 minutes. In some embodiments, the period is between about 5 minutes and about 72 hours. In some embodiments, the period is between about 5 minutes and about 2 hours. In some embodiments, the ligation period is less than about 1 hour, less than about 30 minutes, less than about 15 minutes, or less than about 10 minutes.

In some embodiments, the adaptor ligation period can be any period suitable for ligation. In some embodiments, the period is at least 5 minutes. In some embodiments, the period is between 5 minutes and 72 hours. In some embodiments, the period is between 5 minutes and 2 hours. In some embodiments, the ligation period is less than 1 hour, less than 30 minutes, less than 15 minutes, or less than 10 minutes.

In some embodiments, the adaptor ligation volume is one that is suitable for automation and for sample handling robotics. In some embodiments, the reaction volume is between about 1 µL and about 1000 µL, between about 1 µL and about 350 µL, between about 1 µL and about 200µL, between about 1 µL and about 100 µL, between about 1 µL and about 50 µL, is between about 5 µL and about 25 µL, between about 10 µL and about 40 µL, between about 20 µL and about 40 µL. In some embodiments, the reaction volume is about 100 µL. In some embodiments, the volume is about 30 µL.

In some embodiments, the adaptor ligation volume is one that is suitable for automation and for sample handling robotics. In some embodiments, the reaction volume is between 1 µL and 1000 µL, between 1 µL and 350 µL, between 1 µL and 200µL, between 1 µL and 100 µL, between 1 µL and 50 µL, is between 5 µL and 25 µL, between 10 µL and 40 µL, between 20 µL and 40 µL. In some embodiments, the reaction volume is 100 µL. In some embodiments, the volume is 30 µL.

In some embodiments, the, adaptor ligation can be done in strips of tubes or in microtiter plate wells or any other format suitable to allow for automated and/or high throughput processing.

In some embodiments, the adaptor amount can be any suitable concentration. In some embodiments, the concentration of adaptors is at least 0.01 µM. In some embodiments, the concentration of adaptors is between about 0.01 µM and about 200 µM, between about 0.01 µM and about 50 µM, between about 0.1 µM and about 50 µM, between about 0.2 µM and about 20 µM, between about 0.2 µM and about 10 µM, between about 1 µM and about 10 µM, or between about 2 µM and about 8 µM. In some embodiments, the adaptor concentration is at least about 2 µM. In some embodiments, the adaptor concentration is about 5 µM.

In some embodiments, the concentration of adaptors is between 0.01 µM and 200 µM, between 0.01 µM and 50 µM, between 0.1 µM and 50 µM, between 0.2 µM and 20 µM, between 0.2 µM and 10 µM, between 1 µM and 10 µM, or between 2 µM and 8 µM. In some embodiments, the adaptor concentration is at least 2 µM. In some embodiments, the adaptor concentration is 5 µM.

In some embodiments, the ligation reaction mixture can be incubated at temperatures between about 10 °C and about 30 °C. In some embodiments, the ligation reaction mixture can be incubated at about 20°C.

In some embodiments, the ligation reaction mixture can be incubated at temperatures between 10 °C and 30 °C. In some embodiments, the ligation reaction mixture can be incubated at 20°C.

In some embodiments, following ligation, adaptor-tagged DNA molecules can be isolated and washed. This can be done using DNA purification beads such as Ampure XP^{®} (Beckman^{®}) or Spectra mix such that Adaptor-DNA molecules remain attached to the beads and contaminating materials are washed away. The eluted clarified supernatant contains an isolated library comprising a plurality of adaptor-tagged DNA fragments. The supernatant containing the library can be transferred to a fresh PCR tube or microliter plate well for amplification.

### DNA Library Amplification

In some embodiments, the 5'-3' extension of the dsDNA fragments is first performed to generate contiguous adaptor-tagged dsDNA fragments, then the contiguous adaptor-tagged dsDNA fragments are amplified to generate an adaptor-tagged DNA library comprising a plurality of contiguous adaptor-tagged dsDNA fragments.

In some embodiments, the first step of 5'-3'extension of the dsDNA fragments forming contiguous adaptor-tagged dsDNA fragments and the second step of amplifying the contiguous adaptor-tagged dsDNA fragments is combined, in order to generate the adaptor-tagged DNA library in a single step. The adaptor-tagged dsDNA fragments in the DNA library are flanked by adaptors on both ends comprising the same amplification regions, wherein the sequences in the amplification region can function as amplification primer binding sites recognizable by a single amplification primer, such as a PCR amplification primer.

In some embodiments, the unattached non-ligation strand is displaced by 5'-3'polymerase extension of the DNA fragment using the ligation strand as template. In some embodiments, the non-ligation strand may optionally comprise a modification at its 3' terminus that prevents ligation to the 5' end of the dsDNA fragment and/or adaptor dimer formation.

In some embodiments, the non-ligation strand is ligated to the 3' end of the DNA fragment by DNA polymerase nick-repair, using the ligation strand as template.

In some embodiments, a DNA polymerase is used. The DNA polymerase may be thermophilic for PCR or thermostatic/isothermal amplification. In some embodiments, a master mix (MM) containing reagents and the enzymes for the 5'-3' extension and enzymes for the subsequent amplification are combined. Commercially available enzymes and reagent kits for such extension and amplification include, for example, NEB Ultra II 2X PCR Amplification^{®} kit (New England Biolabs^{®}), Hi-Fidelity Q5^{®} enzyme PCR (NEB), KAPA (Roche^{®}) KAPA 2X (Roche^{®}), TruSeq Nano^{®} (Thermofisher^{®}) AmpliTaq^{®} (Thermofisher^{®}).

In some embodiments, a portion of the adaptor-tagged DNA library will be amplified using standard PCR techniques with a single primer sequence driving amplification. In some embodiments, the single primer sequence is about 25 nucleotides, optionally with a projected Tm of ≥ 55° C under standard ionic strength conditions. In some embodiments, the single primer sequence is 25 nucleotides, optionally with a projected Tm of ≥ 55° C under standard ionic strength conditions. In some embodiments, the single amplification primer is complementary to a sequence within the amplification region of the adaptor module. In some embodiments, the single amplification primer comprises a sequence of TGCAGGACCAGAGAATTCGAATACA (SEQ ID NO: 70).

In some embodiments, amplification can be performed by any amplification known in the art, such as for PCR (polymerase chain reaction), LAMP (loop-mediated isothermal amplification), NASBA (nucleic acid sequence-based amplification), SDA (strand displacement amplification), RCA (rolling circle amplification), LCR (ligase chain reaction).

In some embodiments, during amplification some amplicons will form a stem-loop structures due to adaptors being ligated to both ends of the fragments. This strategy is efficient in preventing very short products (e.g., primer dimers) from being amplified and biasing the resulting library.

In some embodiments, an initial 3 min incubation cycle is performed to form a plurality of contiguous adaptor-tagged dsDNA fragments by ligation strand templated extension.

In some embodiments, PCR amplification is performed on of the plurality of contiguous adaptor-tagged dsDNA fragments to form an amplified Tagged DNA library.

In some embodiments, picograms of the plurality of contiguous adaptor-tagged dsDNA fragments is amplified into micrograms of DNA clones (adaptor-tagged DNA library), implying a 10,000-fold amplification. The amount of amplified product can be measured using methods known in the art, e.g., quantification on a Qubit 2.0 or Nanodrop instrument.

In some embodiments, the amplified adaptor-tagged DNA library can be isolated by use of DNA purification beads and washed with wash buffers, e.g., Tris-EDTA buffer (TEZ) pH 8.0. Clarified supernatant can be transferred to a fresh PCR tube or microtiter plate well or any other format suitable for automation and/or high throughput.

In general, it is preferable to use as few PCR cycles as possible to amplify libraries. In addition to reducing workflow time, this also limits the risk of introducing bias during PCR. A consequence of increased efficiency of the end repair, dA-Tailing and adaptor ligation in the methods of the disclosure is that fewer PCR cycles are required to achieve the library yields necessary for sequencing or other intermediate downstream workflows. The streamlining of the workflow and processes disclosed provides advantages such as: reduced turnaround time, reduced number of reagents, fewer instruments/machines used, and reduced expenses.

These above processes, adaptors, and tagged DNA libraries can be used for making capture probe libraries that are enriched for genetic loci of interest present in any test sample.

### 2. Target Capture and Isolation

In some embodiments, a method for genetic analysis of genomic DNA, e.g., genomic cellular or cfDNA, comprises quantitative genetic analysis of one or more target genetic loci of the DNA library clones. Quantitative genetic analysis comprises one or more of, or all of, the following steps: capturing DNA clones comprising a target genetic locus; amplification of the captured targeted genetic locus; sequencing of the amplified captured targeted genetic locus; and bioinformatic analysis of the resulting sequence reads. As used herein, the terms "DNA library clone" refer to a DNA library fragment wherein the combination of the adaptor and the genomic DNA fragment result in a unique DNA sequence (*e.g.,* a DNA sequence that can be distinguished from that of another DNA library clone).

The present disclosure contemplates, in part, a capture probe module designed to retain the efficiency and reliability of larger probes but that minimizes uninformative sequence generation in a genomic DNA library that comprises smaller DNA fragments, *e.g*., a cfDNA clone library.

The terms "multifunctional capture probe" and "capture probe module" are used interchangeably. In some embodiments, the "capture probe module" or "multifunctional capture probe" comprises a capture probe sequence and a tail sequence, wherein the capture probe sequence is capable of hybridizing to a target region in the tagged genetic DNA library. In some embodiments, a multifunctional capture probe comprises a first region capable of hybridizing to a partner oligonucleotide, wherein, optionally, the first region comprises a tail sequence comprising a PCR primer binding site; and a second region capable of hybridizing to a specific target region in the tagged genetic DNA library. The first region may also be termed a tail region, and the second region may also be termed the capture probe or capture probe sequence.

In some embodiments, a capture probe module comprises a tail sequence. As used herein, the term "tail sequence" refers to a polynucleotide at the 5' end of the capture probe module, which in some embodiments can serve as a primer binding site. In some embodiments, the capture probe comprises a sequencing primer binding site.

In some embodiments, the tail sequence is about 5 to about 100 nucleotides, about 10 to about 100 nucleotides, about 5 to about 75 nucleotides, about 5 to about 50 nucleotides, about 5 to about 25 nucleotides, or about 5 to about 20 nucleotides. In some embodiments, the third region is from about 10 to about 50 nucleotides, about 15 to about 40 nucleotides, about 20 to about 30 nucleotides or about 20 nucleotides, or any intervening number of nucleotides.

In some embodiments, the tail sequence is about 30 nucleotides, about 31 nucleotides, about 32 nucleotides, about 33 nucleotides, about 34 nucleotides, about 35 nucleotides, about 36 nucleotides, about 37 nucleotides, about 38 nucleotides, about 39 nucleotides, or about 40 nucleotides.

In some embodiments, the tail sequence is 5 to 100 nucleotides, 10 to 100 nucleotides, 5 to 75 nucleotides, 5 to 50 nucleotides, 5 to 25 nucleotides, or 5 to 20 nucleotides. In some embodiments, the third region is from 10 to 50 nucleotides, 15 to 40 nucleotides, 20 to 30 nucleotides or 20 nucleotides, or any intervening number of nucleotides.

In some embodiments, the tail sequence is 30 nucleotides, 31 nucleotides, 32 nucleotides, 33 nucleotides, 34 nucleotides, 35 nucleotides, 36 nucleotides, 37 nucleotides, 38 nucleotides, 39 nucleotides, or 40 nucleotides.

In some embodiments, an exemplary partner oligonucleotide can be: GTGAAAACCAGGATCAACTCCCGTGCCAGTCACATCTCAGATGAGCT (SEQ ID NO: 1) with a Biotin-TEG modification at the 3' end.

The contiguous adaptor-tagged DNA fragments (unamplified) and tagged DNA library (amplified) are each useful for a variety of sequencing-based genetic analyses including the preparation of libraries containing hybrid molecules enriched for one or more genetic loci of interest and may be unamplified or amplified library (a "targeted library").

The unamplified adaptor-tagged DNA fragments and/or amplified tagged DNA libraries, prepared as described above, can be hybridized to multifunctional capture probes modules to generate libraries targeted to specific genetic loci, i.e. targeted libraries. The adaptor-tagged DNA fragments can be hybridized with one or more capture probes. Each capture probe can target the same genetic loci in the adaptor-tagged DNA fragments or they may target different genetic loci in the adaptor-tagged DNA fragments. In some embodiments, a plurality of genetic loci in the amplified tagged DNA library fragments are targeted.

In some embodiments, the capture probes are used with genomic DNA library constructed from cellular DNA. In some embodiments, the capture probes are used with genomic DNA library constructed from cfDNA. Because the average size of cfDNA is about 150 to about 170 bp and is highly fragmented, some embodiments are directed compositions and methods contemplated herein comprise the use of high density and relatively short capture probes to interrogate DNA target regions of interest. In some embodiments, the capture probes are capable of hybridizing to DNA target regions that are distributed across all chromosomal segments at a uniform density. A set of such capture probes is referred to herein as "chromosomal stability probes." Chromosomal stability probes are used to interrogate copy number variations on a genome-wide scale in order to provide a genome-wide measurement of chromosomal copy number (*e.g*., chromosomal ploidy).

One particular concern with using high density capture probes is that generally capture probes are designed using specific "sequence rules." For example, regions of redundant sequence or that exhibit extreme base composition biases are generally excluded in designing capture probes. However, it has been discovered that the lack of flexibility in capture probe design rules does not substantially impact probe performance. In contrast, capture probes chosen strictly by positional constraint provided on-target sequence information; exhibit very little off-target and unmappable read capture; and yield uniform, useful, on-target reads with only few exceptions. Moreover, the high redundancy at close probe spacing more than compensates for occasional poor-performing capture probes.

In some embodiments, a target region is targeted by a plurality of capture probes, wherein any two or more capture probes are designed to bind to the target region within 10 nucleotides of each other, within 15 nucleotides of each other, within 20 nucleotides of each other, within 25 nucleotides of each other, within 30 nucleotides of each other, within 35 nucleotides of each other, within 40 nucleotides of each other, within 45 nucleotides of each other, or within 50 nucleotides or more of each other, as well as all intervening nucleotide lengths.

In some embodiments, the capture probe is about 25 nucleotides, about 26 nucleotides, about 27 nucleotides, about 28 nucleotides, about 29 nucleotides, about 30 nucleotides, about 31 nucleotides, about 32 nucleotides, about 33 nucleotides, about 34 nucleotides, about 35 nucleotides, about 36 nucleotides, about 37 nucleotides, about 38 nucleotides, about 39 nucleotides, about 40 nucleotides, about 41 nucleotides, about 42 nucleotides, about 43 nucleotides, about 44 nucleotides, or about 45 nucleotides.

In some embodiments, the capture probe is 25 nucleotides, 26 nucleotides, 27 nucleotides, 28 nucleotides, 29 nucleotides, 30 nucleotides, 31 nucleotides, 32 nucleotides, 33 nucleotides, 34 nucleotides, 35 nucleotides, 36 nucleotides, 37 nucleotides, 38 nucleotides, 39 nucleotides, 40 nucleotides, 41 nucleotides, 42 nucleotides, 43 nucleotides, 44 nucleotides, or 45 nucleotides.

In some embodiments, the capture probe is about 100 nucleotides, about 200 nucleotides, about 300 nucleotides, about 400 nucleotides, or about 100 nucleotides. In another embodiment, the capture probe is from about 100 nucleotides to about 500 nucleotides, about 200 nucleotides to about 500 nucleotides, about 300 nucleotides to about 500 nucleotides, or about 400 nucleotides to about 500 nucleotides, or any intervening range thereof.

In some embodiments, the capture probe is 100 nucleotides, 200 nucleotides, 300 nucleotides, 400 nucleotides, or 100 nucleotides. In another embodiment, the capture probe is from 100 nucleotides to 500 nucleotides, 200 nucleotides to 500 nucleotides, 300 nucleotides to 500 nucleotides, or 400 nucleotides to 500 nucleotides, or any intervening range thereof.

In a particular embodiment, the capture probe is 60 nucleotides. In another embodiment, the capture probe is substantially smaller than 60 nucleotides but hybridizes comparably, as well as, or better than a 60-nucleotide capture probe targeting the same DNA target region. In some embodiments, the capture probe is 40 nucleotides.

In some embodiments, the capture probe module comprises a specific member of a binding pair to enable isolation and/or purification of one or more captured fragments of a tagged and or amplified genomic DNA library (*e.g.,* a cellular or cfDNA library) that hybridizes to the capture probe. In some embodiments, the capture probe module is conjugate to biotin or another suitable hapten, *e.g*., dinitrophenol, digoxigenin.

In some embodiments, the capture probe module is hybridized to a tagged and optionally amplified DNA library to form a complex. In some embodiments, the multifunctional capture probe module substantially hybridizes to a specific genomic target region in the DNA library.

Hybridization or hybridizing conditions can include any reaction conditions where two nucleotide sequences form a stable complex; for example, the tagged DNA library and capture probe module forming a stable tagged DNA library -capture probe module complex. Such reaction conditions are well known in the art and those of skill in the art will appreciated that such conditions can be modified as appropriate, *e.g*., decreased annealing temperatures with shorter length capture probes. Substantial hybridization can occur when the second region of the capture probe complex exhibits 100%, 99%, 98%, 97%, 96%, 95%, 94%, 93%, 92% 91%, 90%, 89%, 88%, 85%, 80%, 75%, or 70% sequence identity, homology or complementarity to a region of the tagged DNA library.

In some embodiments, the capture probe (i.e., the region that hybridizes to the target sequence) is about 40 nucleotides and has an optimal annealing temperature of about 44° C to about 47° C.

In some embodiments, the capture probe (i.e., the region that hybridizes to the target sequence) is 40 nucleotides and has an optimal annealing temperature of 44° C to 47° C.

In some embodiments, the methods contemplated herein comprise isolating a tagged cfDNA library-capture probe module complex. In some embodiments, methods for isolating DNA complexes are well known to those skilled in the art (*See, e.g.,* Ausubel et al., Current Protocols in Molecular Biology, 2007-2012) and any methods deemed appropriate by one of skill in the art can be employed in connection with the methods of the instant disclosure. In some embodiments, the complexes are isolated using biotin-streptavidin isolation techniques.

### 3. Amplification of Targeted Libraries

In some embodiments, removal of the single stranded 3'-ends from the isolated capture probe/adaptor-tagged DNA complexes is contemplated. In some embodiments, the methods comprise 3'-5' exonuclease enzymatic processing of the isolated tagged DNA library-multifunctional capture probe module complex to remove the single stranded 3' ends.

In some other embodiments, the methods comprise performing 5'-3' DNA polymerase extension of multifunctional capture probe utilizing the isolated tagged DNA library fragments as template. Enzymes that are suitable for this extension process can be any thermophilic, thermostable DNA polymerase. Examples of commercially available DNA polymerases include high fidelity Q5 DNA polymerase (NEB^{®}), NEBNext Ultra PCR^{®}, NEBNext Ultra II PCR^{®} (NEB), and KAPA 2X^{®} (Roche^{®}).

In some other embodiments, the methods comprise creating a hybrid capture probe-isolated tagged DNA target molecule, *e.g.,* a tagged cfDNA target molecule or a tagged cellular DNA target molecule, through the concerted action of a 5' FLAP endonuclease, DNA polymerization and nick closure by a DNA ligase.

A variety of enzymes can be employed for the 3'-5' exonuclease enzymatic processing of the isolated tagged DNA library-multifunctional capture probe module complex. Illustrative examples of suitable enzymes, which exhibit 3'-5' exonuclease enzymatic activity, that can be employed in some embodiments include, but are not limited to: T4 or Exonucleases I, III, V (*See also,* Shevelev IV, Hübscher U., Nat Rev Mol Cell Biol. 3(5):364-76 (2002)). In some embodiments, the enzyme comprising 3'-5' exonuclease activity is T4 polymerase. In some embodiments, an enzyme which exhibits 3'-5' exonuclease enzymatic activity and is capable of primer template extension can be employed, including for example T4 or Exonucleases I, III, V. *Id.*

In some embodiments, the methods contemplated herein comprise performing sequencing and/or PCR on the 3'-5' exonuclease enzymatically processed complex discussed *supra* and elsewhere herein. In some embodiments, a tail portion of a capture probe module is copied in order to generate a hybrid nucleic acid molecule. In some embodiments, the hybrid nucleic acid molecule generated comprises the target region capable of hybridizing to the capture probe module and the complement of the capture probe module tail sequence.

In some embodiments, genetic analysis comprises a) hybridizing one or more capture probe modules to one or more target genetic loci in a plurality of genomic DNA library clones to form one or more capture probe module-DNA library clone complexes; b) isolating the one or more capture probe module-DNA library clone complexes from a); c) enzymatically processing the one or more isolated capture probe module-DNA library clone complexes from step b); d) performing PCR on the enzymatically processed complex from c) wherein the tail portion of the capture probe module is copied in order to generate amplified hybrid nucleic acid molecules, wherein the amplified hybrid nucleic acid molecules comprise a target sequence in the target genomic locus capable of hybridizing to the capture probe and the complement of the capture probe module tail sequence; and e) performing quantitative genetic analysis on the amplified hybrid nucleic acid molecules from d).

In some embodiments, methods for determining copy number of a specific target genetic locus are contemplated comprising: a) hybridizing one or more capture probe modules to one or more target genetic loci in a plurality of DNA library clones to form one or more capture probe module-DNA library clone complexes; b) isolating the one or more capture probe module-DNA library clone complexes from a); c) enzymatically processing the one or more isolated capture probe module-DNA library clone complexes from step b); d) performing PCR on the enzymatically processed complex from c) wherein the tail portion of the capture probe module is copied in order to generate amplified hybrid nucleic acid molecules, wherein the amplified hybrid nucleic acid molecules comprise a target sequence in the target genetic locus capable of hybridizing to the capture probe and the complement of the capture probe module tail sequence; e) performing PCR amplification of the amplified hybrid nucleic acid molecules in d); and f) quantitating the PCR reaction in e), wherein the quantitation allows for a determination of copy number of the specific target region.

In some embodiments, the enzymatic processing of step c) comprises performing 3'-5' exonuclease enzymatic processing on the one or more capture probe module-DNA library clone complexes from b) using an enzyme with 3'-5' exonuclease activity to remove the single stranded 3' ends; creating one or more hybrid capture probe module-cfDNA library clone molecules through the concerted action of a 5' FLAP endonuclease, DNA polymerization and nick closure by a DNA ligase; or performing 5'-3' DNA polymerase extension of the capture probe using the isolated DNA clone in the complex as a template.

In some embodiments, the enzymatic processing of step c) comprises performing 5'-3' DNA polymerase extension of the capture probe using the isolated DNA clone in the complex as a template.

In some embodiments, PCR can be performed using any standard PCR reaction conditions well known to those of skill in the art. In some embodiments, the PCR reaction in e) employs two PCR primers. In some embodiments, the PCR reaction in e) employs a first PCR primer that hybridizes to a repeat within the target genetic locus. In a particular embodiment, the PCR reaction in e) employs a second PCR primer that hybridizes to the hybrid nucleic acid molecules at the target genetic locus/tail junction. In some embodiments, the PCR reaction in e) employs a first PCR primer that hybridizes to the target genetic locus and a second PCR primer hybridizes to the amplified hybrid nucleic acid molecules at the target genetic locus/tail junction. In some embodiments, the second primer hybridizes to the target genetic locus/tail junction such that at least one or more nucleotides of the primer hybridize to the target genetic locus and at least one or more nucleotides of the primer hybridize to the tail sequence.

In some embodiments, amplification can be isothermal such as by Loop mediated isothermal amplification (LAMP), whole genome amplification (WGA), Strand displacement amplification (SDA), helicase-dependent amplification (HDA), Recombinase polymerase amplification (RPA), Nucleic acid sequencing based amplification (NASBA), Nicking Enzyme Amplification Reaction (NEAR), and Ligase Chain Reaction (LCR).

In some embodiments, DNA polymerases for isothermal amplification include DNA polymerases such as Klenow Fragment, Bsu large fragment, and phi29 for moderate temperature reactions (25-40°C) and the large fragment of Bst DNA polymerase for higher temperature (50-65°C) reactions. Enzymes suitable for LCR include a thermostable Taq ligase and a thermostable DNA polymerase such as Taq Polymerase.

In some embodiments, the amplified hybrid nucleic acid molecules obtained from step e) are sequenced and the sequences aligned horizontally, i.e., aligned to one another but not aligned to a reference sequence. In some embodiments, steps a) through e) are repeated one or more times with one or more capture probe modules. The capture probe modules can be the same or different and designed to target either cfDNA strand of a target genetic locus. In some embodiments, when the capture probes are different, they hybridize at overlapping or adjacent target sequences within a target genetic locus in the tagged cfDNA clone library. In some embodiments, a high density capture probe strategy is used wherein a plurality of capture probes hybridize to a target genetic locus, and wherein each of the plurality of capture probes hybridizes to the target genetic locus within about 5, about 10, about 15, about 20, about 25, about 30, about 35, about 40, about 45, about 50, about 100, about 200 bp or more of any other capture probe that hybridizes to the target genetic locus in a tagged DNA clone library, including all intervening distances. In some embodiments, a high density capture probe strategy is used wherein a plurality of capture probes hybridize to a target genetic locus, and wherein each of the plurality of capture probes hybridizes to the target genetic locus within 5, 10, 15, 20, 25, 30, 35, 40, 45, 50, 100, 200 bp or more of any other capture probe that hybridizes to the target genetic locus in a tagged DNA clone library, including all intervening distances.

In some embodiments, the method can be performed using two capture probe modules per target genetic locus, wherein one hybridizes to the "Watson" strand (non-coding or template strand) upstream of the target region and one hybridizes to the "Crick" strand (coding or non-template strand) downstream of the target region.

In some embodiments, the methods contemplated herein can further be performed multiple times with any number of capture probe modules, for example 2, 3, 4, 5, 6, 7, 8, 9, or 10 or more capture probe modules per target genetic locus any number of which hybridize to the Watson or Crick strand in any combination. In some embodiments, the sequences obtained can be aligned to one another in order to identify any of a number of differences.

In some embodiments, a plurality of target genetic loci are interrogated, e.g., 100, 200, 300, 400, 500, 600, 700, 800, 900, 1000, 1500, 2000, 2500, 3000, 3500, 4000, 4500, 5000, 10000, 50000, 100000, 500000 or more in a single reaction, using one or more capture probe modules.

In some embodiments, the enzymatic processing step (step c in the disclosure above) is not performed. In some embodiments, the isolated capture probe/tagged DNA fragment complexes are directly amplified wherein the DNA polymerase performs 5' -> 3' extension to form a library of hybrid molecules. In some embodiments, the library containing the hybrid molecules is further amplified using forward and reverse primers that contain sequencing adaptors (e.g. adaptors bind the sequencing primers such as P5 and P7 sequencing primers of Illumina^{®} NextSeq NGS technology) to generate the targeted library of sequencing ready amplified hybrid molecules.

By eliminating the enzymatic processing step, the targeted library is thus generated faster using the methods disclosed. Such faster methods lead to improved and faster performance of genetic analysis of the genetic loci of interest that are present in the DNA fragments in the tagged DNA libraries and in targeted libraries. One of skill in the art will recognize that the genetic loci can be analyzed for DNA alterations e.g., SNV, Indels, gene reorganizations, and copy number changes.

### 4. Determining the Number of Genome Equivalents

In some embodiments, a method for genetic analysis of DNA comprises determining the number of genome equivalents in the DNA clone library. As used herein, the term "genome equivalent" refers to the number of genome copies in each library. An important challenge met by the compositions and methods contemplated herein is achieving sufficient assay sensitivity to detect and analysis rare genetic mutations or differences in genetic sequence. To determine assay sensitivity value on a sample-by-sample basis, the numbers of different and distinct sequences that are present in each sample are measured, by measuring the number of genome equivalents that are present in a sequencing library. To establish sensitivity, the number of genome equivalents should be measured for each sample library.

The number of genome equivalents can be determined by qPCR assay or by using bioinformatics-based counting after sequencing is performed. In the process flow of clinical samples, qPCR measurement of genome equivalents is used as a QC step for DNA libraries. It establishes an expectation for assay sensitivity prior to sequence analysis and allows a sample to be excluded from analysis if its corresponding DNA clone library lacks the required depth of genome equivalents. Ultimately, the bioinformatics-based counting of genome equivalents is also used to identify the genome equivalents - and hence the assay sensitivity and false negative estimates - for each given DNA clone library.

The empirical qPCR assay and statistical counting assays should be well correlated. In cases where sequencing fails to reveal the sequence depth in a DNA clone library, reprocessing of the DNA clone library and/or additional sequencing may be required.

In some embodiments, the genome equivalents in a cellular DNA or cfDNA clone library are determined using a quantitative PCR (qPCR) assay. In some embodiments, a standard library of known concentration is used to construct a standard curve and the measurements from the qPCR assay are fit to the resulting standard curve and a value for genome equivalents is derived from the fit. The present inventors have discovered that a qPCR "repeat-based" assay comprising one primer that specifically hybridizes to a common sequence in the genome, *e.g.,* a repeat sequence, and another primer that binds to the primer binding site in the adaptor, measured an 8-fold increase in genome equivalents compared to methods using just the adaptor specific primer (present on both ends of the DNA clone). The number of genome equivalents measured by the repeat-based assays provides a more consistent library-to-library performance and a better alignment between qPCR estimates of genome equivalents and bioinformatically counted tag equivalents in sequencing runs.

Illustrative examples of repeats suitable for use in the repeat-based genome equivalent assays contemplated herein include, but not limited to: short interspersed nuclear elements (SINEs), *e.g.,* Alu repeats; long interspersed nuclear elements (LINEs), *e.g.,* LINE1, LINE2, LINE3; microsatellite repeat elements, *e.g.,* short tandem repeats (STRs), simple sequence repeats (SSRs); and mammalian-wide interspersed repeats (MIRs).

In some embodiments, the repeat is an Alu repeat.

### 5. Sequencing

In some embodiments, the quantitative genetic analysis comprises sequencing a plurality of hybrid nucleic acid molecules, as discussed elsewhere herein, *supra,* to generate sufficient sequencing depths to obtain a plurality of unique sequencing reads. The terms "unique reads" or "unique genomic sequences" (UGS) are used interchangeably herein and are identified by grouping individual redundant reads together into a "family." Redundant reads are sequence reads that share an identical UMIE *(e.g.,* share the same read code and the same DNA sequence start position within genomic sequence) and are derived from a single attachment event and are therefore amplification-derived "siblings" of one another. A single consensus representative of a family of redundant reads is carried forward as a unique read or UGS. Each unique read or UGS is considered a unique attachment event. The sum of unique reads corresponding to a particular capture probe is referred to as the "raw genomic depth" (RGD) for that particular capture probe. Each capture probe yields a set of unique reads that are computationally distilled from total reads by grouping into families. In some embodiments, the entire capture probe region in the hybrid molecule is sequenced. In some embodiments, a portion of the capture probe region in the hybrid molecule is sequenced.

The unique reads for a given sample (*e.g*., raw genomic depth for a sample) are then computed as the average of all the unique reads observed on a probe-by-probe basis. Unique reads are important because each unique read should be derived from a unique genomic DNA clone. Each unique read represents the input and analysis of a haploid equivalent of genomic DNA. The sum of unique reads is the sum of haploid genomes analyzed. The number of genomes analyzed, in turn, defines the sensitivity of the sequencing assay. By way of a non-limiting example, if the average unique read count is 100 genome equivalents, then that particular assay has a sensitivity of being able to detect one mutant read in 100, or 1%. Any observation less than this is not defensible.

Cases where there is an obvious copy number change (*e.g*., instances of noisy probes) are excluded from the data set used to compute the sample average. Herein, a "noisy probe" refers to a probe that captures a highly variable number of unique reads among a large set identical samples (*e.g.,* a highly variable number of unique reads among 12 - 16 sample replicates). In some embodiments, the number of unique reads associated with a noisy probe is increased compared to the average number of unique reads for the sample by 50% or more. In some embodiments, the number of unique reads associated with a noisy probe is decreased compared to the average number of unique reads for the sample by 50% or more. In some embodiments, about 2% to about 4% of probes used in a particular analysis are identified as noisy probes and are excluded from calculations to determine the average number of unique reads for a given sample. In some embodiments, 2% to 4% of probes used in a particular analysis are identified as noisy probes and are excluded from calculations to determine the average number of unique reads for a given sample.

In some embodiments, sequencing reads are identified as either "on-target reads" or "off-target reads." On-target reads possess a genomic DNA sequence that maps within the vicinity of a capture probe used to create the genomic library. In some embodiments, where each genomic sequence is physically linked to a specific capture probe and where the sequence of the genomic segment and capture probe are both determined as a unified piece of information, an on-target read is defined as any genomic sequence whose starting coordinate maps within 400 bp, and more generally within 200 bp of the 3' end of the corresponding capture probe. Off-target reads are defined as having genomic sequence that aligns to the reference genome at a location ≥ 500 base pairs (and more often mapping to entirely different chromosomes) relative to the capture probe.

In some embodiments, the quantitative genetic analysis comprises multiplex sequencing of hybrid nucleic acid molecules derived from a plurality of samples.

In some embodiments, the quantitative genetic analysis comprises obtaining one or more or a plurality of tagged DNA library clones, each clone comprising a first DNA sequence and a second DNA sequence, wherein the first DNA sequence comprises a sequence in a targeted genetic locus and the second DNA sequence comprises a capture probe sequence; performing a paired end sequencing reaction on the one or more clones and obtaining one or more sequencing reads or performing a sequencing reaction on the one or more clones in which a single long sequencing read of greater than about 100, about 200, about 300, about 400, about 500 or more nucleotides is obtained, wherein the read is sufficient to identify both the first DNA sequence and the second DNA sequence; and ordering or clustering the sequencing reads of the one or more clones according to the probe sequences of the sequencing reads.

### 6. Bioinformatics analysis

In some embodiments, the quantitative genetic analysis further comprises bioinformatic analysis of the sequencing reads. Bioinformatic analysis excludes any purely mental analysis performed in the absence of a composition or method for sequencing. In some embodiments, bioinformatics analysis includes, but is not limited to: sequence alignments; genome equivalents analysis; single nucleotide variant (SNV) analysis; gene copy number variation (CNV) analysis; measurement of chromosomal copy number; and detection of genetic lesions. In some embodiments, bioinformatics analysis is useful to quantify the number of genome equivalents analyzed in the cfDNA clone library; to detect the genetic state of a target genetic locus; to detect genetic lesions in a target genetic locus; and to measure copy number fluctuations within a target genetic locus.

Sequence alignments may be performed between the sequence reads and one or more human reference DNA sequences. In some embodiments, sequencing alignments can be used to detect genetic lesions in a target genetic locus including, but not limited to detection of a nucleotide transition or transversion, a nucleotide insertion or deletion, a genomic rearrangement, a change in copy number, or a gene fusion. Detection of genetic lesions that are causal or prognostic indicators may be useful in the diagnosis, prognosis, treatment, and/or monitoring of a particular genetic condition or disease.

The terms "target genetic locus" and "DNA target region" are used interchangeably herein and refer to a region of interest within a DNA sequence. In some embodiments, targeted genetic analyses are performed on the target genetic locus. In some embodiments, the DNA target region is a region of a gene that is associated with a particular genetic state, genetic condition, genetic diseases; fetal testing; genetic mosaicism, paternity testing; predicting response to drug treatment; diagnosing or monitoring a medical condition; microbiome profiling; pathogen screening; or organ transplant monitoring. In further embodiments, the DNA target region is a DNA sequence that is associated with a particular human chromosome, such as a particular autosomal or X-linked chromosome, or region thereof *(e.g.,* a unique chromosome region).

Also contemplated herein, are methods for sequence alignment analysis that can be performed without the need for alignment to a reference sequence, referred to herein as horizontal sequence analysis. Such analysis can be performed on any sequences generated by the methods contemplated herein or any other methods. In some embodiments, the sequence analysis comprises performing sequence alignments on the reads obtained by the methods contemplated herein.

In some embodiments, the genome equivalents in a cfDNA clone library are determined using bioinformatics-based counting after sequencing is performed. Each sequencing read is associated with a particular capture probe, and the collection of reads assigned to each capture probe is parsed into groups. Within a group, sets of individual reads share the same read code and the same DNA sequence start position within genomic sequence. These individual reads are grouped into a "family" and a single consensus representative of this family is carried forward as a "unique read." All of the individual reads that constituted a family are derived from a single attachment event and thus, they are amplification-derived "siblings" of one another. Each unique read is considered a unique attachment event and the sum of unique reads is considered equivalent to the number of genome equivalents analyzed.

As the number of unique clones approaches the total number of possible sequence combinations, probability dictates that the same code and start site combinations will be created by independent events and that these independent events will be inappropriately grouped within single families. The net result will be an underestimate of genome equivalents analyzed, and rare mutant reads may be discarded as sequencing errors because they overlap with wild-type reads bearing the same identifiers.

In some embodiments, to provide an accurate analysis for cfDNA clone libraries, the number of genome equivalents analyzed is about 1/10, about 1/12, about 1/14, about 1/16, about 1/18, about 1/20, about 1/25 or less the number of possible unique clones. In some embodiments, to provide an accurate analysis for cfDNA clone libraries, the number of genome equivalents analyzed is 1/10, 1/12, 1/14, 1/16, 1/18, 1/20, 1/25 or less the number of possible unique clones. It should be understood that the procedure outlined above is merely illustrative and not limiting.

In some embodiments, the number of genome equivalents to be analyzed may need to be increased. To expand the depth of genome equivalents, at least two solutions are contemplated. The first solution is to use more than one adaptor set per sample. By combining adaptors, it is possible to multiplicatively expand the total number of possible clones and therefore, expand the comfortable limits of genomic input. The second solution is to expand the read code by 1, 2, 3, 4, or 5, or more bases. The number of possible read codes that differ by at least 2 bases from every other read code scales as 4⁽ⁿ⁻¹⁾ where n is the number of bases within a read code. Thus, in a non-limiting example, if a read code is 5 nucleotides and 4⁽⁵⁻¹⁾ = 256; therefore, the inclusion of additional bases expands the available repertoire by a factor of four for each additional base.

In some embodiments, quantitative genetic analysis comprises bioinformatic analysis of sequencing reads to identify rare single nucleotide variants (SNV).

Next-generation sequencing has an inherent error rate of roughly 0.02-0.02%, meaning that anywhere from 1/200 to 1/500 base calls are incorrect. To detect variants and other mutations that occur at frequencies lower than this, for example at frequencies of 1 per 1000 sequences, it is necessary to invoke molecular annotation strategies. By way of a non-limiting example, analysis of 5000 unique molecules using targeted sequence capture technology would generate - at sufficient sequencing depths of >50,000 reads - a collection of 5000 unique reads, with each unique read belonging to a "family" of reads that all possess the same read code. A SNV that occurs within a family is a candidate for being a rare variant. When this same variant is observed in more than one family, it becomes a very strong candidate for being a rare variant that exists within the starting sample. In contrast, variants that occur sporadically within families are likely to be sequencing errors and variants that occur within one and only one family are either rare or the result of a base alteration that occurred *ex vivo* (*e.g.,* oxidation of a DNA base or PCR-introduced errors).

In some embodiments, the methods of detecting SNVs comprise introducing 10-fold more genomic input (genomes or genome equivalents) as the desired target sensitivity of the assay. In one non-limiting example, if the desired sensitivity is 2% (2 in 100), then the experimental target is an input of 2000 genomes.

In some embodiments, bioinformatics analysis of sequencing data is used to detect or identify SNV associated with a genetic state, condition or disease, genetic mosaicism, fetal testing, paternity testing, predicting response to drug treatment, diagnosing or monitoring a medical condition, microbiome profiling, pathogen screening, and monitoring organ transplants.

### 7. Copy number analysis

Provided herein are compositions and methods that are useful for the detection of a mutational change, SNP, translocation, inversion, deletion, change in copy number or other genetic variation within a sample of cellular genomic DNA (*e.g.* from a tissue biopsy sample) or cfDNA (*e.g*. from a blood sample). The compositions and methods disclosed herein are particularly useful in detecting incredibly hard to detect copy number variations in cfDNA from a biological sample (*e.g*. blood) with exquisite resolution. In particular, some embodiments of the disclosure are drawn to a method for the detecting copy number of a DNA target region from a test sample by generating a genomic DNA library made up of genomic DNA fragments attached to an adaptor, capturing DNA target regions with a plurality of capture probes, isolating the DNA library fragments comprising the DNA target region, and performing a quantitative genetic analysis of the DNA target region to thereby determining the copy number of the DNA target region. The adaptors described herein allow for the identification of the individual DNA fragment that is being sequenced, as well as the identity of the sample or source of the genomic DNA.

In some embodiments, the compositions and methods for detection of target-specific copy number changes disclosed herein are applicable to several sample types, including but not limited to direct tissue biopsies and peripheral blood. In the context of cancer genomics, and in particular cell free DNA (cfDNA) assays for the analysis of solid tumors, the amount of tumor DNA is often a very small fraction of the overall DNA. Further, copy number loss is difficult to detect in genomic DNA assays, and in particular, genomic DNA assays where copy number change may only be present in a portion of the total genomic DNA from a sample, *e.g.,* cfDNA assays. For example, most of the cell-free DNA extracted from a cancer patient will be derived from normal sources and have a diploid copy number (except for X-linked genes in male subjects). In a cancer patient, the fraction of DNA derived from tumors often has a low minor allele frequency, such as for example, a patient in which 2% of the circulating DNA extracted from plasma is derived from the tumor. The loss of one copy of a tumor suppressor gene (for example, BRCA1 in breast cancer) means that the minor allele frequency for the absence of detectable genomic fragments is 1%. In this scenario, a copy number loss assay engineered should be able to discriminate between 100 copies (normal) and 99 copies (heterozygous gene loss). Thus, some embodiments contemplate that the methods and compositions described herein allow for the detection of copy number change with sufficient resolution to detect changes in copy number at minor allele frequencies even in the context of cfDNA.

In some embodiments, a method for copy number analysis of a DNA target region DNA is provided. In some embodiments, copy number analysis is performed by generating a genomic DNA library of DNA library fragments that each contain genomic DNA fragment and an adaptor, isolating the DNA library fragments containing the DNA target regions, and performing a quantitative genetic analysis of the DNA target region. By "quantitative genetic analysis" it is meant an analysis performed by any molecular biological technique that is able to quantify changes in a DNA *(e.g.,* a gene, genetic locus, target region of interest, etc.) including but not limited to DNA mutations, SNPs, translocations, deletions, and copy number variations (CNVs). In some embodiments, the quantitative genetic analysis is performed by sequencing, for example, next generation sequencing.

In some embodiments, a method for copy number determination analysis is provided comprising obtaining one or more or a plurality of clones, each clone comprising a first DNA sequence and a second DNA sequence, wherein the first DNA sequence comprises a sequence in a targeted genetic locus and the second DNA sequence comprises a capture probe sequence. In related embodiments, a paired end sequencing reaction on the one or more clones is performed and one or more sequencing reads are obtained. In another embodiment, a sequencing reaction on the one or more clones is performed in which a single long sequencing read of greater than about 100 nucleotides is obtained, wherein the read is sufficient to identify both the first DNA sequence and the second DNA sequence. The sequencing reads of the one or more clones can be ordered or clustered according to the probe sequence of the sequencing reads.

Copy number analyses include, but are not limited to, analyses that examine the number of copies of a particular gene or mutation that occurs in a given genomic DNA sample and can further include quantitative determination of the number of copies of a given gene or sequence differences in a given sample. In some embodiments, copy number analysis is used to detect or identify gene amplification associated with genetic states, conditions, or diseases, fetal testing, genetic mosaicism, paternity testing, predicting response to drug treatment, diagnosing or monitoring a medical condition, microbiome profiling, pathogen screening, and monitoring organ transplants.

In some embodiments, copy number analysis is used to measure chromosomal instability. In such embodiments, sets of capture probes that comprise chromosomal stability probes are used to determine copy number variations at a uniform density across all sets of chromosomes. Copy number analyses are performed for each chromosomal stability probe and the chromosomal stability probes are then ordered according to their chromosomal target. This allows for visualization of copy number losses or gains across the genome and can serve as a measure of chromosomal stability.

In some embodiments, bioinformatics analysis of sequencing data is used to detect or identify one or more sequences or genetic lesions in a target locus including, but not limited to detection of a nucleotide transition or transversion, a nucleotide insertion or deletion, a genomic rearrangement, a change in copy number, or a gene fusion. Detection of genetic lesions that are causal or prognostic indicators may be useful in the diagnosis, prognosis, treatment, and/or monitoring of a particular genetic condition or disease. In some embodiments, genetic lesions are associated with genetic states, conditions, or diseases, fetal testing, genetic mosaicism, paternity testing, predicting response to drug treatment, diagnosing or monitoring a medical condition, microbiome profiling, pathogen screening, and monitoring organ transplants.

In some embodiments, the number of copies of the DNA target region present in the sample is determined by the quantitative genetic analysis. In some embodiments, the copy number of the DNA target region is determined by comparing the amount of copies of DNA target regions present in the sample and comparing it to amounts of DNA target regions present in one or more samples with known copy number.

Some embodiments contemplate that the compositions and methods described herein are particularly useful for detecting changes in copy number in a sample of genomic DNA, where only a portion of the total genomic DNA in the sample has a change in copy number. For example, a significant tumor mutation may be present in a sample, *e.g.* a sample of cell free DNA, that is present in a minor allele frequency that is significantly less than 50% (*e.g.,* in the range of 0.1% to >20%), in contrast to conventional SNP genotyping where allele frequencies are generally ~100%, 50% or 0%. One of skill of the art will recognize that the compositions and methods described herein are also useful in detecting other types of mutation including single nucleotide variants (SNVs), short (*e.g.*, less than 40 base pairs (bp)) insertions, and deletions (indels), and genomic rearrangements including oncogenic gene fusions.

In some embodiments, the compositions and/or methods described herein are useful for, capable of, suited for, and/or able to detect, identify, observe, and/or reveal a change in copy number of one or more DNA target regions present in less than about 20%, less than about 19%, less than about 18%, less than about 17%, less than about 16%, less than about 15%, less than about 14%, less than about 13%, less than about 12%, less than about 11%, less than about 10%, less than about 9%, less than about 8%, less than about 7%, less than about 6%, less than about 5%, less than about 4%, less than about 3%, less than about 2%, less than about 1%, less than about 0.5%, less than about 0.2%, or less than about 0.1% of the total genomic DNA from the sample. In some embodiments, the methods described herein are useful for, capable of, suited for, and/or able to detect, identify, observe, and/or reveal a change in copy number of one or more DNA target regions present in between about 0.01% to about 100%, about 0.01% to about 50%, or about 0.1% to about 20% of the total genomic DNA from the sample.

In some embodiments, the compositions and/or methods described herein are useful for, capable of, suited for, and/or able to detect, identify, observe, and/or reveal a change in copy number of one or more DNA target regions present in less than 20%, less than 19%, less than 18%, less than 17%, less than 16%, less than 15%, less than 14%, less than 13%, less than 12%, less than 11%, less than 10%, less than 9%, less than 8%, less than 7%, less than 6%, less than 5%, less than 4%, less than 3%, less than 2%, less than 1%, less than 0.5%, less than 0.2%, or less than 0.1% of the total genomic DNA from the sample. In some embodiments, the methods described herein are useful for, capable of, suited for, and/or able to detect, identify, observe, and/or reveal a change in copy number of one or more DNA target regions present in between 0.01% to 100%, 0.01% to 50%, or 0.1% to 20% of the total genomic DNA from the sample.

In some embodiments, a method for genetic analysis of cfDNA comprises: generating and amplifying a cfDNA library, determining the number of genome equivalents in the cfDNA library; and performing a quantitative genetic analysis of one or more genomic target loci.

Some embodiments contemplate that the any of the methods and compositions described herein are effective for use to efficiently analyze, detect, diagnose, and/or monitor genetic states, genetic conditions, genetic diseases, genetic mosaicism, fetal diagnostics, paternity testing, microbiome profiling, pathogen screening, and organ transplant monitoring using genomic DNA, *e.g.,* cellular or cfDNA, where all or where only a portion of the total genomic DNA in the sample has a feature of interest, *e.g.* a genetic lesion, mutation, single nucleotide variant (SNV). In some embodiments, a feature of interest is a genetic feature associated with a disease or condition. For example, a significant tumor mutation may be present in a sample, *e.g.* a sample of cfDNA, that is present in a minor allele frequency that is significantly less than 50% (*e.g.* in the range of 0.1% to >20%), in contrast to conventional SNP genotyping where allele frequencies are generally ~100%, 50% or 0%.

### 8. Clinical Applications

In some embodiments, disclosed herein is a method of detecting, identifying, predicting, diagnosing, or monitoring a condition or disease in a subject by detecting a mutational change, SNP, translocation, inversion, deletion, change in copy number or other genetic variation in a region of interest.

In some embodiments, disclosed herein is a method of detecting, identifying, predicting, diagnosing, or monitoring a condition or disease in a subject.

In some embodiments, disclosed herein is a method of detecting, identifying, predicting, diagnosing, or monitoring a genetic state, condition or disease in a subject comprises performing a quantitative genetic analysis of one or more target genetic loci in a DNA clone library to detect or identify a change in the sequence at the one or more target genetic loci. In some embodiments, the change is a change in copy number.

In some embodiments, disclosed herein is a method of detecting, identifying, predicting, diagnosing, or monitoring a genetic state, condition or disease comprises isolating or obtaining cellular DNA or cfDNA from a biological sample of a subject; treating the cellular DNA or cfDNA with one or more end-repair enzymes to generate end-repaired DNA; attaching one or more adaptors to each end of the end-repaired DNA to generate a genomic DNA library; amplifying the DNA library to generate a DNA clone library; determining the number of genome equivalents in the DNA clone library; and performing a quantitative genetic analysis of one or more target genetic loci in a DNA clone library to detect or identify a change in the sequence, e.g., an SNP, a translocation, an inversion, a deletion, or a change in copy number at of the one or more target genetic loci.

In some embodiments, disclosed herein is a method of detecting, identifying, predicting, diagnosing, or monitoring a genetic state, or genetic condition or disease selected from the group consisting of: genetic diseases; genetic mosaicism; fetal testing; paternity testing; paternity testing; predicting response to drug treatment; diagnosing or monitoring a medical condition; microbiome profiling; pathogen screening; and organ transplant monitoring comprising isolating or obtaining genomic DNA from a biological sample of a subject; treating the DNA with one or more end-repair enzymes to generate end-repaired DNA; attaching one or more adaptors to each end of the end-repaired DNA to generate a genomic DNA library; amplifying the genomic DNA library to generate a DNA clone library; determining the number of genome equivalents in the DNA clone library; and performing a quantitative genetic analysis of one or more target genetic loci in a DNA clone library to detect or identify a nucleotide transition or transversion, a nucleotide insertion or deletion, a genomic rearrangement, a change in copy number, or a gene fusion in the sequence at the one or more target genetic loci.

Illustrative examples of genetic diseases that can be detected, identified, predicted, diagnosed, or monitored with the compositions and methods contemplated herein include, but are not limited to cancer, Alzheimer's disease (APOE1), Charcot-Marie-Tooth disease, Leber hereditary optic neuropathy (LHON), Angelman syndrome (UBE3A, ubiquitin-protein ligase E3A), Prader-Willi syndrome (region in chromosome 15), β-Thalassaemia (HBB, β-Globin), Gaucher disease (type I) (GBA, Glucocerebrosidase), Cystic fibrosis (CFTREpithelial chloride channel), Sickle cell disease (HBB, β-Globin), Tay-Sachs disease (HEXA, Hexosaminidase A), Phenylketonuria (PAH, Phenylalanine hydrolyase), Familial hypercholesterolaemia (LDLR, Low density lipoprotein receptor), Adult polycystic kidney disease (PKD1, Polycystin), Huntington disease (HDD, Huntingtin), Neurofibromatosis type I (NF1, NF1 tumour suppressor gene), Myotonic dystrophy (DM, Myotonin), Tuberous sclerosis (TSC1, Tuberin), Achondroplasia (FGFR3, Fibroblast growth factor receptor), Fragile X syndrome (FMR1, RNA-binding protein), Duchenne muscular dystrophy (DMD, Dystrophin), Haemophilia A (F8C, Blood coagulation factor VIII), Lesch-Nyhan syndrome (HPRT1, Hypoxanthine guanine ribosyltransferase 1), and Adrenoleukodystrophy (ABCD1).

Illustrative examples of cancers that can be detected, identified, predicted, diagnosed, or monitored with the compositions and methods contemplated herein include, but are not limited to: B cell cancer, e.g., multiple myeloma, melanomas, breast cancer, lung cancer (such as non-small cell lung carcinoma or NSCLC), bronchus cancer, colorectal cancer, prostate cancer, pancreatic cancer, stomach cancer, ovarian cancer, urinary bladder cancer, brain or central nervous system cancer, peripheral nervous system cancer, esophageal cancer, cervical cancer, uterine or endometrial cancer, cancer of the oral cavity or pharynx, liver cancer, kidney cancer, testicular cancer, biliary tract cancer, small bowel or appendix cancer, salivary gland cancer, thyroid gland cancer, adrenal gland cancer, osteosarcoma, chondrosarcoma, cancer of hematological tissues, adenocarcinomas, inflammatory myofibroblastic tumors, gastrointestinal stromal tumor (GIST), colon cancer, multiple myeloma (MM), myelodysplastic syndrome (MDS), myeloproliferative disorder (MPD), acute lymphocytic leukemia (ALL), acute myelocytic leukemia (AML), chronic myelocytic leukemia (CML), chronic lymphocytic leukemia (CLL), polycythemia Vera, Hodgkin lymphoma, non-Hodgkin lymphoma (NHL), soft-tissue sarcoma, fibrosarcoma, myxosarcoma, liposarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, squamous cell carcinoma, basal cell carcinoma, adenocarcinoma, sweat gland carcinoma, sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, seminoma, embryonal carcinoma, Wilms' tumor, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, neuroblastoma, retinoblastoma, follicular lymphoma, diffuse large B-cell lymphoma, mantle cell lymphoma, hepatocellular carcinoma, thyroid cancer, gastric cancer, head and neck cancer, small cell cancers, essential thrombocythemia, agnogenic myeloid metaplasia, hypereosinophilic syndrome, systemic mastocytosis, familiar hypereosinophilia, chronic eosinophilic leukemia, neuroendocrine cancers, carcinoid tumors, and the like.

In some embodiments, the genetic lesion is a lesion annotated in the Cosmic database (the lesions and sequence data are available online and can be downloaded from the Cancer Gene Census section of the Cosmic website) or a lesion annotated in the Cancer Genome Atlas (the lesions and sequence data are available online and can be downloaded from The Cancer Genome Atlas website).

Illustrative examples of genes that harbor one or more genetic lesions associated with cancer that can be detected, identified, predicted, diagnosed, or monitored with the compositions and methods contemplated herein include, but are not limited to ABCB1, ABCC2, ABCC4, ABCG2, ABL1, ABL2, AKT1, AKT2, AKT3, ALDH4A1, ALK, APC, AR, ARAF, ARFRP1, ARID1A, ATM, ATR, AURKA, AURKB, BCL2, BCL2A1, BCL2L1, BCL2L2, BCL6, BRAF, BRCA1, BRCA2, Clorf144, CARD11, CBL, CCND1, CCND2, CCND3, CCNE1, CDH1, CDH2, CDH20, CDH5, CDK4, CDK6, CDK8, CDKN2A, CDKN2B, CDKN2C, CEBPA, CHEK1, CHEK2, CRKL, CRLF2, CTNNB1, CYP1B1, CYP2C19, CYP2C8, CYP2D6, CYP3A4, CYP3A5, DNMT3A, DOT1L, DPYD, EGFR, EPHA3, EPHA5, EPHA6, EPHA7, EPHB1, EPHB4, EPHB6, EPHX1, ERBB2, ERBB3, ERBB4, ERCC2, ERG, ESR1, ESR2, ETV1, ETV4, ETV5, ETV6, EWSR1, EZH2, FANCA, FBXW7, FCGR3A, FGFR1, FGFR2, FGFR3, FGFR4, FLT1, FLT3, FLT4, FOXP4, GATA1, GNA11, GNAQ, GNAS, GPR124, GSTP1, GUCY1A2, HOXA3, HRAS, HSP90AA1, IDH1, IDH2, IGF1R, IGF2R, IKBKE, IKZF1, INHBA, IRS2, ITPA, JAK1, JAK2, JAK3, JUN, KDR, KIT, KRAS, LRP1B, LRP2, LTK, MAN1B1, MAP2K1, MAP2K2, MAP2K4, MCL1, MDM2, MDM4, MEN1, MET, MITF, MLH1, MLL, MPL, MRE11A, MSH2, MSH6, MTHFR, MTOR, MUTYH, MYC, MYCL1, MYCN, NF1, NF2, NKX2-1, NOTCH1, NPM1, NQO1, NRAS, NRP2, NTRK1, NTRK3, PAK3, PAX5, PDGFRA, PDGFRB, PIK3CA, PIK3R1, PKHD1, PLCG1, PRKDC, PTCH1, PTEN, PTPN11, PTPRD, RAF1, RARA, RB1, RET, RICTOR, RPTOR, RUNX1, SLC19A1, SLC22A2, SLCO1B3, SMAD2, SMAD3, SMAD4, SMARCA4, SMARCB1, SMO, SOD2, SOX10, SOX2, SRC, STK11, SULT1A1, TBX22, TET2, TGFBR2, TMPRSS2, TNFRSF14, TOP1, TP53, TPMT, TSC1, TSC2, TYMS, UGT1A1, UMPS, USP9X, VHL, and WT1.

In some embodiments, the genetic lesion comprises a nucleotide transition or transversion, a nucleotide insertion or deletion (i.e., an indel), a genomic rearrangement, a change in copy number, or a gene fusion. In some embodiments, the genetic lesion comprises a frameshift. In some embodiments, the genetic lesion comprises a change in splicing. In some embodiments, the genetic lesion comprises a single nucleotide variation (SNV).

In some embodiments, the genetic lesion is a gene fusion that fuses the 3' coding region of the ALK gene to another gene.

In some embodiments, the genetic lesion is a gene fusion that fuses the 3' coding region of the ALK gene to the EML4 gene.

In some embodiments, the genetic lesion is any one of the lesions shown in Table 5 or Table 6. For example, the genetic lesion may be a TM mutation, a BRCA1 frameshift, a BRCA2 frameshift, a BRCA2 G4 mutation (i.e., a mutation that causes formation of a G-quadruplex structure), a FANCA splice mutation, a HDAC2 frameshift, a PALB2 Q479 mutation, or a ATM frameshift. In some embodiments, the genetic lesion may be a ERBB2 1655V mutation, a TP53 Q331 mutation, a TP53 frameshift, a EML4-ALK fusion, or an EGFR amplification.

Illustrative examples of conditions suitable for fetal testing that can be detected, identified, predicted, diagnosed, or monitored with the compositions and methods contemplated herein include but are not limited to: Down Syndrome (Trisomy 21), Edwards Syndrome (Trisomy 18), Patau Syndrome (Trisomy 13), Klinefelter's Syndrome (XXY), Triple X syndrome, XYY syndrome, Trisomy 8, Trisomy 16, Turner Syndrome (XO), Robertsonian translocation, DiGeorge Syndrome and Wolf-Hirschhorn Syndrome.

Illustrative examples of alleles suitable for paternity testing that can be detected, identified, predicted, diagnosed, or monitored with the compositions and methods contemplated herein include but are not limited to 16 or more of: D20S1082, D6S474, D12ATA63, D22S1045, D10S1248, D1S1677, D11S4463, D4S2364, D9S1122, D2S1776, D10S1425, D3S3053, D5S2500, D1S1627, D3S4529, D2S441, D17S974, D6S1017, D4S2408, D9S2157, Amelogenin, D17S1301, D1GATA113, D18S853, D20S482, and D14S1434.

Illustrative examples of genes suitable for predicting the response to drug treatment that can be detected, identified, predicted, diagnosed, or monitored with the compositions and methods contemplated herein include, but are not limited to, one or more of the following genes: ABCB1 (ATP-binding cassette, sub-family B (MDR/TAP), member 1), ACE (angiotensin I converting enzyme), ADH1A (alcohol dehydrogenase 1A (class I), alpha polypeptide), ADH1B (alcohol dehydrogenase IB (class I), beta polypeptide), ADH1C (alcohol dehydrogenase 1C (class I), gamma polypeptide), ADRB1 (adrenergic, beta-1-, receptor), ADRB2 (adrenergic, beta-2-, receptor, surface), AHR (aryl hydrocarbon receptor), ALDH1A1 (aldehyde dehydrogenase 1 family, member A1), ALOX5 (arachidonate 5-lipoxygenase), BRCA1 (breast cancer 1, early onset), COMT (catechol-O-methyltransferase), CYP2A6 (cytochrome P450, family 2, subfamily A, polypeptide 6), CYP2B6 (cytochrome P450, family 2, subfamily B, polypeptide 6), CYP2C9 (cytochrome P450, family 2, subfamily C, polypeptide 9), CYP2C19 (cytochrome P450, family 2, subfamily C, polypeptide 19), CYP2D6 (cytochrome P450, family 2, subfamily D, polypeptide 6), CYP2J2 (cytochrome P450, family 2, subfamily J, polypeptide 2), CYP3A4 (cytochrome P450, family 3, subfamily A, polypeptide 4), CYP3A5 (cytochrome P450, family 3, subfamily A, polypeptide 5), DPYD (dihydropyrimidine dehydrogenase), DRD2 (dopamine receptor D2), F5 (coagulation factor V), GSTP1 (glutathione S-transferase pi), HMGCR (3-hydroxy-3-methylglutaryl-Coenzyme A reductase), KCNH2 (potassium voltage-gated channel, subfamily H (eag-related), member 2), KCNJ11 (potassium inwardly-rectifying channel, subfamily J, member 11), MTHFR (5,10-methylenetetrahydrofolate reductase (NADPH)), NQO1 (NAD(P)H dehydrogenase, quinone 1), P2RY1 (purinergic receptor P2Y, G-protein coupled, 1), P2RY12 (purinergic receptor P2Y, G-protein coupled, 12), PTGIS (prostaglandin I2 (prostacyclin) synthase), SCN5A (sodium channel, voltage-gated, type V, alpha (long QT syndrome 3)), SLC19A1 (solute carrier family 19 (folate transporter), member 1), SLCO1B1 (solute carrier organic anion transporter family, member 1B1), SULT1A1 (sulfotransferase family, cytosolic, 1A, phenol-preferring, member 1), TPMT (thiopurine S-methyltransferase), TYMS (thymidylate synthetase), UGT1A1 (UDP glucuronosyltransferase 1 family, polypeptide A1), VDR (vitamin D (1,25- dihydroxyvitamin D3) receptor), VKORC1 (vitamin K epoxide reductase complex, subunit 1).

Illustrative examples of medical conditions that can be detected, identified, predicted, diagnosed, or monitored with the compositions and methods contemplated herein include, but are not limited to: stroke, transient ischemic attack, traumatic brain injury, heart disease, heart attack, angina, atherosclerosis, and high blood pressure.

Illustrative examples of pathogens that can be screened for with the compositions and methods contemplated herein include, but are not limited to: bacteria fungi, and viruses.

Illustrative examples of bacterial species that can be screened for with the compositions and methods contemplated herein include, but are not limited to: a *Mycobacterium* spp., a *Pneumococcus* spp., an *Escherichia* spp., a *Campylobacter* spp., a *Corynebacterium* spp., a *Clostridium* spp., a *Streptococcus* spp., a *Staphylococcus* spp., a *Pseudomonas* spp., a *Shigella* spp., a *Treponema* spp., or a *Salmonella* spp.

Illustrative examples of fungal species that can be screened for with the compositions and methods contemplated herein include, but are not limited to: an *Aspergillis spp., a Blastomyces spp.,* a *Candida spp.,* a *Coccicioides spp.,* a *Cryptococcus spp.,* dermatophytes, a *Tinea spp., a Trichophyton spp., a Microsporum spp., a Fusarium spp., a Histoplasma spp., a Mucoromycotina spp.,* a *Pneumocystis spp.,* a *Sporothrix spp.,* an *Exserophilum spp.,* or a *Cladosporium spp.*

Illustrative examples of viruses that can be screened for with the compositions and methods contemplated herein include, but are not limited to: Influenza A such as H1N1, H1N2, H3N2 and H5N1 (bird flu), Influenza B, Influenza C virus, Hepatitis A virus, Hepatitis B virus, Hepatitis C virus, Hepatitis D virus, Hepatitis E virus, Rotavirus, any virus of the Norwalk virus group, enteric adenoviruses, parvovirus, Dengue fever virus, Monkey pox, Mononegavirales, Lyssavirus such as rabies virus, Lagos bat virus, Mokola virus, Duvenhage virus, European bat virus 1 & 2 and Australian bat virus, Ephemerovirus, Vesiculovirus, Vesicular Stomatitis Virus (VSV), Herpesviruses such as Herpes simplex virus types 1 and 2, varicella zoster, cytomegalovirus, Epstein-Bar virus (EBV), human herpesviruses (HHV), human herpesvirus type 6 and 8, Moloney murine leukemia virus (M-MuLV), Moloney murine sarcoma virus (MoMSV), Harvey murine sarcoma virus (HaMuSV), murine mammary tumor virus (MuMTV), gibbon ape leukemia virus (GaLV), feline leukemia virus (FLV), spumavirus, Friend murine leukemia virus, Murine Stem Cell Virus (MSCV) and Rous Sarcoma Virus (RSV), HIV (human immunodeficiency virus; including HIV type 1, and HIV type 2), visna-maedi virus (VMV) virus, the caprine arthritis-encephalitis virus (CAEV), equine infectious anemia virus (EIAV), feline immunodeficiency virus (FIV), bovine immune deficiency virus (BIV), and simian immunodeficiency virus (SIV), papilloma virus, murine gammaherpesvirus, Arenaviruses such as Argentine hemorrhagic fever virus, Bolivian hemorrhagic fever virus, Sabia-associated hemorrhagic fever virus, Venezuelan hemorrhagic fever virus, Lassa fever virus, Machupo virus, Lymphocytic choriomeningitis virus (LCMV), Bunyaviridiae such as Crimean-Congo hemorrhagic fever virus, Hantavirus, hemorrhagic fever with renal syndrome causing virus, Rift Valley fever virus, Filoviridae (filovirus) including Ebola hemorrhagic fever and Marburg hemorrhagic fever, Flaviviridae including Kaysanur Forest disease virus, Omsk hemorrhagic fever virus, Tick-borne encephalitis causing virus and Paramyxoviridae such as Hendra virus and Nipah virus, variola major and variola minor (smallpox), alphaviruses such as Venezuelan equine encephalitis virus, eastern equine encephalitis virus, western equine encephalitis virus, SARS-associated coronavirus (SARS-CoV), West Nile virus, and any encephaliltis causing virus.

Illustrative examples of genes suitable for monitoring an organ transplant in a transplant recipient that can be detected, identified, predicted, diagnosed, or monitored with the compositions and methods contemplated herein include, but are not limited to, one or more of the following genes: HLA-A, HLA-B, HLA-C, HLA-DR, HLA-DP, and HLA-DQ.

In some embodiments, a bioinformatic analysis is used to quantify the number of genome equivalents analyzed in the cfDNA clone library; detect genetic variants in a target genetic locus; detect mutations within a target genetic locus; detect genetic fusions within a target genetic locus; or measure copy number fluctuations within a target genetic locus.

In some embodiments, a companion diagnostic for a genetic disease is provided, comprising: isolating or obtaining genomic DNA from a biological sample of a subject; treating the DNA with one or more end-repair enzymes to generate end-repaired DNA; attaching one or more adaptors to each end of the end-repaired DNA to generate a DNA library; amplifying the DNA library to generate a DNA clone library; determining the number of genome equivalents in the DNA clone library; and performing a quantitative genetic analysis of one or more biomarkers associated with the genetic disease in the DNA clone library, wherein detection of, or failure to detect, at least one of the one or more biomarkers indicates whether the subject should be treated for the genetic disease. In some embodiments, the DNA is cfDNA. In some embodiments, the DNA is cellular DNA.

As used herein, the term "companion diagnostic" refers to a diagnostic test that is linked to a particular anti-cancer therapy. In a particular embodiment, the diagnostic methods comprise detection of genetic lesion in a biomarker associated with in a biological sample, thereby allowing for prompt identification of patients should or should not be treated with the anti-cancer therapy.

Anti-cancer therapy includes, but is not limited to surgery, radiation, chemotherapeutics, anti-cancer drugs, and immunomodulators.

Illustrative examples of anti-cancer drugs include, but are not limited to: alkylating agents such as thiotepa and cyclophosphamide (CYTOXAN^{™}); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine resume; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, carminomycin, carzinophilin, chromomycins, dactinomycin, daunorubicin, detorubicin, 6-diazo-5-oxo-L-norleucine, doxorubicin and its pegylated formulations, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elformithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK^{®}; razoxane; sizofiran; spirogermanium; tenuazonic acid; triaziquone; 2, 2',2"-trichlorotriethylamine; urethan; vindesine; dacarbazine; mannomustine; mitobronitol; mitolactol; pipobroman; gacytosine; arabinoside ("Ara-C"); cyclophosphamide; thiotepa; taxoids, *e.g.,* paclitaxel (TAXOL^{®}, Bristol-Myers Squibb Oncology, Princeton, N.J.) and doxetaxel (TAXOTERE^{®}., Rhne-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; aminopterin; xeloda; ibandronate; CPT-11; topoisomerase inhibitor RFS 2000; difluoromethylomithine (DMFO); retinoic acid derivatives such as Targretin^{™} (bexarotene), Panretin^{™} (alitretinoin); ONTAK^{™} (denileukin diftitox) ; esperamicins; capecitabine; and pharmaceutically acceptable salts, acids or derivatives of any of the above. Also included in this definition are anti-hormonal agents that act to regulate or inhibit hormone action on cancers such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens such as flutamide, nilutamide, bicalutamide, leuprolide, and goserelin; and pharmaceutically acceptable salts, acids or derivatives of any of the above.

Illustrative examples of immunomodulators include, but are not limited to: cyclosporine, tacrolimus, tresperimus, pimecrolimus, sirolimus, verolimus, laflunimus, laquinimod and imiquimod, as well as analogs, derivatives, salts, ions and complexes thereof.

In some embodiments, an anti-cancer drug may include a poly-ADP ribose polymerase (PARP) inhibitor. Illustrative examples of PARP inhibitors include, but are not limited to, olaparib (AZD-2281), rucaparib (AG014699 or PF-01367338, niraparib (MK-4827), talazoparib (BMN-673) veliparib (ABT-888), CEP 9722, E7016, BGB-290, 3-aminobenzamide.

The following examples are provided by way of illustration only and not by way of limitation. Those of skill in the art will readily recognize a variety of noncritical parameters that could be changed or modified to yield essentially similar results.

The practice of some embodiments of the invention will employ, unless indicated specifically to the contrary, conventional methods of chemistry, biochemistry, organic chemistry, molecular biology, microbiology, recombinant DNA techniques, genetics, immunology, and cell biology that are within the skill of the art, many of which are described below for the purpose of illustration. Such techniques are explained fully in the literature. See, *e.g.,* Sambrook, et al., Molecular Cloning: A Laboratory Manual (3rd Edition, 2001); Sambrook, et al., Molecular Cloning: A Laboratory Manual (2nd Edition, 1989); Maniatis et al., Molecular Cloning: A Laboratory Manual (1982); Ausubel et al., Current Protocols in Molecular Biology (John Wiley and Sons, updated July 2008); Short Protocols in Molecular Biology: A Compendium of Methods from Current Protocols in Molecular Biology, Greene Pub. Associates and Wiley-Interscience; Glover, DNA Cloning: A Practical Approach, vol. I & II (IRL Press, Oxford, 1985); Anand, Techniques for the Analysis of Complex Genomes, (Academic Press, New York, 1992); Transcription and Translation (B. Hames & S. Higgins, Eds., 1984); Perbal, A Practical Guide to Molecular Cloning (1984); and Harlow and Lane, Antibodies, (Cold Spring Harbor Laboratory Press, Cold Spring Harbor, N.Y., 1998).

### EXAMPLES

The disclosure is further illustrated by the following examples. It is to be understood that the examples are provided to illustrate certain embodiments and that no limitation to the scope of the disclosure is intended thereby. It is to be further understood that resort may be had to various other embodiments, modifications, and equivalents thereof which may suggest themselves to those skilled in the art without departing from the spirit of the present disclosure.

### EXAMPLE 1: PREPARATION OF DNA LIBRARY

Cell-free DNA and genomic DNA isolated from immortalized cells harboring gene variants (Coriell Institute for Medical Research or SeraCare Life Sciences, Inc.) were used for NGS library (adaptor-tagged DNA library) construction in this example.

**Table 1: Samples Used in Experiment**

| **Sample type** | **Sample Input (ng)** | **Sample Description** |
|---|---|---|
| Wild type (cfDNA) | 50 | cfDNA isolated from blood sample of a healthy donor |
| DNA Mixture 1 | 20 | Mixture of genomic and synthetic DNA that harbor HRD (Homologous Repair Deficient) gene variants (ATM, BRCA1 and BRCA2, BRCA2, FANCA, HDAC2, and PALB2. Genomic DNA are fragmented and processed in parallel with cfDNA. (Custom DNA mixture purchased from SeraCare) |
| DNA Mixture 2 | 25 | Mixture of genomic DNA that harbor lung cancer gene variants. Genomic DNA are fragmented and processed in parallel with cfDNA (ERBB2, TP53, EML4-Alk fusion (Fusion), EGFR. Cell line DNA used include DNA from the following cell lines: NA12878, PC-3 and H2228. |
| Disease Sample 1 | | Wild type cfDNA + DNA Mixture 1 |
| Disease Sample 2 | | Wild type cfDNA + DNA Mixture 2 |

Cell-free DNA from a healthy donor was extracted from plasma samples (see Table 1) using a QIAmp DSP Circulating NA kit (Qiagen).

The advantage of using lab-generated Disease Sample 1 and Disease Sample 2 is that the compositions can be carefully controlled as detailed below, and sample availability is essentially unlimited.

Genomic DNA was sheared by sonication using an ultra sonicator (Covaris^{®}) on a setting to generate 200 bp fragments, then further purified and size-selected using "double-sided" bead purification with paramagnetic AMPure XP^{®} beads (Beckman^{®}).

Mixtures of fragmented cell line genomic DNA and synthetic DNA were combined with the WT cfDNA to produce Disease Sample 1 and Disease Sample 2 with known single nucleotide variants (SNVs), insertion and deletions (Indels variants), copy number variants (CNVs), and fusions at defined allele frequencies (AF). Appropriate combinations of input sample amounts listed in the table above were blended into defined percentages to allow for detection of low allele frequency (AF), end-repaired and converted to tagged DNA libraries as described below.

### EXAMPLE 2: OPTIONAL SINGLE-STEP DNA END-REPAIR

Input DNA fragments were converted to "end-repaired DNA fragments" such that the end-repaired DNA fragments possess 5' phosphate groups and 3' dA nucleotide overhangs in a single reaction mixture (single-step end repair).

A commercially available kit (NEB Ultra II End Repair^{®}/dA tailing module (E7546L) was used to end repair the DNA fragments. The End Repair Master Mix^{®} ("End Repair MM") was added to the extended DNA fragments in a single tube reaction mixture. End Repair MM was prepared by combining NEBNext Ultra II End Prep Enzyme Mix^{®} with NEBNext Ultra II End Prep Reaction Buffer^{®}, each mix or buffer a component of NEBNext Ultra II End Prep/dA-tailing module (New England Biolabs^{®}). The reaction mixture containing the extended DNA fragments was incubated in a thermocycler under the following reaction conditions: 20°C for 15 min and then at 70°C for 10 min (a "single step reaction").

In some embodiments, the end-repair/dA-tailing step was optimized such that the single step reaction uses significantly lower amounts of End Repair master mix (MM) than the manufacturer's recommended amounts for performing such a reaction. In some embodiments, reduction in the amounts of End Repair MM also surprisingly had no adverse impact on the formation of End Repaired DNA fragments (averaging > 3500 GEs) as demonstrated by cloning efficiency of the End Repaired DNA fragments using the adaptors in the disclosure and the genomic equivalents of the resulting NGS library that was observed. In fact, surprisingly, the cloning efficiency was increased using this single step end repair process as described in the disclosure.

### EXAMPLE 3: ADAPTOR LIGATION

A pool of 3' dT-tailed ligation strands of the multifunctional adaptors modules were ligated to end-repaired DNA fragments from the samples above, resulting in adaptor attachment to the 5' end of fragments. Complementary non-ligation strands were not ligated to the 3' dA tailed end of DNA fragments. A description of the adaptors used in this experiment is provided in Table 2 and Table 3.

45uL of the End Repair reaction mixture (containing end-repaired DNA fragments having 5' phosphorylated ends and 3'dA nucleotide overhangs) was added to 5.0 µL of a pool of unique multifunctional Adaptor modules (5 µM) and 30 µL of NEB Ultra II Ligation Mix^{®} (New England Biolabs^{®}, MA, U.S.A). Each ligation strand of the adaptor modules was 47 nt in length, and comprises (from 5' -> 3') an amplification region (AMP, 25 nt), a multifunctional ID region (8 nt) capable of identifying both the sample and the unique fragment, a UMI multiplier (3 nt), an anchor (10 nt), and a 3' dT overhang. The ligation strands used in this example are provided in Table 2. The pool of the adaptor modules was prepared such that each adaptor pool contained equimolar amounts of adaptors modules comprising the four types of anchor regions, where each anchor type has a 3' terminal nucleotide selected from A, T, C, and G.

**Table 2: Adaptor structures**

| Adaptor name | Description/Sequence |
|---|---|
| Ligation Strand/ Anchor Region 1 (16-1) | AMP-ID Region/UMI Multiplier-ACGTATGCCA (SEQ ID NO: 2)-3'dT |
| Ligation Strand/ Anchor Region 2 (16-2) | AMP-ID Region/UMI Multiplier-CTAGCGTTAC (SEQ ID NO: 3)-3'dT |
| Ligation Strand/ Anchor Region 3 (16-3) | AMP-ID Region/UMI Multiplier-GATCGACATG (SEQ ID NO: 4)-3'dT |
| Ligation Strand/ Anchor Region 4 (16-4) | AMP-ID Region/UMI Multiplier-TGCATCAGGT (SEQ ID NO: 5)-3'dT |
| Non-ligation strand/Anchor Region 1 (16_1) | ***TGGCATACGT*** (SEQ ID NO: 6) |
| Non-ligation strand/Anchor Region 2 (16_2) | ***GTAACGCTAG*** (SEQ ID NO: 7) |
| Non-ligation strand/Anchor Region 3 (16_3) | ***CATGTCGATC*** (SEQ ID NO: 8) |
| Non-ligation strand/Anchor Region 4 (16_4) | ***ACCTGATGCA*** (SEQ ID NO: 9) |

The reaction mixture was incubated at 20°C for 30 min to generate the adaptor-tagged DNA fragments.

**Table 3. Exemplary adaptor sequences used for making an unamplified and an amplified tagged DNA library**

| **Adaptor Module ligation strands with 3' dT overhangs** | **SEQ ID NO** |
|---|---|
| TGCAGGACCAGAGAATTCGAATACAAAAATCCTNNNACGTATGCCAT | 10 |
| TGCAGGACCAGAGAATTCGAATACAAATGATCTNNNACGTATGCCAT | 11 |
| TGCAGGACCAGAGAATTCGAATACAAGTAATAGNNNACGTATGCCAT | 12 |
| TGCAGGACCAGAGAATTCGAATACACACCTCCGNNNACGTATGCCAT | 13 |
| TGCAGGACCAGAGAATTCGAATACACGCCCCATNNNACGTATGCCAT | 14 |
| TGCAGGACCAGAGAATTCGAATACACTACCAAGNNNACGTATGCCAT | 15 |
| TGCAGGACCAGAGAATTCGAATACACTGTCGTTNNNACGTATGCCAT | 16 |
| TGCAGGACCAGAGAATTCGAATACAGCAAATGGNNNACGTATGCCAT | 17 |
| TGCAGGACCAGAGAATTCGAATACAGCTCGAGCNNNACGTATGCCAT | 18 |
| TGCAGGACCAGAGAATTCGAATACAGTCCACAANNNACGTATGCCAT | 19 |
| TGCAGGACCAGAGAATTCGAATACAGTTACCCTNNNACGTATGCCAT | 20 |
| TGCAGGACCAGAGAATTCGAATACATAGTTTTCNNNACGTATGCCAT | 21 |
| TGCAGGACCAGAGAATTCGAATACATCTCAGAGNNNACGTATGCCAT | 22 |
| TGCAGGACCAGAGAATTCGAATACATGACCTTCNNNACGTATGCCAT | 23 |
| TGCAGGACCAGAGAATTCGAATACATTACGGCANNNACGTATGCCAT | 24 |
| TGCAGGACCAGAGAATTCGAATACAAACAAAACNNNTGCATCAGGTT | 25 |
| TGCAGGACCAGAGAATTCGAATACAACACTGCANNNTGCATCAGGTT | 26 |
| TGCAGGACCAGAGAATTCGAATACAATCGCGATNNNTGCATCAGGTT | 27 |
| TGCAGGACCAGAGAATTCGAATACAATGGTGGANNNTGCATCAGGTT | 28 |
| TGCAGGACCAGAGAATTCGAATACACAACTCTCNNNTGCATCAGGTT | 29 |
| TGCAGGACCAGAGAATTCGAATACACGCCCGAANNNTGCATCAGGTT | 30 |
| TGCAGGACCAGAGAATTCGAATACACGTATGACNNNTGCATCAGGTT | 31 |
| TGCAGGACCAGAGAATTCGAATACAGAAACGACNNNTGCATCAGGTT | 32 |
| TGCAGGACCAGAGAATTCGAATACAGACTCTGANNNTGCATCAGGTT | 33 |
| TGCAGGACCAGAGAATTCGAATACAGTCACTCTNNNTGCATCAGGTT | 34 |
| TGCAGGACCAGAGAATTCGAATACATACTGGACNNNTGCATCAGGTT | 35 |
| TGCAGGACCAGAGAATTCGAATACATGCGATACNNNTGCATCAGGTT | 36 |
| TGCAGGACCAGAGAATTCGAATACATGTTAATGNNNTGCATCAGGTT | 37 |
| TGCAGGACCAGAGAATTCGAATACATTGTACTTNNNTGCATCAGGTT | 38 |
| TGCAGGACCAGAGAATTCGAATACATTTGGCTCNNNTGCATCAGGTT | 39 |
| TGCAGGACCAGAGAATTCGAATACAAACGCCTANNNGATCGACATGT | 40 |
| TGCAGGACCAGAGAATTCGAATACAAAGTTTCANNNGATCGACATGT | 41 |
| TGCAGGACCAGAGAATTCGAATACAACAGCGAANNNGATCGACATGT | 42 |
| TGCAGGACCAGAGAATTCGAATACAAGCGCCTGNNNGATCGACATGT | 43 |
| TGCAGGACCAGAGAATTCGAATACACAACCCTTNNNGATCGACATGT | 44 |
| TGCAGGACCAGAGAATTCGAATACACAGAATAANNNGATCGACATGT | 45 |
| TGCAGGACCAGAGAATTCGAATACACGGACACCNNNGATCGACATGT | 46 |
| TGCAGGACCAGAGAATTCGAATACAGCCTATTCNNNGATCGACATGT | 47 |
| TGCAGGACCAGAGAATTCGAATACAGCGTCCAGNNNGATCGACATGT | 48 |
| TGCAGGACCAGAGAATTCGAATACAGGTACAAGNNNGATCGACATGT | 49 |
| TGCAGGACCAGAGAATTCGAATACATAACCCTCNNNGATCGACATGT | 50 |
| TGCAGGACCAGAGAATTCGAATACATAGGAGTGNNNGATCGACATGT | 51 |
| TGCAGGACCAGAGAATTCGAATACATCCGCATTNNNGATCGACATGT | 52 |
| TGCAGGACCAGAGAATTCGAATACATGCGTCAANNNGATCGACATGT | 53 |
| TGCAGGACCAGAGAATTCGAATACATTGGTAATNNNGATCGACATGT | 54 |
| TGCAGGACCAGAGAATTCGAATACAAATAGCTTNNNCTAGCGTTACT | 55 |
| TGCAGGACCAGAGAATTCGAATACAAGAGAGAGNNNCTAGCGTTACT | 56 |
| TGCAGGACCAGAGAATTCGAATACACAACCTGANNNCTAGCGTTACT | 57 |
| TGCAGGACCAGAGAATTCGAATACACATATGGCNNNCTAGCGTTACT | 58 |
| TGCAGGACCAGAGAATTCGAATACACCATATCCNNNCTAGCGTTACT | 59 |
| TGCAGGACCAGAGAATTCGAATACACGAGGTCCNNNCTAGCGTTACT | 60 |
| TGCAGGACCAGAGAATTCGAATACACGTCAATGNNNCTAGCGTTACT | 61 |
| TGCAGGACCAGAGAATTCGAATACACTTATCATNNNCTAGCGTTACT | 62 |
| TGCAGGACCAGAGAATTCGAATACAGCATTGACNNNCTAGCGTTACT | 63 |
| TGCAGGACCAGAGAATTCGAATACAGGAGGTATNNNCTAGCGTTACT | 64 |
| TGCAGGACCAGAGAATTCGAATACATAACAGTTNNNCTAGCGTTACT | 65 |
| TGCAGGACCAGAGAATTCGAATACATCGAACACNNNCTAGCGTTACT | 66 |
| TGCAGGACCAGAGAATTCGAATACATGCATAATNNNCTAGCGTTACT | 67 |
| TGCAGGACCAGAGAATTCGAATACATGTCATAANNNCTAGCGTTACT | 68 |
| TGCAGGACCAGAGAATTCGAATACATTGCGCGGNNNCTAGCGTTACT | 69 |

| | |
|---|---|
| *NNN in the sequences of Table 3 represents a 3-nucleotide UMI multiplier wherein each N may be selected from any one of A, G, C, T. | |

After ligation, 100 µL of DNA purification beads (Ampure XP^{®}; Beckman^{®}) were added to the ligation mix. The reaction mixture was incubated at room temperature for 2 min. The beads were washed two times with 200 µL of 80% ethanol/water (v/v) while on a magnet, air-dried, then eluted with 25 µL of TRIS-EDTA (TEZ). The eluted clarified supernatant, about 25 µL containing the adaptor-tagged DNA fragments, was transferred to a fresh PCR tube or microtiter plate well for amplification to generate the adaptor-tagged DNA library.

### EXAMPLE 4: TAGGED DNA LIBRARY EXTENSION AND AMPLIFICATION

Following adaptor ligation in Example 3, 75 µL of a master mix (MM) containing reagents and thermophilic DNA polymerase enzyme (NEB Ultra II 2X PCR Amplification^{®}; New England Biolabs^{®}) was added and the reaction mixture was amplified using the following run parameters:
60°C for 30 sec, 72°C for 2 min, 98°C for 30 sec;
8 cycles of 98°C for 30 sec, 65°C for 30 sec and 72°C for 30 sec.

A single amplification primer was used: TGCAGGACCAGAGAATTCGAATACA (SEQ ID NO: 70).

The initial 3 min incubation cycle was performed to form a plurality of contiguous adaptor-tagged dsDNA fragments by ligation-strand-templated extension, followed by an 8 cycle PCR amplification of the contiguous adaptor-tagged dsDNA fragments to form an amplified Tagged DNA library containing adaptor tagged DNA fragment molecules.

After amplification, 120 µL of DNA purification beads were added to the ligation mix. The reaction mixture was incubated at room temperature for 2 min. The beads were washed two times with 200 µL of 80% ethanol/water (v/v) while on a magnet, air-dried, then eluted with 14 µL of TEZ. Clarified supernatant containing the amplified tagged DNA library was transferred to a fresh PCR tube.

Using these methods, we reduced the time to preparing high efficiency libraries from about 8 hours to about 3 to 4 hours. The complexity was reduced as the number of enzymatic/kit reagents, including enzymes used in the examples were decreased from 16 to 4.

### EXAMPLE 5: CAPTURE PROBE LIBRARY AMPLIFICATION (PROPHETIC)

In order to capture and enrich genetic loci of interest, each Tagged DNA library (e.g. Disease Sample 1 and Disease Sample 2) prepared as described in Example 2 is combined, multiplexed, and hybridized to a pool of multifunctional capture probe modules specific for homologous repair deficient genes (e.g. ATM, BRCA1, BRCA2, FANCA, HDAC2, and PALB2) or to a pool of multifunctional capture probe modules specific for lung cancer genes (e.g. ERBB2, TP53, EML4-ALK fusion, EGFR, MET).

Next, 100uL of streptavidin-coated beads (Dynabeads MyOne C1) is combined with the hybridization reaction and allowed to stand at room temperature for 20 min. The beads are collected on a magnet and washed once with 200 µL of TEZ buffer. The washed beads are re-suspended in 40 µL of TEZ buffer. 160 µL of a wash buffer is added to the resuspended beads, and the mixture is incubated for 5 min at 45 °C. The beads are then separated using a magnet and washed with 200 µL of TEZ buffer.

Following hybridization, primer extension of the capture probe is used to copy the captured genomic sequences, the A/T overhang at the junction of the DNA fragment, and the attached adaptor module to form a library of hybrid molecules. The hybrid molecules thus formed comprise the DNA fragment flanked by the capture probe module on one end and the adaptor module on the other end.

Following on-bead probe extension, PCR is performed to incorporate Illumina^{®} sequencing adaptors. The beads are re-suspended in 20uL of TEZ, combined with PCR master mix (55uL of Ultra II PCR Mix, 5.5uL of Primer F, 5.5uL of Primer R), placed on a thermal cycler and run according to the following program: 60°C for 30 sec; 72°C for 30 sec; 98°C for 30 sec; 5 cycles of 98°C for 30 sec; 65°C for 30 sec; 72°C for 30 sec.

Next, the beads are separated from the reaction mixture on a magnet. The supernatant is transferred to a fresh PCR tube, combined with PCR MM and amplified on a thermal cycler using the following amplification cycles: 10 cycles of 98°C for 30 sec; 65°C for 30 sec; 72°C for 30 sec.

Forward Primer:

AATGATACGGCGACCACCGAGATCTACACGTCATGCAGGACCAGAGAATTCGAA
TACA (SEQ ID NO: 71).

Reverse Primer:

CAAGCAGAAGACGGCATACGAGATGTGACTGGCACGGGAGTTGATCCTGGTTTT
CAC (SEQ ID NO: 72).

This library of hybrid molecules is PCR amplified to provide amplified targeted DNA libraries containing amplified hybrid molecules for each of the samples. These amplified hybrid molecules are "sequencing ready" in that they contain sequencing primer binding sites at the two ends of the molecule as shown in **Fig. 8****.**

### EXAMPLE 6: SEQUENCE ANALYSIS

Genetic analysis was performed on hybrid molecules. Sequencing Read 1 (151 nt) and Read 2 (17 nt) were used for genetic analysis. For proper cluster and alignment analyses, each individual sequence read was processed to bioinformatically exclude the A/T nucleotide insertions (that were generated from the 3' terminal overhangs of the adaptor and the 5' terminal overhangs of the DNA fragments). This exclusion of the A/T insertion was performed by subjecting the sequence reads to genetic analysis using bioinformatics methods. The variant callers identified the redundant reads and processed the redundant reads into a single consensus read that was then quantified at each probe location. The variant callers further identified the junction of the adaptor and the 5' end of each DNA fragment to bioinformatically exclude the inserted A/T overhangs in order to obtain proper sample-specific DNA fragment sequences. Exclusion of the A/T insertion during genetic analysis increased the quality and reduced misalignment and/or inaccurate clustering of the sequence reads. Finally, statistical significance was assigned to deviations detected in each variant measurement.

### EXAMPLE 7: IMPROVEMENT TO SEQUENCING DEPTH

Sequencing was performed on the above-generated tagged DNA library. The tagged DNA library was aligned to a human reference genome and mapped to the intended target.

The average depth of 3 tagged DNA libraries (WT cfDNA, Disease Sample 1, and Disease Sample 2) using a Comparator Process and the Automatable Process was measured (See Fig. 4C).

### EXAMPLE 8: UNIFORM ADAPTOR DISTRIBUTION

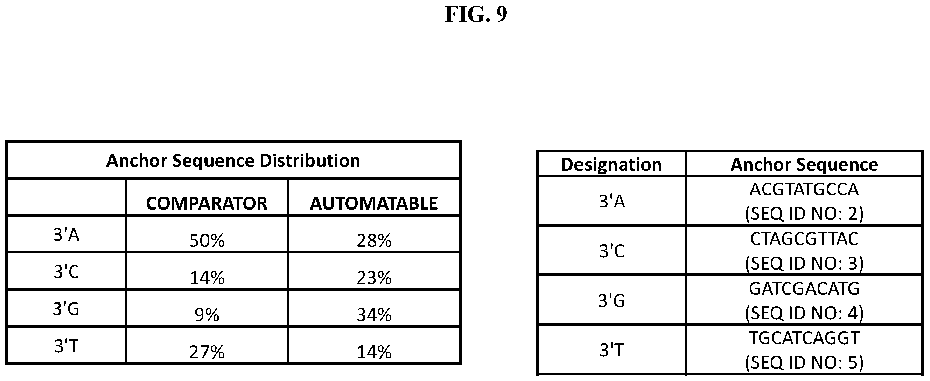

In the current example, bias against inclusion of certain adaptor sequences in a tagged DNA library as measured by sequence reads was reduced. Library preparation using the Automatable Process showed improved adaptor distribution compared with library preparation using the Comparator Process, eliminating the need to compensate for less-efficient adaptors. The resulting anchor distribution is depicted in **Fig. 9** and Table 4.

**Table 4: Library Preparation Comparison for Adaptor Distribution**

| | Anchor Sequence Distribution | |
|---|---|---|
| | Comparator Process % Distribution | Automatable Process % Distribution |
| 16-1 | 50% | 28% |
| 16-2 | 14% | 23% |
| 16-3 | 9% | 34% |
| 16-4 | 27% | 14% |

### EXAMPLE 9: VARIANT DETECTION

Tables 6 and 7 showed the number of sequencing reads for each variant on the Watson (+) or Crick strands (-) that results for samples prepared in Example 1 (Disease Sample 1 and Disease Sample 2). As can be seen in Table 5, for Disease Sample 1 the average reads for the variant Plus strand (+ strand) using the automatable process was 94 whereas it was 66 using the comparator process. Similarly, for Disease Sample 2, the average reads for the variant Plus strand (+ strand) using the automatable process was 238 whereas it was 199 using the comparator process for Disease Sample 1. These results suggest that for each of the detected variants tested, the process for making the tagged DNA library and probe capture library was much more efficient as can be measured by the increased number of reads for each variant and indicate an increase in assay sensitivity.

**Table 5: Variant detection comparison between the automatable process and comparator process using libraries prepared from Disease Sample 1**

| **DISEASE SAMPLE 1: Automatable Process** | | | | | | |
|---|---|---|---|---|---|---|
| | **Type** | **Mutation** | **WT(+)** | **WT(-)** | **Var(+)** | **Var(-)** |
| **Rep 1** | Indel | ATM frameshift | 1784 | 2328 | 78 | 86 |
| | Indel | BRCA1 frameshift | 2762 | 2446 | 167 | 80 |
| | Indel | BRCA2 frameshift | 1084 | 3630 | 58 | 94 |
| | SNV | BRCA2 G4* | 1178 | 3016 | 85 | 117 |
| | SNV | FANCA splice | 2313 | 2944 | 125 | 123 |
| | Indel | HDAC2 frameshift | 2170 | 2380 | 76 | 101 |
| | SNV | PALB2 Q479 | 2464 | 3079 | 110 | 186 |
| **Rep 2** | Indel | ATM frameshift | 1672 | 2156 | 89 | 87 |
| | Indel | BRCA1 frameshift | 2608 | 2321 | 126 | 83 |
| | Indel | BRCA2 frameshift | 1130 | 3346 | 53 | 151 |
| | SNV | BRCA2 G4* | 1143 | 2763 | 73 | 105 |
| | SNV | FANCA splice | 2292 | 2980 | 127 | 123 |
| | Indel | HDAC2 frameshift | 1967 | 2145 | 79 | 86 |
| | SNV | PALB2 Q479 | 2358 | 2925 | 70 | 129 |
| | **Avg** | | **1923** | **2747** | **94** | **111** |

| **DISEASE SAMPLE 1: Comparator Process** | | | | | | |
|---|---|---|---|---|---|---|
| | **Type** | **Mutation** | **WT(+)** | **WT(-)** | **Var(+)** | **Var(-)** |
| **Rep 1** | Indel | ATM frameshift | 1090 | 1839 | 49 | 86 |
| | Indel | BRCA1 frameshift | 1816 | 1652 | 71 | 60 |
| | Indel | BRCA2 frameshift | 531 | 2768 | 40 | 117 |
| | SNV | BRCA2 G4* | 354 | 1884 | 32 | 98 |
| | SNV | FANCA splice | 2327 | 2386 | 115 | 91 |
| | Indel | HDAC2 frameshift | 1174 | 1318 | 82 | 54 |
| | SNV | PALB2 Q479 | 1614 | 2198 | 65 | 64 |
| **Rep 2** | Indel | ATM frameshift | 1139 | 1874 | 65 | 109 |
| | Indel | BRCA1 frameshift | 1974 | 1650 | 66 | 63 |
| | Indel | BRCA2 frameshift | 616 | 2913 | 32 | 102 |
| | SNV | BRCA2 G4* | 427 | 1980 | 45 | 93 |
| | SNV | FANCA splice | 2484 | 2628 | 86 | 116 |
| | Indel | HDAC2 frameshift | 1723 | 1381 | 104 | 70 |
| | SNV | PALB2 Q479 | 1756 | 2378 | 65 | 102 |
| | **Avg** | | **1402** | **2061** | **66** | **88** |

**Table 6: Variant detection comparison between the automatable process and comparator process using libraries prepared from Disease Sample 2**

| **DISEASE SAMPLE 2: Automatable Process** | | | | | | | |
|---|---|---|---|---|---|---|---|
| | **Type** | **Mutation** | **WT(+)** | **WT(-)** | **Var(+)** | **Var(-)** | **Score** |
| **Rep 1** | SNV | ERBB2 1655V | 1453 | 1483 | 419 | 472 | |
| | SNV | TP53 Q331 | 2040 | 1679 | 270 | 216 | |
| | Indel | TP53 frameshift | 1197 | 2124 | 73 | 111 | |
| | Fusion | EML4-ALK fusion | 3457 | | 171 | | |
| | CNV | EGFR amplification | | | | | 5 .54E-17 |
| **Rep 2** | SNV | ERBB2 1655V | 1414 | 1422 | 491 | 534 | |
| | SNV | TP53 Q331 | 2126 | 1811 | 243 | 240 | |
| | Indel | TP53 frameshift | 1226 | 2198 | 76 | 137 | |
| | Fusion | EML4-ALK fusion | 3157 | | 161 | | |
| | CNV | EGFR amplification | | | | | 8.08E-17 |
| | **Avg** | | **2009** | **1786** | **238** | **285** | |

| **DISEASE SAMPLE 2: Comparator Process** | | | | | | | |
|---|---|---|---|---|---|---|---|
| | **Type** | **Mutuation** | **WT(+)** | **WT(-)** | **Var(+)** | **Var(-)** | **Score** |
| **Rep 1** | SNV | ERBB2 1655V | 1093 | 1197 | 347 | 385 | |
| | SNV | TP53 Q331 | 1650 | 1252 | 213 | 157 | |
| | Indel | TP53 frameshift | 909 | 1921 | 66 | 107 | |
| | Fusion | EML4-ALK fusion | 2879 | | 151 | | |
| | CNV | EGFR amplification | | | | | 2.89E-11 |
| **Rep 2** | SNV | ERBB2 1655V | 1134 | 1171 | 395 | 371 | |
| | SNV | TP53 Q331 | 1667 | 1297 | 190 | 100 | |
| | Indel | TP53 frameshift | 933 | 1875 | 53 | 115 | |
| | Fusion | EML4-ALK fusion | 2894 | | 174 | | |
| | CNV | EGFR amplification | | | | | 1.71E-11 |
| | **Avg** | | **1645** | **1452** | **199** | **218** | |

### EXAMPLE 10: IMPROVEMENTS TO AMPLIFICATION OF GENOMIC LIBRARIES

The conditions for amplifying genomic libraries were tested under three different conditions:
1) carrying out amplification of a library that had been divided into 2 separate tubes under an annealing temperature of 69 °C;
2) carrying out amplification of a library that had been divided into 2 separate tube under an annealing temperature of 65 °C; and
3) carrying out the amplification without dividing the library (in 1 tube) under an annealing temperature of 65 °C (Table 7).

Amplification conditions that were carried out without dividing the library under an annealing temperature of 65 °C (condition 3) performed well, eliminating the need to divide the library into 2 sample tubes and simplifying the library preparation process.

**Table 7: Optimization of amplification conditions**

| | **Input GEs** | **On Target Unique** | **On target Dup** | **Off target** | **Unaligned** | **Total reads** | **% Off Target** | **% Unaligned** |
|---|---|---|---|---|---|---|---|---|
| 2 tubes, 69 °C Anneal | 1000 | 1655079 | 8376345 | 275174 | 72971 | 10379569 | 2.7 | 0.7 |
| | 4000 | 6192870 | 27466258 | 992782 | 282263 | 34934173 | 2.8 | 0.8 |
| | 10000 | 12209458 | 31194609 | 1248334 | 369438 | 45021839 | 2.8 | 0.8 |
| | 20000 | 18387168 | 30512373 | 1388364 | 415609 | 50703514 | 2.7 | 0.8 |
| 2 tubes, 65° Anneal | 1000 | 1548719 | 5810708 | 191047 | 42991 | 7593465 | 2.5 | 0.6 |
| | 4000 | 5754578 | 19615062 | 712530 | 170191 | 26252361 | 2.7 | 0.6 |
| | 10000 | 13604693 | 31733716 | 1134323 | 298200 | 46770932 | 2.4 | 0.6 |
| | 20000 | 23349605 | 32085921 | 1277457 | 354548 | 57067531 | 2.2 | 0.6 |
| 1 tube, 65° anneal | 1000 | 1727578 | 10688885 | 326101 | 68866 | 12811430 | 2.5 | 0.5 |
| | 4000 | 6517578 | 28182111 | 882117 | 202677 | 35784483 | 2.5 | 0.6 |
| | 10000 | 12933835 | 29309214 | 976804 | 248028 | 43467881 | 2.2 | 0.6 |
| | 20000 | 20496827 | 29537398 | 1093181 | 290146 | 51417552 | 2.1 | 0.6 |

## Claims

1. A multifunctional adaptor comprising:
a) a ligation strand oligonucleotide, and
b) a non-ligation strand oligonucleotide that is capable of hybridizing to a region at the 3' end of the ligation strand oligonucleotide and forming a duplex therewith;
wherein, upon contact with a dsDNA fragment from a sample, the ligation strand oligonucleotide ligates to the 5' end of each strand of the dsDNA fragment;
wherein the ligation strand oligonucleotide comprises:
i) a 3' terminal overhang;
ii) an amplification region comprising a polynucleotide sequence capable of serving as a primer recognition site;
iii) a unique multifunctional ID region;
iv) a unique molecule identifier (UMI) multiplier; and
v) an anchor region comprising a polynucleotide sequence that is at least partially complementary to the non-ligation strand oligonucleotide;
wherein the dsDNA fragment comprises a phosphate group at the 5' terminus of each strand and an overhang at the 3' terminus of each strand;
wherein the combination of the multifunctional ID region and the UMI multiplier identifies the dsDNA fragment; and
wherein the multifunctional ID region identifies the sample.

2. The multifunctional adaptor of claim 1,
wherein the ligation strand oligonucleotide comprises a dT overhang at the 3' terminus and the dsDNA fragment comprises a dA overhang at the 3' terminus of each strand;
wherein the ligation strand oligonucleotide comprises a dA overhang at the 3' terminus and the dsDNA fragment comprises a dT overhang at the 3' terminus of each strand;
wherein the ligation strand oligonucleotide comprises a dC overhang at the 3' terminus and the dsDNA fragment comprises a dG overhang at the 3' terminus of each strand; or
wherein the ligation strand oligonucleotide comprises a dG overhang at the 3' terminus and the dsDNA fragment comprises a dC overhang at the 3' terminus of each strand;
and/or wherein the non-ligation strand oligonucleotide comprises a modification at its 3' terminus that prevents ligation to the 5' end of the dsDNA fragment and/or adaptor dimer formation, wherein the non-ligation strand is capable of being displaced from the duplex.

3. The multifunctional adaptor of any one of claims 1-2,
wherein the amplification region is 25 nucleotides in length;
wherein the multifunctional ID region is 8 nucleotides in length;
wherein the UMI multiplier is 3 nucleotides in length;
wherein the anchor region is 10 nucleotides in length;
wherein the UMI multiplier is adjacent to or contained within the multifunctional ID region; and
wherein the anchor region comprises one of four nucleotide sequences.

4. A complex comprising a multifunctional adaptor and a dsDNA fragment, wherein the multifunctional adaptor is selected from any one of the multifunctional adaptors of claims 1-3.

5. A method for making an adaptor-tagged DNA library comprising:
a) ligating a plurality of multifunctional adaptors with a plurality of dsDNA fragments to generate a plurality of multifunctional adaptor/dsDNA fragment complexes, wherein each of the plurality of multifunctional adaptors is selected from any one of the multifunctional adaptors of claims 1-3; and, optionally,
b) contacting the plurality of complexes from step (a) with one or more enzymes to form an adaptor-tagged DNA library comprising a plurality of contiguous adaptor-tagged DNA fragments.

6. The method of claim 5, wherein each multifunctional adaptor/dsDNA fragment complex of the plurality of complexes comprises a multifunctional adaptor ligated to each end of the dsDNA fragment.

7. The method of claim 5 or claim 6, wherein the plurality of dsDNA fragments comprises cell free DNA (cfDNA), genomic DNA (gDNA), complementary DNA (cDNA), mitochondrial DNA, methylated DNA, or demethylated DNA, and/or wherein the plurality of dsDNA fragments is end repaired prior to ligating with a plurality of multifunctional adaptors.

8. The method of any one of claims 5-7, wherein the non-ligation strand oligonucleotide is displaced from the multifunctional adaptor/dsDNA fragment complex in step (b).

9. The method of any one of claims 5-8, wherein the method comprises amplifying the plurality of contiguous adaptor-tagged DNA fragments to generate an amplified adaptor-tagged DNA library comprising a plurality of amplified contiguous adaptor-tagged dsDNA fragments, optionally wherein one or more primers are used for amplification, wherein the one or more primers comprise a universal primer binding sequence that hybridizes to the primer-binding region of the adaptor.

10. A method for making a probe-captured library comprising:
a) making an adaptor-tagged library according to the method of any one of claims 5-9;
b) hybridizing the adaptor-tagged DNA library with one or more multifunctional capture probes to form one or more capture probe/adaptor-tagged DNA complexes, wherein each multifunctional capture probe comprises
i) a first region capable of hybridizing to a partner oligonucleotide, wherein, optionally, the first region comprises a tail sequence comprising a PCR primer binding site; and
ii) a second region capable of hybridizing to a target region in the adaptor-tagged DNA library;
c) isolating the one or more capture probe/adaptor-tagged DNA complexes from step (b), wherein each isolated capture probe/adaptor-tagged DNA complex comprises a capture probe and an adaptor-tagged DNA fragment; and
d) enzymatically processing the isolated capture probe/DNA fragment complexes from step (c) to generate a probe-captured DNA library comprising hybrid molecules, each hybrid molecule comprising:
i) at least a portion of a capture probe or a complement thereof;
ii) at least a portion of a DNA fragment or a complement thereof; and
iii) an adaptor.

11. The method of claim 10, wherein the enzymatic processing step of (d) comprises performing 5'-3' DNA polymerase extension of the capture probe using the adaptor-tagged DNA fragment in the complex as a template, and/or wherein at least one capture probe hybridizes downstream of a specific region in the target region and at least one capture probe hybridizes upstream of the specific region in the target region.

12. The method of any one of claims 10-11, further comprising:
e) performing PCR on the hybrid molecules from step (d) to generate amplified hybrid molecules.

13. A probe-captured library comprising hybrid molecules or amplified hybrid molecules produced according to any one of claims 10-12.

14. A method comprising performing targeted genetic analysis on the probe-captured library of claim 13 optionally wherein the targeted genetic analysis comprises sequence analysis or copy number analysis, and/or wherein all or a portion of the capture probe region in each of the hybrid molecules is sequenced.

## Patentansprüche

1. Multifunktionaler Adapter, umfassend:
a) ein Ligationsstrang-Oligonukleotid und
b) ein Nicht-Ligationsstrang-Oligonukleotid, das in der Lage ist, mit einer Region am 3'-Ende des Ligationsstrang-Oligonukleotids zu hybridisieren und damit einen Duplex zu bilden;
wobei sich das Ligationsstrang-Oligonukleotid bei Kontakt mit einem dsDNA-Fragment aus einer Probe an das 5'-Ende jedes Strangs des dsDNA-Fragments ligiert;
wobei das Ligationsstrang-Oligonukleotid umfasst:
i) einen 3'-Endüberhang;
ii) eine Amplifikationsregion, die eine Polynukleotidsequenz umfasst, die als Primer-Erkennungsstelle dienen kann;
iii) eine eindeutige multifunktionale ID-Region;
iv) einen Multiplikator für die eindeutige Molekülkennung (UMI); und
v) eine Ankerregion, die eine Polynukleotidsequenz umfasst, die mindestens teilweise komplementär zu dem Nicht-Ligationsstrang-Oligonukleotid ist;
wobei das dsDNA-Fragment eine Phosphatgruppe am 5'-Ende jedes Strangs und einen Überhang am 3'-Ende jedes Strangs umfasst;
wobei die Kombination aus der multifunktionalen ID-Region und dem UMI-Multiplikator das dsDNA-Fragment identifiziert; und
wobei die multifunktionale ID-Region die Probe identifiziert.

2. Multifunktionaler Adapter nach Anspruch 1,
wobei das Ligationsstrang-Oligonukleotid einen dT-Überhang am 3'-Ende umfasst und das dsDNA-Fragment einen dA-Überhang am 3'-Ende jedes Strangs umfasst;
wobei das Ligationsstrang-Oligonukleotid einen dA-Überhang am 3'-Ende umfasst und das dsDNA-Fragment einen dT-Überhang am 3'-Ende jedes Strangs umfasst;
wobei das Ligationsstrang-Oligonukleotid einen dC-Überhang am 3'-Ende umfasst und das dsDNA-Fragment einen dG-Überhang am 3'-Ende jedes Strangs umfasst; oder
wobei das Ligationsstrang-Oligonukleotid einen dG-Überhang am 3'-Ende umfasst und das dsDNA-Fragment einen dC-Überhang am 3'-Ende jedes Strangs umfasst;
und/oder wobei das Nicht-Ligationsstrang-Oligonukleotid eine Modifikation an seinem 3'-Ende umfasst, die die Ligation an das 5'-Ende des dsDNA-Fragments und/oder die Bildung eines Adapterdimers verhindert, wobei der Nicht-Ligationsstrang aus dem Duplex verdrängt werden kann.

3. Multifunktionaler Adapter nach einem der Ansprüche 1 bis 2,
wobei die Amplifikationsregion 25 Nukleotide lang ist;
wobei die multifunktionale ID-Region 8 Nukleotide lang ist;
wobei der UMI-Multiplikator 3 Nukleotide lang ist;
wobei die Ankerregion 10 Nukleotide lang ist;
wobei der UMI-Multiplikator neben der multifunktionalen ID-Region liegt oder in dieser enthalten ist; und
wobei die Ankerregion eine von vier Nukleotidsequenzen umfasst.

4. Komplex, der einen multifunktionalen Adapter und ein dsDNA-Fragment umfasst, wobei der multifunktionale Adapter aus einem der multifunktionalen Adapter der Ansprüche 1 bis 3 ausgewählt ist.

5. Verfahren zum Herstellen einer Adapter-markierten DNA-Bibliothek, das Folgendes umfasst:
a) Ligieren einer Vielzahl von multifunktionalen Adaptern mit einer Vielzahl von dsDNA-Fragmenten, um eine Vielzahl von Komplexen aus multifunktionalen Adaptern und dsDNA-Fragmenten zu erzeugen, wobei jeder der Vielzahl von multifunktionalen Adaptern aus einem der multifunktionalen Adapter der Ansprüche 1 bis 3 ausgewählt ist; und optional,
b) Inkontaktbringen der Vielzahl von Komplexen aus Schritt (a) mit einem oder mehreren Enzymen, um eine Adapter-markierte DNA-Bibliothek zu bilden, die eine Vielzahl von zusammenhängenden Adapter-markierten DNA-Fragmenten umfasst.

6. Verfahren nach Anspruch 5, wobei jeder Komplex aus multifunktionalem Adapter und dsDNA-Fragment der Vielzahl von Komplexen einen multifunktionalen Adapter umfasst, der an jedes Ende des dsDNA-Fragments ligiert ist.

7. Verfahren nach Anspruch 5 oder 6, wobei die Vielzahl der dsDNA-Fragmente zellfreie DNA (cfDNA), genomische DNA (gDNA), komplementäre DNA (cDNA), mitochondriale DNA, methylierte DNA oder demethylierte DNA umfasst und/oder wobei die Enden der Vielzahl der dsDNA-Fragmente vor der Ligation mit einer Vielzahl multifunktionaler Adapter repariert werden.

8. Verfahren nach einem der Ansprüche 5 bis 7, wobei das Nicht-Ligationsstrang-Oligonukleotid in Schritt (b) aus dem Komplex aus multifunktionalem Adapter und dsDNA-Fragment-verdrängt wird.

9. Verfahren nach einem der Ansprüche 5 bis 8, wobei das Verfahren das Amplifizieren der Vielzahl von zusammenhängenden, Adapter-markierten DNA-Fragmenten umfasst, um eine amplifizierte, Adapter-markierte DNA-Bibliothek zu erzeugen, die eine Vielzahl von amplifizierten, zusammenhängenden, Adapter-markierten dsDNA-Fragmenten umfasst, wobei optional ein oder mehrere Primer zur Amplifikation verwendet werden, wobei der eine oder die mehreren Primer eine universelle Primerbindungssequenz umfassen, die mit der Primerbindungsregion des Adapters hybridisiert.

10. Verfahren zum Herstellen einer durch Sonden erfassten Bibliothek, das Folgendes umfasst:
a) Herstellen einer Adapter-markierten Bibliothek nach dem Verfahren nach einem der Ansprüche 5 bis 9;
b) Hybridisieren der Adapter-markierten DNA-Bibliothek mit einer oder mehreren multifunktionalen Erfassungssonden, um einen oder mehrere Komplexe aus Erfassungssonde und Adapter-markierter DNA zu bilden, wobei jede multifunktionale Erfassungssonde umfasst
i) eine erste Region, die mit einem Partner-Oligonukleotid hybridisieren kann, wobei die erste Region optional eine Schwanzsequenz umfasst, die eine PCR-Primer-Bindungsstelle umfasst; und
ii) eine zweite Region, die mit einer Zielregion in der Adapter-markierten DNA-Bibliothek hybridisieren kann;
c) Isolieren des einen oder der mehreren Komplexe aus Erfassungssonde und Adapter-markierter DNA aus Schritt (b), wobei jeder isolierte Komplex aus Erfassungssonde und Adapter-markierter DNA eine Erfassungssonde und ein Adapter-markiertes DNA-Fragment umfasst; und
d) enzymatisches Verarbeiten der isolierten Komplexe aus Erfassungssonde und DNA-Fragment aus Schritt (c), um eine Sonden-erfasste DNA-Bibliothek zu erzeugen, die Hybridmoleküle umfasst, wobei jedes Hybridmolekül Folgendes umfasst:
i) mindestens einen Teil einer Erfassungssonde oder ein Komplement davon;
ii) mindestens einen Teil eines DNA-Fragments oder ein Komplement davon; und
iii) einen Adapter.

11. Verfahren nach Anspruch 10, wobei der enzymatische Verarbeitungsschritt aus (d) das Durchführen einer 5'-3'-DNA-Polymeraseverlängerung der Erfassungssonde unter Verwendung des Adapter-markierten DNA-Fragments im Komplex als Matrize umfasst, und/oder wobei mindestens eine Erfassungssonde stromabwärts einer spezifischen Region in der Zielregion hybridisiert und mindestens eine Erfassungssonde stromaufwärts der spezifischen Region in der Zielregion hybridisiert.

12. Verfahren nach einem der Ansprüche 10 bis 11, ferner umfassend:
e) Durchführen einer PCR an den Hybridmolekülen aus Schritt (d), um amplifizierte Hybridmoleküle zu erzeugen.

13. Sonden-erfasste Bibliothek, die Hybridmoleküle oder amplifizierte Hybridmoleküle umfasst, die nach einem der Ansprüche 10 bis 12 hergestellt wurden.

14. Verfahren, das das Durchführen einer gezielten genetischen Analyse an der Sonden-erfassten Bibliothek von Anspruch 13 umfasst, wobei die gezielte genetische Analyse optional eine Sequenzanalyse oder eine Kopienzahlanalyse umfasst und/oder wobei die gesamte oder ein Teil der Erfassungssondenregion in jedem der Hybridmoleküle sequenziert wird.

## Revendications

1. Adaptateur multifonctionnel comprenant :
a) un oligonucléotide de brin de ligature, et
b) un oligonucléotide de brin de non-ligature qui est capable de s'hybrider à une région au niveau de l'extrémité 3' de l'oligonucléotide de brin de ligature et de former un duplex avec celle-ci ;
dans lequel, lors d'un contact avec un fragment d'ADNdb provenant d'un échantillon, l'oligonucléotide de brin de ligature forme une ligature avec l'extrémité 5' de chaque brin du fragment d'ADNdb ;
dans lequel l'oligonucléotide de brin de ligature comprend :
i) une saillie de terminaison 3' ;
ii) une région d'amplification comprenant une séquence polynucléotidique capable de servir de site de reconnaissance d'amorce ;
iii) une région d'identification multifonctionnelle unique ;
iv) un multiplicateur d'identificateur moléculaire unique (UMI) ; et
v) une région d'ancrage comprenant une séquence polynucléotidique qui est au moins partiellement complémentaire à l'oligonucléotide de brin de non-ligature ;
dans lequel le fragment d'ADNdb comprend un groupe phosphate au niveau de la terminaison 5' de chaque brin et une saillie au niveau de la terminaison 3' de chaque brin ;
dans lequel la combinaison de la région d'identification multifonctionnelle et du multiplicateur UMI identifie le fragment d'ADNdb ; et
dans lequel la région d'identification multifonctionnelle identifie l'échantillon.

2. Adaptateur multifonctionnel selon la revendication 1,
dans lequel l'oligonucléotide de brin de ligature comprend une saillie dT au niveau de la terminaison 3' et le fragment d'ADNdb comprend une saillie dA au niveau de la terminaison 3' de chaque brin ;
dans lequel l'oligonucléotide de brin de ligature comprend une saillie dA au niveau de la terminaison 3' et le fragment d'ADNdb comprend une saillie dT au niveau de la terminaison 3' de chaque brin ;
dans lequel l'oligonucléotide de brin de ligature comprend une saillie dC au niveau de la terminaison 3' et le fragment d'ADNdb comprend une saillie dG au niveau de la terminaison 3' de chaque brin ; ou
dans lequel l'oligonucléotide de brin de ligature comprend une saillie dG au niveau de la terminaison 3' et le fragment d'ADNdb comprend une saillie dC au niveau de la terminaison 3' de chaque brin ;
et/ou dans lequel l'oligonucléotide de brin de non-ligature comprend une modification au niveau de sa terminaison 3' qui empêche une ligature à l'extrémité 5' du fragment d'ADNdb et/ou une formation de dimère d'adaptateur, dans lequel le brin de non-ligature est capable d'être déplacé du duplex.

3. Adaptateur multifonctionnel selon l'une quelconque des revendications 1 à 2,
dans lequel la région d'amplification a une longueur de 25 nucléotides ;
dans lequel la région d'identification multifonctionnelle a une longueur de 8 nucléotides ;
dans lequel le multiplicateur UMI a une longueur de 3 nucléotides ;
dans lequel la région d'ancrage a une longueur de 10 nucléotides ;
dans lequel le multiplicateur UMI est adjacent à, ou contenu au sein de, la région d'identification multifonctionnelle ; et
dans lequel la région d'ancrage comprend l'une parmi quatre séquences nucléotidiques.

4. Complexe comprenant un adaptateur multifonctionnel et un fragment d'ADNdb, dans lequel l'adaptateur multifonctionnel est choisi parmi l'un quelconque des adaptateurs multifonctionnels selon les revendications 1 à 3.

5. Procédé permettant de fabriquer une banque d'ADN marquée par adaptateur comprenant :
a) la ligature d'une pluralité d'adaptateurs multifonctionnels avec une pluralité de fragments d'ADNdb pour générer une pluralité de complexes adaptateur multifonctionnel/fragment d'ADNdb, dans lequel chacun parmi la pluralité d'adaptateurs multifonctionnels est choisi parmi l'un quelconque des adaptateurs multifonctionnels selon les revendications 1 à 3 ; et, facultativement,
b) la mise en contact de la pluralité de complexes provenant de l'étape (a) avec une ou plusieurs enzymes pour former une banque d'ADN marquée par adaptateur comprenant une pluralité de fragments d'ADN marqués par adaptateur contigus.

6. Procédé selon la revendication 5, dans lequel chaque complexe adaptateur multifonctionnel/fragment d'ADNdb de la pluralité de complexes comprend un adaptateur multifonctionnel ligaturé à chaque extrémité du fragment d'ADNdb.

7. Procédé selon la revendication 5 ou la revendication 6, dans lequel la pluralité de fragments d'ADNdb comprend de l'ADN acellulaire (ADNac), de l'ADN génomique (gDNA), de l'ADN complémentaire (cDNA), de l'ADN mitochondrial, de l'ADN méthylé ou de l'ADN déméthylé, et/ou dans lequel la pluralité de fragments d'ADNdb est réparée en extrémité, avant ligature avec une pluralité d'adaptateurs multifonctionnels.

8. Procédé selon l'une quelconque des revendications 5 à 7, dans lequel l'oligonucléotide de brin de non-ligature est déplacé du complexe adaptateur multifonctionnel/fragment d'ADNdb à l'étape (b).

9. Procédé selon l'une quelconque des revendications 5 à 8, dans lequel le procédé comprend l'amplification de la pluralité de fragments d'ADN marqués par adaptateur contigus pour générer une banque d'ADN marquée par adaptateur amplifiée comprenant une pluralité de fragments d'ADNdb marqués par adaptateur contigus amplifiés, facultativement dans lequel une ou plusieurs amorces sont utilisées pour l'amplification, dans lequel la ou les amorces comprennent une séquence de liaison d'amorce universelle qui s'hybride à la région de liaison d'amorce de l'adaptateur.

10. Procédé permettant de fabriquer une banque capturée par sonde comprenant :
a) la fabrication d'une banque marquée par adaptateur selon le procédé de l'une quelconque des revendications 5 à 9 ;
b) l'hybridation de la banque d'ADN marquée par adaptateur avec une ou plusieurs sondes de capture multifonctionnelles pour former un ou plusieurs complexes sonde de capture/ADN marqué par adaptateur, dans lequel chaque sonde de capture multifonctionnelle comprend
i) une première région capable de s'hybrider à un oligonucléotide partenaire, dans lequel, facultativement, la première région comprend une séquence de queue comprenant un site de liaison d'amorce de PCR ; et
ii) une seconde région capable de s'hybrider à une région cible dans la banque d'ADN marquée par adaptateur ;
c) l'isolement du ou des complexes sonde de capture/ADN marqué par adaptateur provenant de l'étape (b), dans lequel chaque complexe sonde de capture/ADN marqué par adaptateur isolé comprend une sonde de capture et un fragment d'ADN marqué par adaptateur ; et
d) le traitement enzymatique des complexes sonde de capture/fragment d'ADN isolés provenant de l'étape (c) pour générer une banque d'ADN capturé par sonde comprenant des molécules hybrides, chaque molécule hybride comprenant :
i) au moins une partie d'une sonde de capture ou d'un complément de celle-ci ;
ii) au moins une partie d'un fragment d'ADN ou d'un complément de celui-ci ; et
iii) un adaptateur.

11. Procédé selon la revendication 10, dans lequel l'étape de traitement enzymatique de (d) comprend la mise en œuvre d'une extension d'ADN polymérase 5'-3' de la sonde de capture à l'aide du fragment d'ADN marqué par adaptateur dans le complexe en tant que modèle, et/ou dans lequel au moins une sonde de capture s'hybride en aval d'une région spécifique dans la région cible et au moins une sonde de capture s'hybride en amont de la région spécifique dans la région cible.

12. Procédé selon l'une quelconque des revendications 10 à 11, comprenant en outre :
e) la mise en œuvre d'une PCR sur les molécules hybrides provenant de l'étape (d) pour générer des molécules hybrides amplifiées.

13. Banque capturée par sonde comprenant des molécules hybrides ou des molécules hybrides amplifiées produites selon l'une quelconque des revendications 10 à 12.

14. Procédé comprenant la mise en œuvre d'une analyse génétique ciblée sur la banque capturée par sonde selon la revendication 13 facultativement dans lequel l'analyse génétique ciblée comprend une analyse de séquence ou une analyse de nombre de copies, et/ou dans lequel la totalité ou une partie de la région de sonde de capture dans chacune des molécules hybrides est séquencée.
